# EUROPEAN PATENT SPECIFICATION

(11) **EP 3 981 762 B1**
(45) Date of publication and mention of the grant of the patent: **15.10.2025**
(21) Application number: 20906208.2
(22) Date of filing: 16.06.2020
(51) Int. Cl.: H10K 50/16, C07D 213/16, C07D 239/26, C07D 251/24, C07D 401/04, C07D 401/10, C07D 401/14, C07D 403/04, C07D 403/10, C07D 405/04, C07D 409/04, C07D 411/14, C07D 413/04, C07D 471/04, C07D 405/14, C07D 409/14, H10K 85/60

(54) **NITROGEN-CONTAINING COMPOUND, ELECTRONIC ELEMENT, AND ELECTRONIC DEVICE**
STICKSTOFFHALTIGE VERBINDUNG, ELEKTRONISCHES ELEMENT UND ELEKTRONISCHE VORRICHTUNG
COMPOSÉ CONTENANT DE L'AZOTE, ÉLÉMENT ÉLECTRONIQUE ET DISPOSITIF ÉLECTRONIQUE

(30) Priority: 27.12.2019 CN 201911382838; 15.05.2020 CN 202010414445
(43) Date of publication of application: 13.04.2022
(73) Proprietor: Shaanxi Lighte Optoelectronics Material Co., Ltd., Xi'an, Shaanxi 710065 (CN)
(72) Inventor: YANG, Min, Shaanxi 710111 (CN); MA, Tiantian, Shaanxi 710084 (CN); NAN, Peng, Shaanxi 710065 (CN)
(74) Representative: Frick, Robert
(86) International application number: PCT/CN2020/096367
(87) International publication number: WO 2021/128764

(56) References cited:
- EP-A1- 3 845 522
- EP-A1- 3 978 475
- WO-A1-2020/046049
- CN-A- 110 128 279
- CN-A- 110 615 759
- CN-A- 111 039 881

## Description

### Technical field

The present disclosure relates to the technical field of organic materials, in particular to a nitrogen-containing compound, an electronic element using the nitrogen-containing compound, and an electronic device using the electronic element.

### Background

With the development of electronic technology and the advancement of materials science, the application scope of electronic components for realizing electroluminescence or photoelectric conversion becomes more and more extensive. Such electronic component usually includes a cathode and an anode disposed oppositely, and a functional layer disposed between the cathode and the anode. The functional layer is composed of multiple organic or inorganic film layers, and generally includes an energy conversion layer, a hole transporting layer disposed between the energy conversion layer and the anode, and an electron transporting layer disposed between the energy conversion layer and the cathode.

For example, when the electronic component is an organic electroluminescent device, it generally includes an anode, a hole transporting layer, an electroluminescent layer as an energy conversion layer, an electron transporting layer and a cathode, which are sequentially stacked. When a voltage is applied to between anode and cathode, the two electrodes generate an electric field. Under the action of the electric field, the electrons on the cathode side move to the electroluminescent layer, while the holes on the anode side move to the electroluminescent layer, so the electrons and the holes combine in the electroluminescent layer to form excitons, and the excitons are in an excited state and release energy outwards, which in turn makes the electroluminescent layer emit light outward.

Tris (8-hydroxyquinoline) aluminum, an electron transporting material commonly used in the market, is a unipolar transporting material with unstable cation radicals and low electron mobility. Although it has been reported that the use of new high-mobility electron transporting materials can improve the luminous efficiency of the device, the thermal stability of the material is not good enough, thus affecting the stability and service life of the device. At present, there is a lack of electron transporting materials that have both excellent electron transport properties and thermal stability. Therefore, the performance of the existing electron transporting layer materials needs to be further improved.

CN 110 128 279 A discloses an organic electroluminescent material that can be used as a hole injection layer, a hole transport layer, and an electron barrier layer in organic electroluminescent devices.

### Summary

The object of the present disclosure is to provide a nitrogen-containing compound, an electronic element and an electronic device to improve the performance of the electronic element and the electronic device.

In order to achieve the above object of the disclosure, the present disclosure adopts the following technical solutions.

According to the first aspect of the present disclosure, there is provided a nitrogen-containing compound according to claim 1 having the structure shown in Chemical Formula 1: 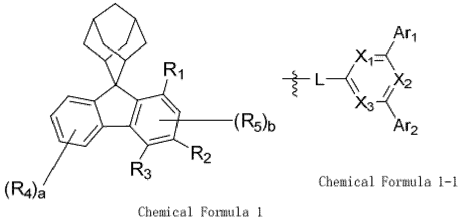
wherein 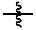 represents a chemical bond;
X₁, X₂, and X₃ are the same or different, and are each independently selected from C or N, and at least one is N;
R₁, R₂ and R₃ are each independently selected from hydrogen or a group represented by Chemical Formula 1-1, and one and only one of R₁, R₂ and R₃ has the group represented by Chemical Formula 1-1; when R_{1,} R₂ or R₃ is selected from hydrogen, said R₁, R₂ or R₃ may be replaced by R₅;
R₄, R₅ are the same or different, and are each independently selected from deuterium, halogen, a heteroaryl having 3 to 20 carbon atoms, an aryl having 6 to 20 carbon atoms, a trialkylsily having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, and an alkylthio having 1 to 10 carbon atoms, an aryloxy having 6 to 18 carbon atoms, an arylthio having 6 to 18 carbon atoms, a phosphoryloxy having 6 to 18 carbon atoms;
a is selected from 0, 1, 2, 3, or 4; when a is greater than 1, any two R₄ are the same or different;
b is selected from 0, 1, 2, or 3, when b is greater than 1, any two R₅ are the same or different;
L is selected from a single bond, a substituted or unsubstituted arylene having 6 to 30 carbon atoms, or a substituted or unsubstituted heteroarylene having 3 to 30 carbon atoms;
Ar₁ and Ar₂ are each independently selected from the following substituted or unsubstituted groups: an alkyl group having 1 to 20 carbon atoms, a cycloalkyl group having 3 to 20 carbon atoms, an aryl having 6 to 30 carbon atoms, a heteroaryl having 3 to 30 carbon atoms.

The substituents of L are selected from deuterium, halogen, cyano, a heteroaryl having 3 to 20 carbon atoms, an aryl having 6 to 20 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, and an alkylthio having 1 to 10 carbon atoms, an aryloxy having 6 to 18 carbon atoms, an arylthio having 6 to 18 carbon atoms, a phosphoryloxy having 6 to 18 carbon atoms.

The substituents in Ar₁ and Ar₂ are the same or different, and are each independently selected from deuterium, halogen, a heteroaryl having 3 to 20 carbon atoms, an aryl having 6 to 20 carbon atoms, and a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms.

The nitrogen-containing compound provided in the present disclosure has a molecular structure combining a nitrogen-containing heterocyclic ring with 1, 3 or 4-position adamantane fluorene. In one aspect, the nitrogen-containing heterocyclic compound can reduce the energy level injection into the barrier, thereby reducing the operating voltage of the organic electroluminescent device. At the same time, the nitrogen-containing heterocyclic structure is relatively stable, not easy to decompose, and has strong high temperature resistance, which can extend the service life of electronic elements, such as organic electroluminescence devices and photoelectric conversion devices. In another aspect, the adamantane-fluorene group has a large molecular weight, which can effectively increase the glass transition temperature of the material, and the large steric hindrance of its structure makes the material difficult to crystallize or aggregate, and the material has a better lifespan in electronic elements. Not only that, on the basis of the nitrogen-containing heterocyclic ring, the introduction of the adamantane-fluorene group as an electron injection and transport group makes the nitrogen-containing heterocyclic compound have electron-rich characteristics, and the polarity of the entire molecule is enhanced, which is more conducive to the directional arrangement of the material molecules, thereby enhancing the injection and transportation of electrons, enhancing the electronic conductivity of electron transporting materials. At the same time, the luminous efficiency of the organic electroluminescent device using the nitrogen-containing compound can be improved, and the conversion efficiency of the photoelectric conversion device using the nitrogen-containing compound can be improved.

According to the second aspect of the present disclosure, there is provided an electronic element including an anode and a cathode disposed oppositely, and a functional layer disposed between the anode and the cathode, wherein the functional layer contains the above nitrogen-containing compound. According to an embodiment of the present disclosure, the electronic element is an organic electroluminescence device. According to another embodiment of the present disclosure, the electronic element is a solar cell.

According to the third aspect of the present disclosure, there is provided an electronic device including the above electronic element.

### Brief Decrption of the Drawings

The above and other features and advantages of the present disclosure will become more apparent by describing in detail exemplary embodiments thereof with reference to the drawings.
FIG. 1 is a schematic structural diagram of an organic electroluminescent device according to an embodiment of the present disclosure.
FIG. 2 is a schematic structural diagram of a photoelectric conversion device according to an embodiment of the present disclosure.
FIG. 3 is a schematic structural diagram of an electronic device according to an embodiment of the present disclosure.
FIG. 4 is a schematic structural diagram of an electronic device according to another embodiment of the present disclosure.

The reference symbols of the main elements in the figure are as follows:
100, anode; 200, cathode; 300, functional layer; 310, hole injecting layer; 321, hole transporting layer; 322, electron blocking layer; 330, organic electroluminescent layer; 340, hole blocking layer; 350, electron transporting layer; 360, electron injecting layer; 370, photoelectric conversion layer; 400, the first electronic device; 500, the second electronic device.

### Detailed Description

Exemplary embodiments will now be described more fully with reference to the accompanying drawings. In the figures, the area and layer thickness may be exaggerated for clarity. The same reference symbols in the figures denote the same or similar structures, and thus their detailed description will be omitted.

The present disclosure provides a nitrogen-containing compound having a structure shown in Chemical Formula 1: 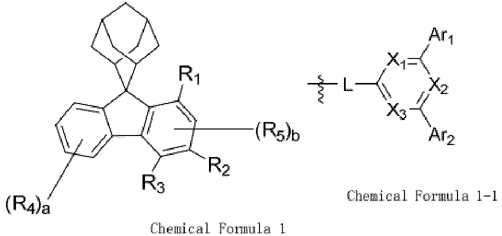
wherein 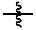 represents a chemical bond;
X₁, X₂, and X₃ are the same or different, and are each independently selected from C or N, and at least one is N;
R₁, R₂ and R₃ are each independently selected from hydrogen or a group represented by Chemical Formula 1-1, and one and only one of R_{1,} R₂ and R₃ has the group represented by Chemical Formula 1-1; when R₁, R₂ or R₃ is selected from hydrogen, said R₁, R₂ or R₃ may be replaced by R₅;
R₄, R₅ are the same or different, and are each independently selected from deuterium, halogen, a heteroaryl having 3 to 20 carbon atoms, an aryl having 6 to 20 carbon atoms, a trialkylsily having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, and an alkylthio having 1 to 10 carbon atoms, an aryloxy having 6 to 18 carbon atoms, an arylthio having 6 to 18 carbon atoms, a phosphoryloxy having 6 to 18 carbon atoms;
a is selected from 0, 1, 2, 3, or 4; when a is greater than 1, any two R₄ are the same or different;
b is selected from 0, 1, 2, or 3, when b is greater than 1, any two R₅ are the same or different;
L is selected from a single bond, a substituted or unsubstituted arylene having 6 to 30 carbon atoms, or a substituted or unsubstituted heteroarylene having 3 to 30 carbon atoms;
Ar₁ and Ar₂ are each independently selected from the following substituted or unsubstituted groups: an alkyl group having 1 to 20 carbon atoms, a cycloalkyl group having 3 to 20 carbon atoms, an aryl having 6 to 30 carbon atoms, a heteroaryl having 3 to 30 carbon atoms.

In the present disclosure, when R₁, R₂ or R₃ is selected from hydrogen, said R₁, R₂ or R₃ selected from hydrogen can be substituted by R₅. It means that when two of R_{1,} R₂ and R₃ are selected from hydrogen, one or both of them may be substituted by R₅, or none of them may be substituted by R₅. For example, when R₃ is selected from the chemical formula 1-1, and R₁ and R₂ are selected from hydrogen, one or both of R₁ and R₂ may be substituted by R₅, or both R₁ and R₂ may not be substituted by R₅, and the specific structural formula may include: 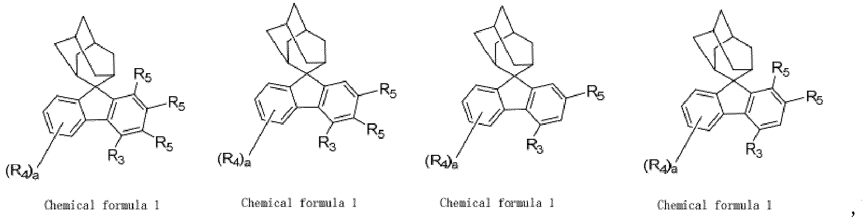 but it is not limited thereto.

In the present disclosure, since adamantane is a three-dimensional structure, in the structural diagram of compounds, because of the different drawing angles, it will show different planar shapes. The ring structure formed on 9,9-dimethylfluorene is all adamantane, and the connection position is also the same. For example: 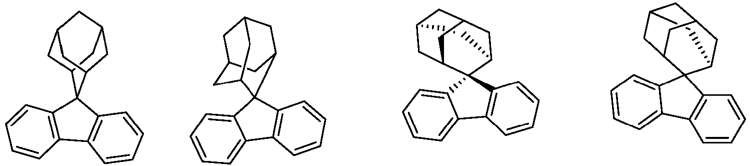 all have the same structure.

The nitrogen-containing compound provided in the present disclosure has a molecular structure combining a nitrogen-containing heterocyclic ring with 1, 3 or 4 adamantane fluorene. In one aspect, the nitrogen-containing heterocyclic compound can reduce the energy level injection into the barrier, thereby reducing the operating voltage of the organic electroluminescent device and increasing the open circuit voltage of the photoelectric conversion device. At the same time, the nitrogen-containing heterocyclic structure is relatively stable, not easy to decompose, and has strong high temperature resistance, which can extend the service life of electronic elements, such as organic electroluminescence devices and photoelectric conversion devices. In another aspect, the adamantane-fluorene group has a large molecular weight, which can effectively increase the glass transition temperature of the material, and the large steric hindrance of its structure makes the material difficult to crystallize or aggregate, and the material has a better lifespan in electronic elements. Not only that, on the basis of the nitrogen-containing heterocyclic ring, the introduction of the adamantane-fluorene group as an electron injection and transport group makes the nitrogen-containing heterocyclic compound have electron-rich characteristics, and the polarity of the entire molecule is enhanced, which is more conducive to the directional arrangement of the material molecules, thereby enhancing the injection and transportation of electrons, enhancing the electronic conductivity of electron transporting materials. At the same time, the luminous efficiency of the organic electroluminescent device using the nitrogen-containing compound can be improved, and the conversion efficiency of the photoelectric conversion device using the nitrogen-containing compound can be improved.

In the present disclosure, the number of carbon atoms in L, Ar₁ and Ar₂ refers to the number of all carbon atoms. For example, if L is selected from a substituted arylene group having 12 carbon atoms, the number of all carbon atoms of the arylene group and its substituents is 12. For example, if L is selected from a substituted arylene group having 12 carbon atoms, the number of all the carbon atoms of the arylene group and its substituents is 12. For example,

Ar₁ is 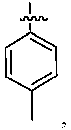 the number of carbon atoms is 7; L is 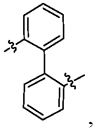 the number of carbon atoms is 12.

In the present disclosure, the substituted L, Ar₁ and Ar₂ are composed of its own group and the substituents linked to it. The substituents of L, Ar₁ and Ar₂ refer to the substituents linked to its own group. For example, in Ar_{1,} the heteroaryl substituted aryl group is 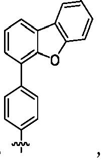 where phenyl is the basic group of an aryl and dibenzofuran is the substituent, then Ar₁ has 18 carbon atoms.

In the present disclosure, when no specific definition is provided otherwise, "hetero" means that a functional group includes at least one heteroatom such as B, N, O, S, or P, and the remaining atoms are carbon and hydrogen. An unsubstituted alkyl group may be a "saturated alkyl group" without any double or triple bonds.

In the present disclosure, "alkyl" may include linear or branched alkyl. The alkyl group may have 1 to 20 carbon atoms. In the present disclosure, a numerical range such as "1 to 20" refers to each integer in the given range; for example, "1 to 20 carbon atoms" refers to an alkyl that may contain 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms or 20 carbon atoms. The alkyl group may also be a medium-sized alkyl group having 1 to 10 carbon atoms. The alkyl group may also be a lower alkyl group having 1 to 6 carbon atoms. In addition, the alkyl group may be substituted or unsubstituted.

Preferably, the alkyl group is selected from alkyl groups having 1 to 6 carbon atoms. Specific examples include, but are not limited to, methyl, ethyl, propyl, isopropyl, n-butyl, isobutyl, and sec-butyl, tert-butyl, pentyl and hexyl.

In the present disclosure, "alkenyl" refers to a hydrocarbon group containing one or more double bonds in a linear or branched hydrocarbon chain. The alkenyl group may be unsubstituted or substituted. An alkenyl group can have 1 to 20 carbon atoms, and whenever it appears herein, a numerical range such as "1 to 20" refers to each integer in the given range. For example, "1 to 20 carbon atoms" means that it can include alkenyl having 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. For example, the alkenyl group may be vinyl, butadienyl, or 1,3,5-hexatrienyl.

In the present disclosure, "cycloalkyl" refers to a saturated hydrocarbon containing an alicyclic structure, which includes monocyclic and fused ring structures. The cycloalkyl group may have 3 to 20 carbon atoms, and a numerical range such as "3 to 20" refers to each integer in the given range. For example, "3 to 20 carbon atoms" means that it can include a cycloalkyl having 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. The cycloalkyl group may be a small ring, an ordinary ring, or a large ring with 3 to 20 carbon atoms. Cycloalkyl groups may have a structure selected from monocyclic rings (single ring), bicyclic rings (two rings), polycyclic rings (three or more rings). Cycloalkyl may also have a structure of spiro ring (two rings sharing one carbon atom-spiro ring), fused ring (two rings sharing two carbon atoms), and bridge ring (two rings sharing more than two carbon atoms). In addition, the cycloalkyl group may be substituted or unsubstituted.

Preferably, the cycloalkyl group is selected from cycloalkyl groups having 3 to 10 carbon atoms, and specific examples thereof include, but are not limited to, cyclopentyl, cyclohexyl, and adamantyl.

In the present disclosure, "aryl" refers to an optional functional group or substituent derived from an aromatic hydrocarbon ring. The aryl group may be a monocyclic aryl group or a polycyclic aryl group. In other words, the aryl group may be a monocyclic aryl group, or a fused ring aryl group, which is formed by two or more monocyclic aryls conjugatedly connected through a carbon-carbon bond, formed by a monocyclic aryl and a fused ring aryl conjugatedly connected by a carbon-carbon bond, or formed by two or more fused ring aryl groups conjugatedly connected by a carbon-carbon bond. That is, two or more aromatic groups conjugatedly connected through a carbon-carbon bond can also be regarded as aryl groups in the present disclosure. Among them, the aryl group does not contain heteroatoms such as B, N, O, S, or P. For example, biphenyl, terphenyl and the like are aryl groups in the present disclosure. Examples of aryl groups may include phenyl, naphthyl, fluorenyl, anthryl, phenanthryl, biphenyl, terphenyl, quaterphenyl, quinquephenyl, benzo[9,10]phenanthryl, pyrenyl, benzofluoranthenyl, chrysen, fluorenyl, etc., which are not limited thereto.

In the present disclosure, an aryl group having 6 to 25 ring-forming carbon atoms means that the number of carbon atoms in the aromatic ring in the aryl group is 6 to 25, and the number of carbon atoms in the substituents on the aryl group is not counted. The number of ring-forming carbon atoms in the aryl group may be 6, 10, 12, 14, 15, 18, 20, 25, but it is not limited thereto.

In the present disclosure, substituted aryl refers to one or more hydrogen atoms in the aryl group being substituted by other groups. For example, at least one hydrogen atom is substituted by deuterium, F, Cl, I, CN, branched alkyl, linear alkyl, cycloalkyl, alkoxy, or other groups. It should be understood that the substituted aryl having 18 carbon atoms means that the total number of carbon atoms of the aryl group and the substituents thereon is 18. For example, the number of carbon atoms of 2,3-dimethyl-6-phenylnaphthalene is 18, and the number of carbon atoms of both 9,9-diphenylfluorenyl and spirobifluorenyl is 25. Among them, biphenyl may be interpreted as aryl or substituted phenyl.

In the present disclosure, the fluorenyl group may be substituted, and two substituents may be combined with each other to form a spiro structure. Specific examples include but are not limited to the following structures:

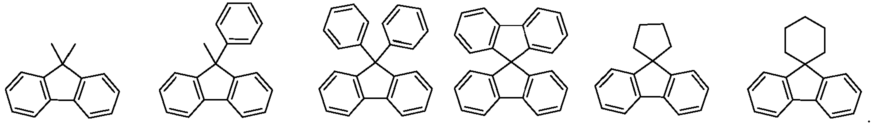

In the present disclosure, the "heteroaryl" refers to a heteroaryl group including at least one of B, O, N, P, Si, and S as a hetero atom. The heteroaryl group may be a monocyclic heteroaryl group or a polycyclic heteroaryl group. In other words, the heteroaryl group may be a single aromatic ring system or a polycyclic ring system formed by multiple aromatic rings conjugatedly connected through a carbon-carbon bond, where any one of the aromatic rings is an aromatic monocyclic ring or an aromatic fused ring. Exemplary the heteroaryl may include thienyl, furyl, pyrrolyl, imidazolyl, thiazolyl, oxazolyl, oxadiazolyl, triazolyl, pyridyl, bipyridyl, pyrimidinyl, triazinyl, acridinyl, pyridazinyl, pyrazinyl, quinolyl, quinazolinyl, quinoxalinyl, phenoxazinyl, phthalazinyl, pyridopyrimidinyl, pyridopyrazinyl, pyrazinopyrazinyl, isoquinolyl, indolyl, carbazolyl, N-arylcarbazolyl, N-heteroarylcarbazolyl, N-alkylcarbazolyl, benzoxazolyl, benzimidazoly, benzothiazolyl, benzocarbazolyl, benzothienyl, dibenzothienyl, thienothienyl, benzofuranyl, phenanthrolinyl, isoxazolyl, thiadiazolyl, benzothiazoly, phenothiazinyl, dibenzosilyl, dibenzofuranyl, phenyl substituted dibenzofuranyl, dibenzofuranyl substituted phenyl, etc., which are not limited thereto. Among them, thienyl, furyl, phenanthrolinyl, etc. are heteroaryl groups of a single aromatic ring system, and N-arylcarbazolyl, N-heteroarylcarbazolyl, phenyl substituted dibenzofuranyl, diphenylfuranyl substituted phenyl and the like are heteroaryl groups of multiple aromatic ring systems conjugatedly connected through a carbon-carbon bond.

In the present disclosure, the heteroaryl having 5 to 24 ring-forming carbon atoms refers to the number of carbon atoms of the heteroaryl ring in the heteroaryl group is 5 to 24, and the carbon atoms of the substituent on the heteroaryl group are not counted. The number of ring-forming carbon atoms in the heteroaryl group may be 5, 8, 12, 18, 20, 24, but not limited thereto.

In the present disclosure, the interpretation of aryl may be applied to arylene, and the interpretation of heteroaryl may also be applied to heteroarylene.

In the present disclosure, the halogen may be fluorine, chlorine, bromine, or iodine.

In the present disclosure, among the substituents of L, Ar₁ and Ar₂, optionally, any two adjacent substituents may or may not form a ring. When forming a ring, two adjacent substituents may form a ring through a single bond, or may be fused to form a ring.

In the present disclosure, optionally, R₄, R₅ are each independently selected from deuterium, halogen, alkyl having 1 to 5 carbon atoms, an aryl having 6 to 12 carbon atoms. In one embodiment of the present disclosure, examples of R₄ and R₅ include, but are not limited to, deuterium, fluorine, methyl, tert-butyl, phenyl, biphenyl, or naphthyl.

The substituents in L are selected from deuterium, halogen, cyano, a heteroaryl having 3 to 20 carbon atoms, an aryl having 6 to 20 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms.

Optionally, the substituents in L are selected from deuterium, halogen, cyano, a heteroaryl having 3 to 12 carbon atoms, an aryl groups having 6 to 15 carbon atoms, and a trialkylsilyl having 3 to 7 carbon atoms, an alkyl group having 1 to 5 carbon atoms, a haloalkyl having 1 to 5 carbon atoms, and a cycloalkyl having 5 to 10 carbon atoms.

The substituents in Ar₁ and Ar₂ are the same or different, and are independently selected from deuterium, halogen, a heteroaryl having 3 to 20 carbon atoms, an aryl having 6 to 20 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms.

Optionally, the substituents of Ar₁ and Ar₂ are selected from deuterium, halogen, a heteroaryl having 3 to 12 carbon atoms, an aryl having 6 to 15 carbon atoms, a trialkylsilyl and having 3 to 7 carbon atoms, an alkyl having 1 to 5 carbon atoms, a haloalkyl having 1 to 5 carbon atoms, and a cycloalkyl having 5 to 10 carbon atoms.

Optionally, L is selected from a single bond, a substituted or unsubstituted arylene having 6 to 25 ring-forming carbon atoms, and a substituted or unsubstituted heteroarylene having 5 to 18 ring-forming carbon atoms.

Optionally, L is selected from a single bond, a substituted or unsubstituted arylene group having 6 to 25 carbon atoms, and a substituted or unsubstituted heteroarylene group having 5 to 20 carbon atoms.

According to a specific embodiment, L is selected from a single bond, a substituted or unsubstituted phenylene, a substituted or unsubstituted naphthylene, a substituted or unsubstituted phenanthrylene, a substituted or unsubstituted anthrylene, a substituted or unsubstituted fluorenylene, a substituted or unsubstituted biphenylene, a substituted or unsubstituted terphenylene, a substituted or unsubstituted pyridylene, a substituted or unsubstituted dibenzofuranyl, a substituted or unsubstituted dibenzothienylene, a substituted or unsubstituted N-phenylcarbazolylidene.

Optionally, the substituents in L are selected from deuterium, halogen, cyano, an aryl having 6 to 12 carbon atoms, a heteroaryl having 5 to 18 carbon atoms, and an alkyl having 1 to 5 carbon atoms, a cycloalkyl having 3 to 10 carbon atoms.

Further optionally, the substituents in L are selected from deuterium, fluorine, cyano, an alkyl having 1 to 5 carbon atoms, a cycloalkyl having 3 to 10 carbon atoms, phenyl, naphthyl, and pyridyl. The substituents in L may be selected from, for example, deuterium, fluorine, cyano, phenyl, naphthyl, biphenyl, pyridyl, methyl, ethyl, n-propyl, isopropyl, tert-butyl, cyclohexyl, cyclopentyl, adamantyl; more specifically, for example, deuterium, fluorine, cyano, phenyl, methyl, propyl, isopropyl, tert-butyl.

Optionally, L is selected from a single bond or the group consisting of groups of the chemical formula j-1 to chemical formula j-13: 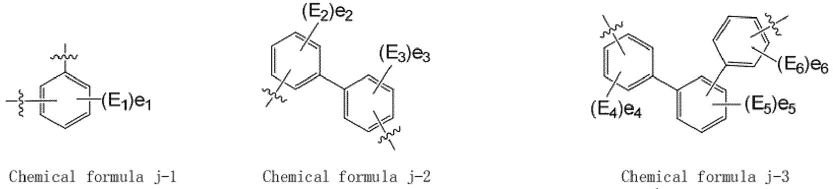 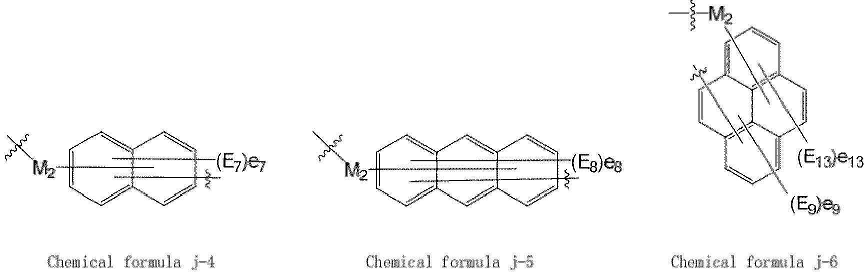 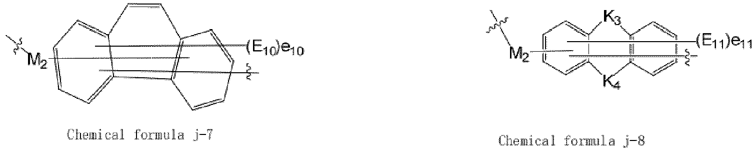 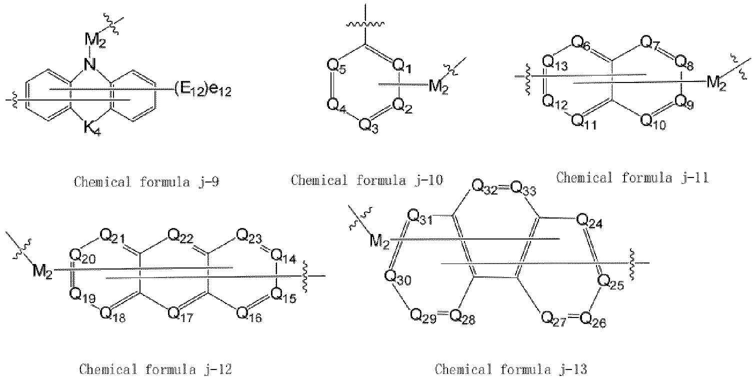 wherein M₂ is selected from a single bond or 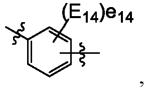
Q₁ to Q₅ are each independently selected from N or C(F₅), and at least one of Q₁ to Q₅ is selected from N; when two or more of Q₁ to Q₅ are selected from C(F₅), any two of F₅ are the same or different;
Q₆ to Q₁₃ are each independently selected from N or C(F₆), and at least one of Q₆ to Q₁₃ is selected from N; when two or more of Q₆ to Q₁₃ are selected from C(F₆), any two of F₆ are the same or different;
Q₁₄ to Q₂₃ are each independently selected from N or C(F₇₎, and at least one of Q₁₄ to Q₂₃ is selected from N; when two or more of Q₁₄ to Q₂₃ are selected from C(F₇), any two of F₇ are the same or different;
Q₂₄ to Q₃₃ are each independently selected from N or C(F₈), and at least one of Q₂₄ to Q₃₃ is selected from N; when two or more of Q₂₄ to Q₃₃ are selected from C(F₈), any two of F₈ are the same or different;
E₁ to E₁₄, F₅ to F₈ are each independently selected from deuterium, halogen, cyano, a heteroaryl having 3 to 18 carbon atoms, an aryl having 6 to 18 carbon atoms and optionally substituted by deuterium, fluorine, chlorine or cyano, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms; among them, "an aryl having 6 to 18 carbon atoms and optionally substituted by deuterium, fluorine, chlorine or cyano" refers to an aryl that may or may not be substituted by deuterium, fluorine, chlorine, cyano.
any one of F₅ to F₈ may also be independently selected from hydrogen;
eᵣ is the number of substituents E_{r,} r is any integer from 1 to 14; when r is selected from 1, 2, 3, 4, 5, 6, 9, 13 or 14, eᵣ is selected from 0, 1, 2, 3 or 4; when r is selected from 7 or 11, eᵣ is selected from 0, 1, 2, 3, 4, 5 or 6; when r is 12, eᵣ is selected from 0, 1, 2, 3, 4, 5, 6 or 7; when r is selected from 8 or 10, eᵣ is selected from 0, 1, 2, 3, 4, 5, 6, 7 or 8; when eᵣ is greater than 1, any two of Eᵣ are the same or different;
K₃ is selected from O, S, Se, N(E₁₅), C(E₁₆E₁₇), Si(E₁₆E₁₇); wherein, E₁₅, E₁₆, E₁₇ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocycloalkenyl group having 4 to 10 carbon atoms, or the above E₁₆ and E₁₇ are connected to each other to form a ring with the atom that they are commonly connected to;
K₄ is selected from a single bond, O, S, Se, N(E₁₈), C(E₁₉E₂₀), Si(E₁₉E₂₀); wherein, E₁₈, E₁₉, E₂₀ are each independently selected from an aryl having 6 to 18 carbon atoms, a heteroaryl having 3 to 18 carbon atoms, an alkyl having 1 to 10 carbon atoms, alkenyl having 2 to 6 carbon atoms, alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, or the above E₁₉ and E₂₀ are connected to each other to form a ring with the atom that they are commonly connected to.

In the present disclosure, E₁₆ and E₁₇ are independent of each other and are not directly connected; or E₁₆ and E₁₇ are directly connected to form a ring with the commonly connected atom, for example: a ring having 3 to 15 carbon atoms can be formed, or a ring having 3 to 10 carbon atoms can be formed; the ring may be saturated (such as a five-membered ring, a six-membered ring, adamantane, etc.), or unsaturated, such as an aromatic ring.In other words, E₁₆ and E₁₇ can be independent substituents, or they maybe connected to each other to form a cyclic group. Specific examples of cyclic groups that can be formed include but are not limited to: cyclopropane, cyclobutane, cyclopentane, cyclohexane and adamantane. This explanation also applies to E₁₉ and E₂₀.

Optionally, L is selected from a single bond or the group consisting of the following groups:

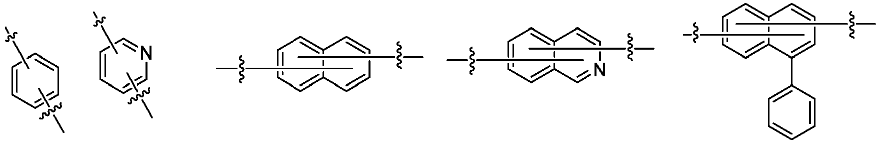

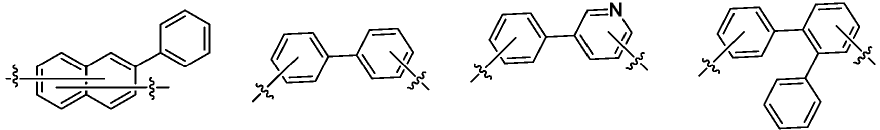

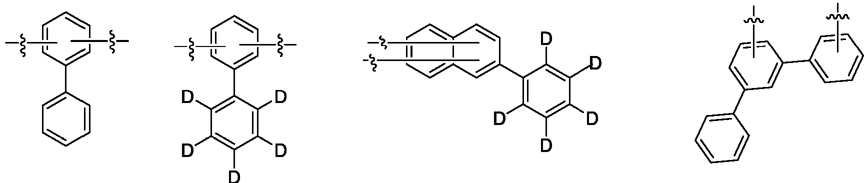

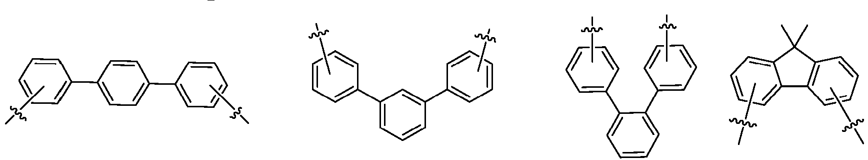

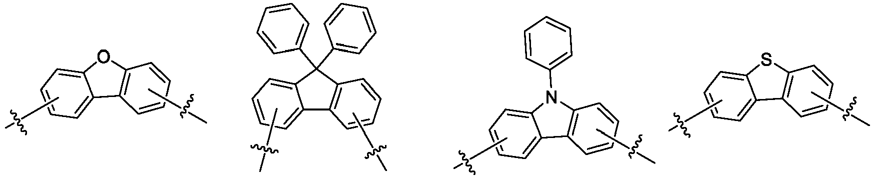

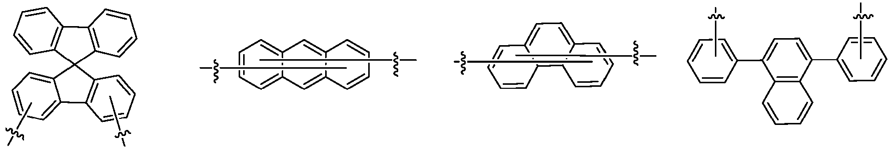

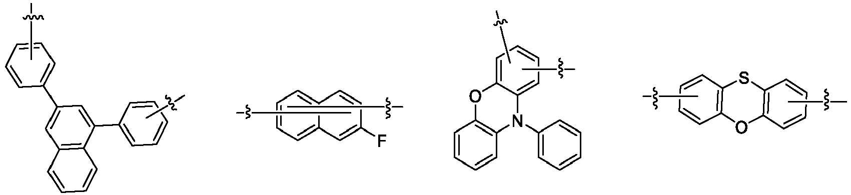

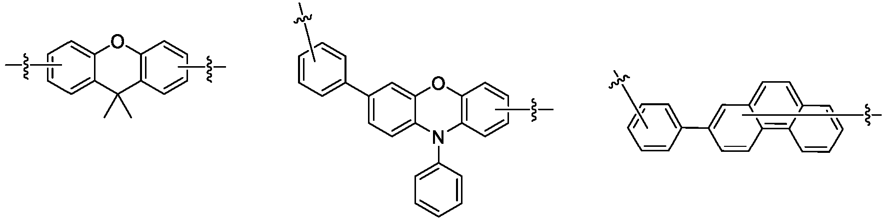

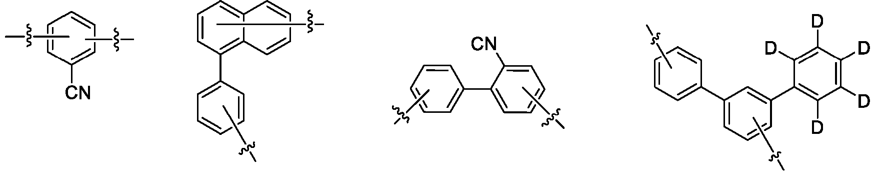

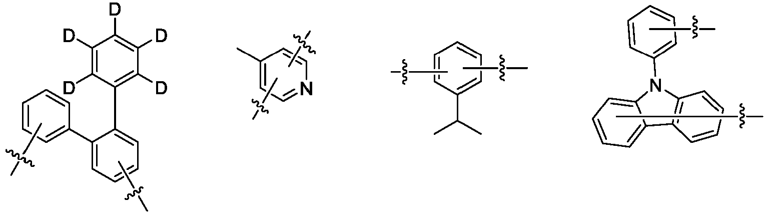

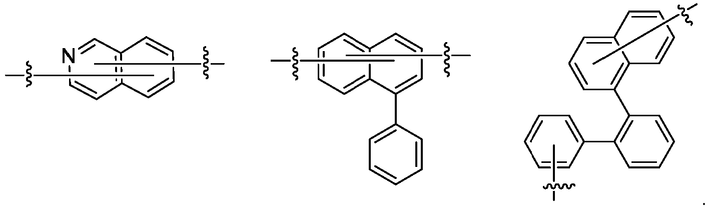

Optionally, Ar₁ and Ar₂ are independently selected from the following substituted or unsubstituted groups: an aryl having 6 to 25 ring-forming carbon atoms, and a heteroaryl having 5 to 20 ring-forming carbon atoms.

Optionally, Ar₁ and Ar₂ are independently selected from substituted or unsubstituted aryl groups having 6 to 25 carbon atoms, and a substituted or unsubstituted heteroaryls having 5 to 24 carbon atoms.

Further optionally, Ar₁ and Ar₂ are each independently selected from substituted or unsubstituted aryl groups having 6 to 25 carbon atoms, and substituted or unsubstituted heteroaryl groups having 5 to 20 carbon atoms.

Optionally, the substituents in Ar₁ and Ar₂ are independently selected from deuterium, halogen, an aryl having 6 to 12 carbon atoms, a heteroaryl having 3 to 20 carbon atoms, and an alkyl having 1 to 5 carbon atoms, a cycloalkyl having 3 to 10 carbon atoms.

Preferably, the substituents in Ar₁ and Ar₂ are each independently selected from deuterium, fluorine, phenyl, naphthyl, biphenyl, dimethylfluorenyl, methyl, propyl, isopropyl, tert-butyl, pyridyl, pyrimidyl, dibenzofuranyl, dibenzothienyl, N-phenylcarbazolyl.
In some embodiments, Ar₁ and Ar₂ are selected from substituents represented by the following chemical formula i-1 to i-15: 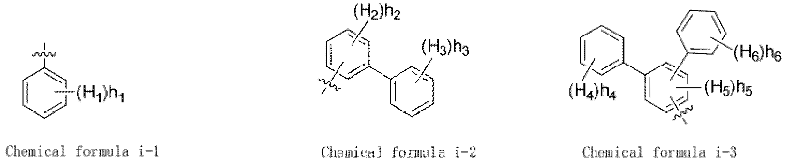 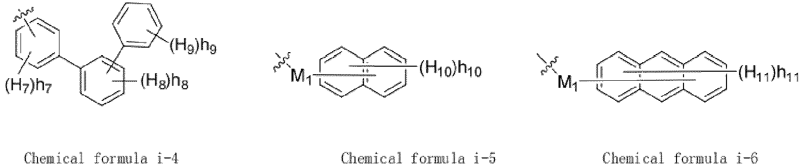 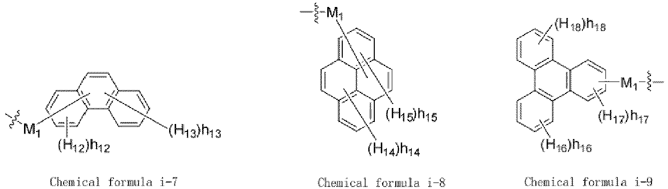 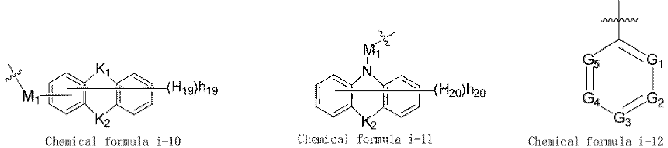 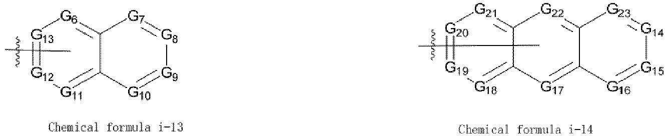 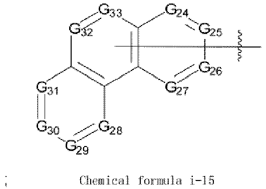
wherein M₁ is selected from a single bond or 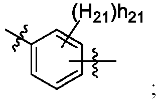
G₁ to G₅ are each independently selected from N or C(F₁), and at least one of G₁ to G₅ is selected from N; when two or more of G₁ to G₅ are selected from C(F₁), any two F₁ are the same or different;
G₆ to G₁₃ are each independently selected from N or C(F₂), and at least one of G₆ to G₁₃ is selected from N; when two or more of G₆ to G₁₃ are selected from C(F₂), any two F₂ are the same or different;
G₁₄ to G₂₃ are each independently selected from N or C(F₃), and at least one of G₁₄ to G₂₃ is selected from N; when two or more of G₁₄ to G₂₃ are selected from C(F₃), any two F₂ are the same or different;
G₂₄ to G₃₃ are each independently selected from N or C(F₄), and at least one of G₂₄ to G₃₃ is selected from N; when two or more of G₂₄ to G₃₃ are selected from C(F₄), any two F₂ are the same or different;
H₁ to H₂₁, F₁ to F₄ are each independently selected from deuterium, halogen, a heteroaryl having 3 to 18 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms;
any one of H₄ to H₂₀ may also be independently selected from an aryl having 6 to 18 carbon atoms;
any one of F₁ to F₄ may also be independently selected from hydrogen and an aryl having 6 to 18 carbon atoms;
hₖ is the number of substituents Hₖ, k is any integer from 1 to 21; wherein, when k is selected from 5 or 17, hₖ is selected from 0, 1, 2 or 3; when k is selected from 2, 7, 8, 12, 15, 16, 18, or 21, hₖ is selected from 0, 1, 2, 3, or 4; when k is 1, 3, 4, 6, 9 or 14, hₖ is selected from 0, 1, 2, 3, 4, or 5; when k is 13, hₖ is selected from 0, 1, 2, 3, 4, 5, or 6; when k is selected from 10 or 19, hₖ is selected from 0, 1, 2, 3, 4, 5, 6 or 7; when k is selected from 20, hₖ is selected from 0, 1, 2, 3, 4, 5, 6, 7 or 8; when k is 11, hₖ is selected from 0, 1, 2, 3, 4, 5, 6, 7, 8, or 9;
When hₖ is greater than 1, any two Hk are the same or different;
K₁ is selected from O, S, Se, N (H₂₂), C (H₂₃H₂₄), Si (H₂₃H₂₄); wherein, H₂₂, H₂₃, and H₂₄ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms,a cycloalkenyl having 5 to 10 carbon atoms, a heterocyclenyl having 4 to 10 carbon atoms, or the above H₂₃ and H₂₄ are connected to each other to form a ring with the atom they are commonly connected to;
K₂ is selected from a single bond, O, S, Se, N(H₂₅), C(H₂₆H₂₇), Si(H₂₆H₂₇);
wherein, H₂₅, H₂₆, H₂₇ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, or the above H₂₆ and H₂₇ are connected to each other to form a ring with the atom they are commonly connected to.

In the present disclosure, H₂₃ and H₂₄ are independent of each other and are not directly connected; or H₂₃ and H₂₄ are directly connected to form a ring with the commonly connected atoms, for example: a ring having 3 to 15 carbon atoms can be formed, or a ring having 3 to 10 carbon atoms can be formed; the ring may be saturated (such as a five-membered ring, a six-membered ring, adamantane, etc.), or unsaturated, such as an aromatic ring. In other words, H₂₃ and H₂₄ can be independent substituents, or they maybe connected to each other to form a cyclic group. Specific examples of cyclic groups that can be formed include, but are not limited to: cyclopropane, cyclobutane, cyclopentane, cyclohexane and adamantane. This explanation also applies to H₂₆ and H₂₇.

According to one embodiment, Ar₁ and Ar₂ are each independently selected from the group consisting of the following groups:

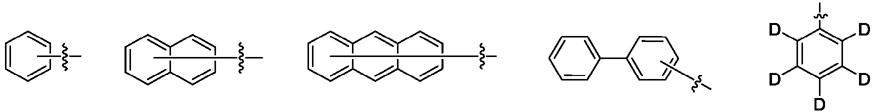

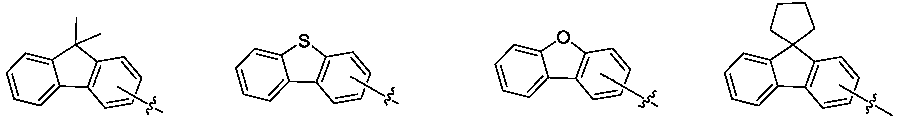

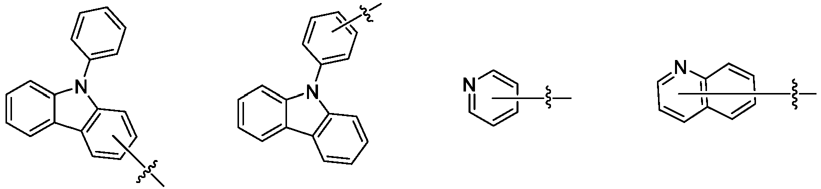

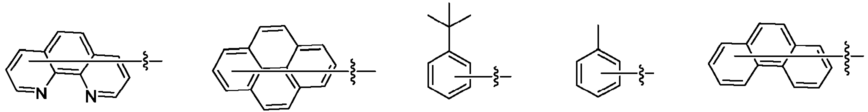

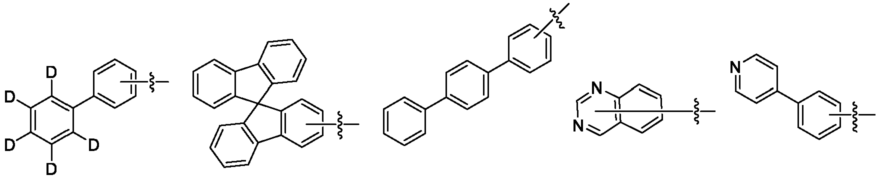

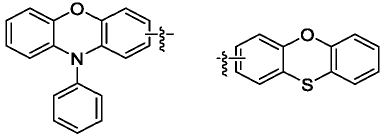

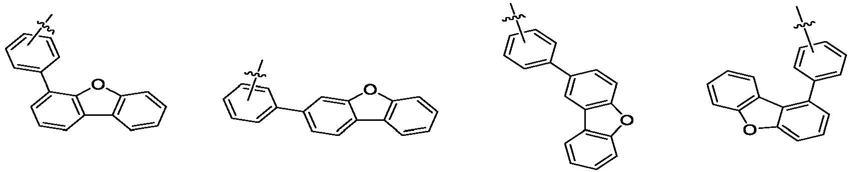

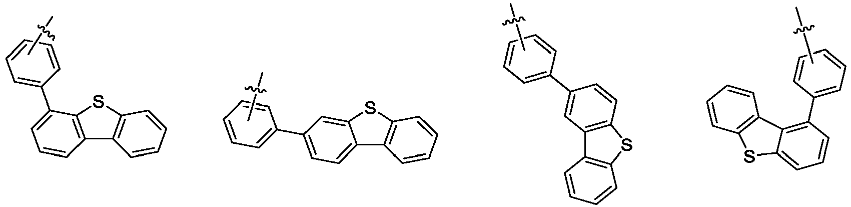

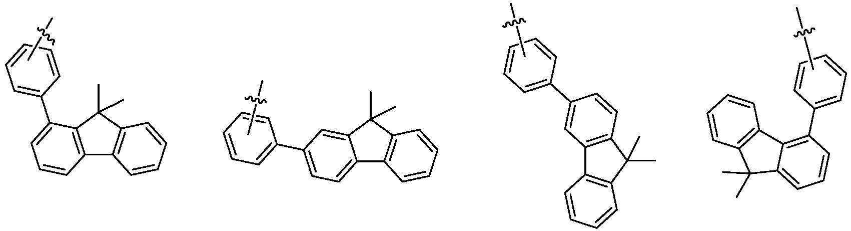

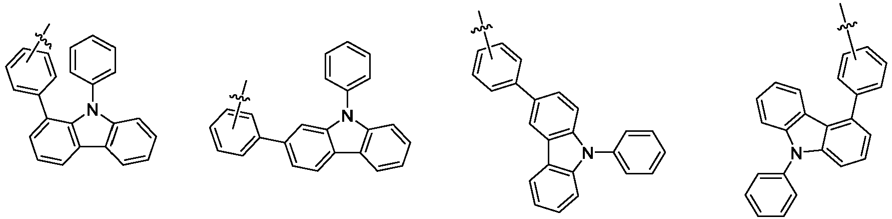

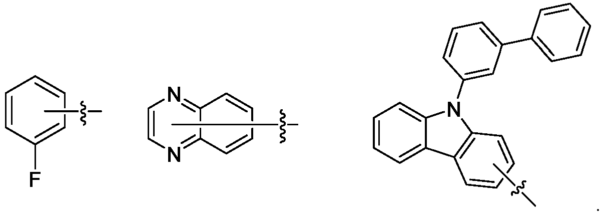

Optionally, the nitrogen-containing compound is selected from the group consisting of the following compounds:

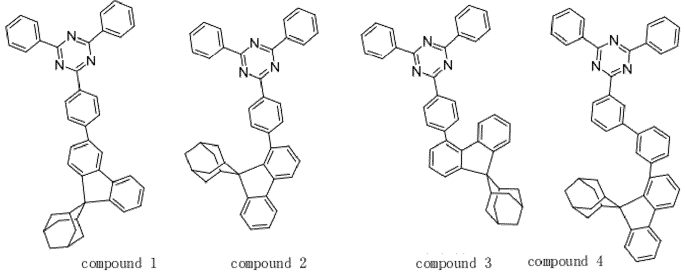

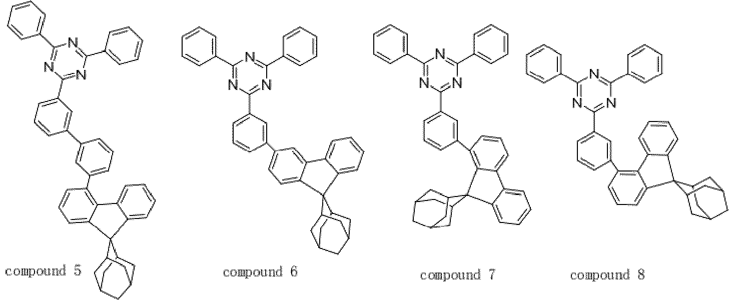

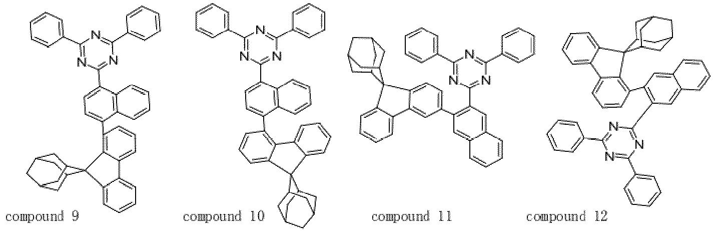

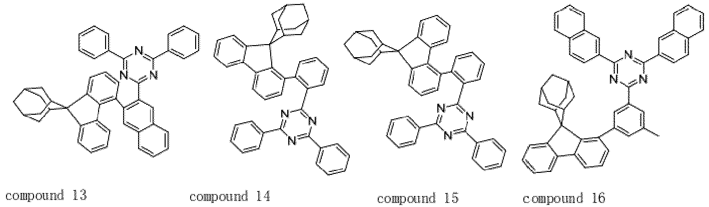

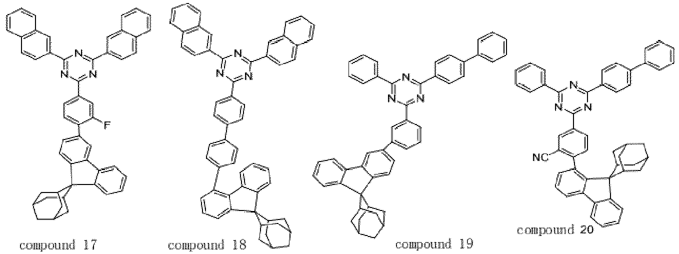

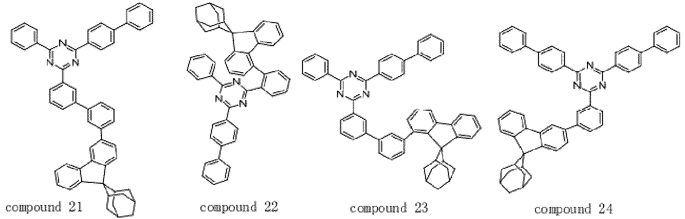

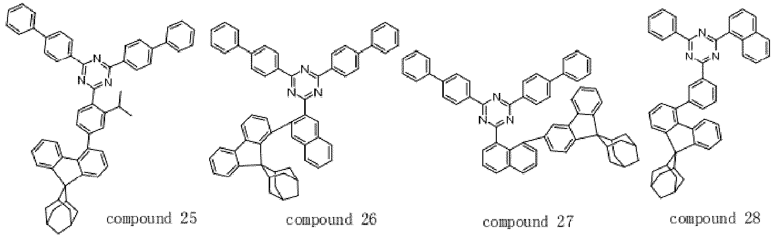

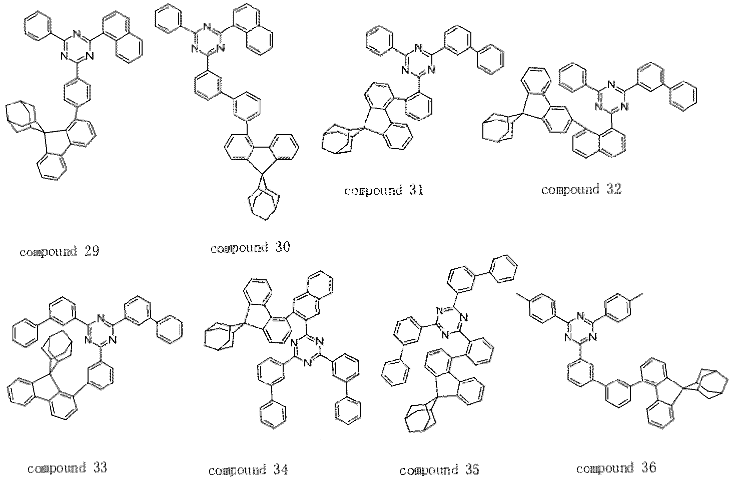

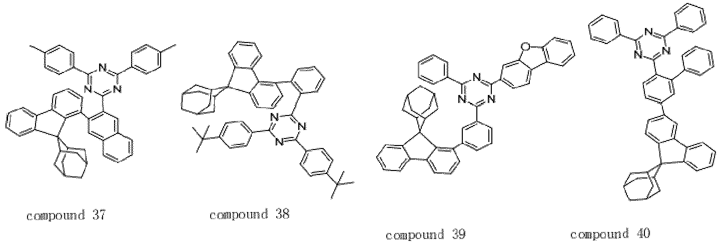

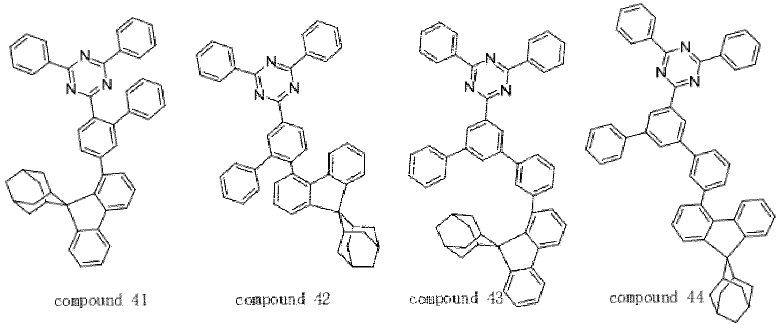

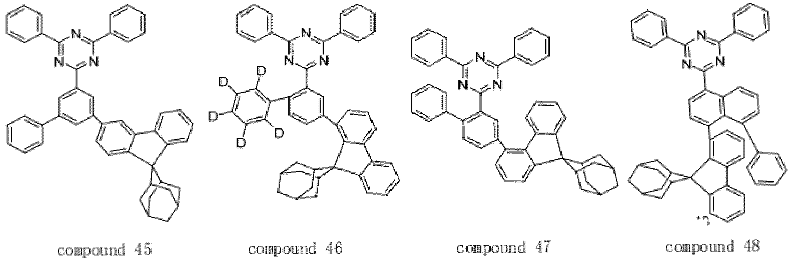

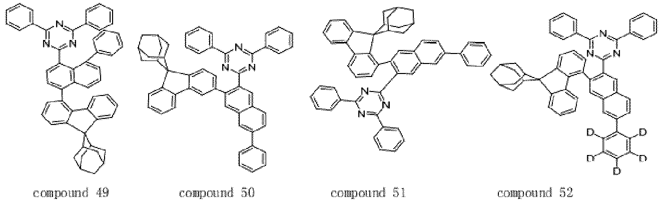

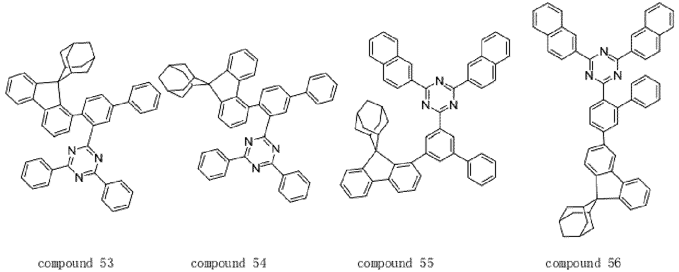

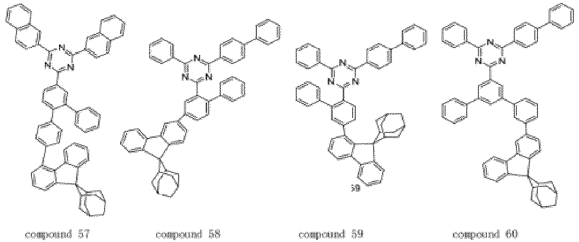

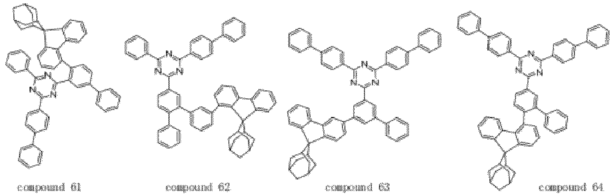

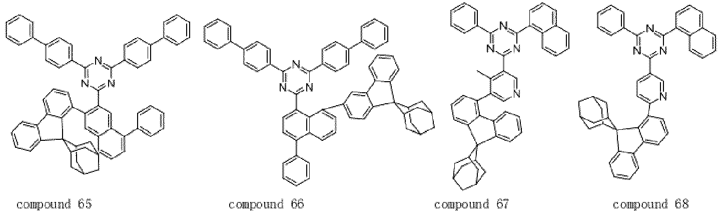

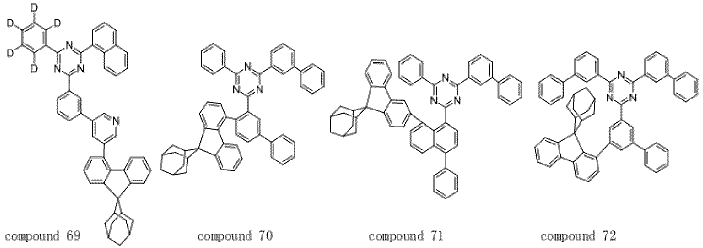

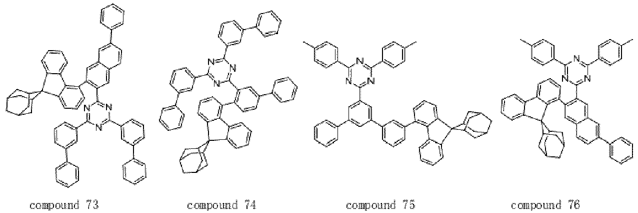

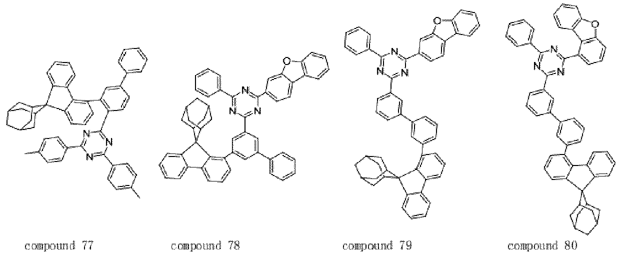

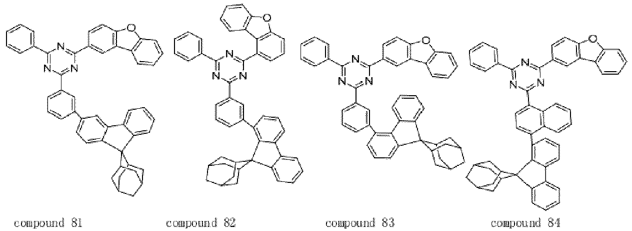

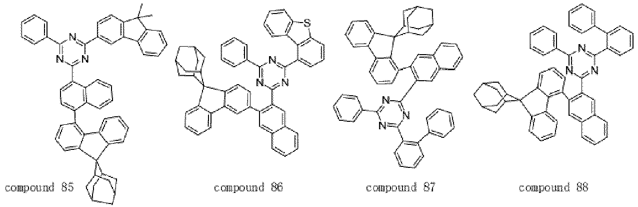

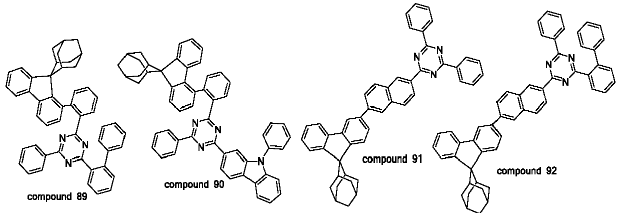

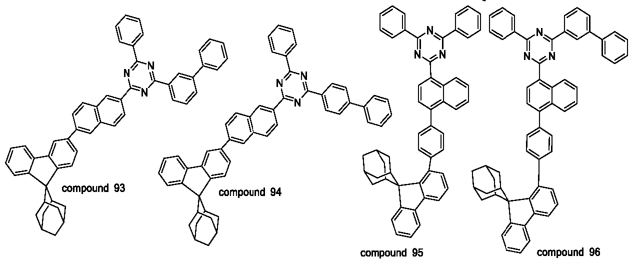

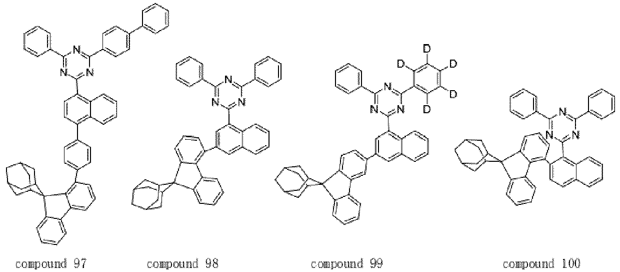

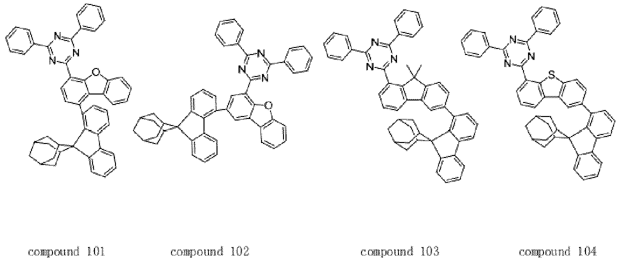

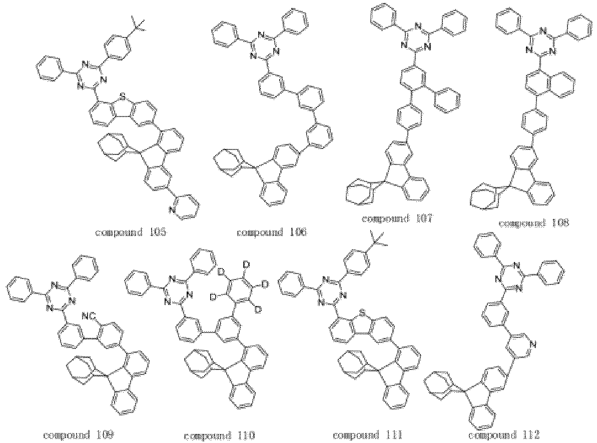

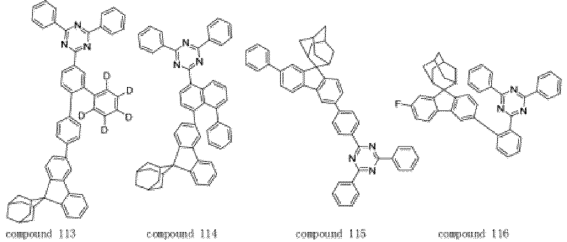

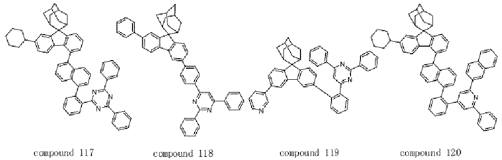

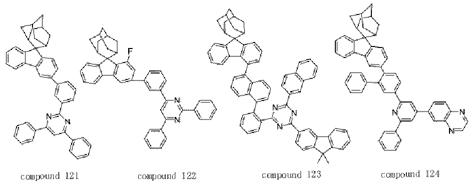

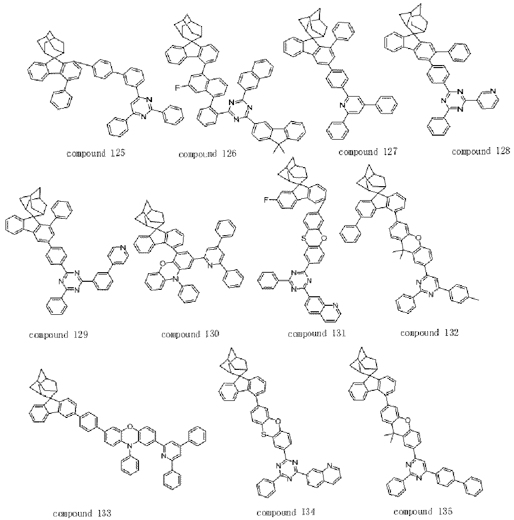

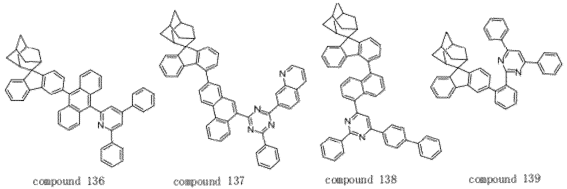

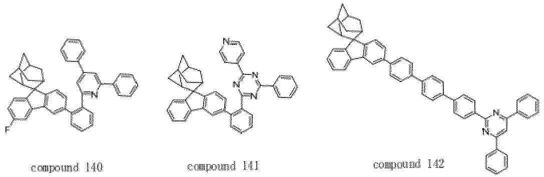

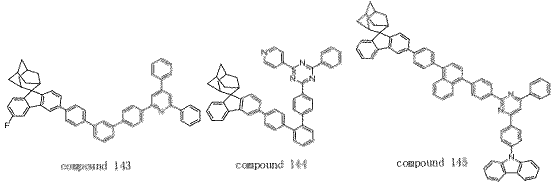

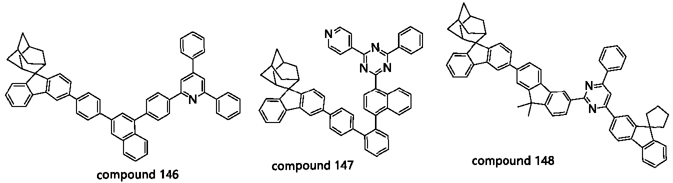

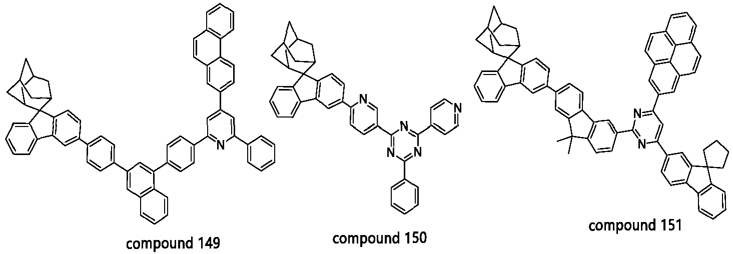

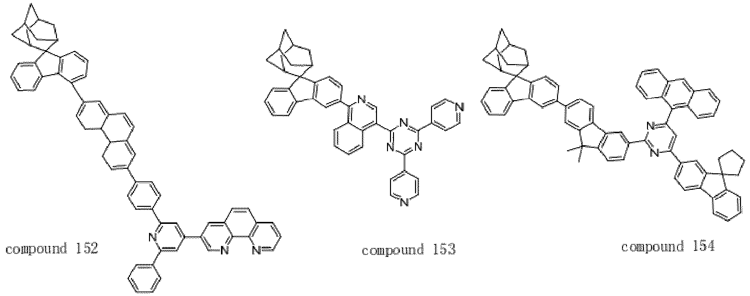

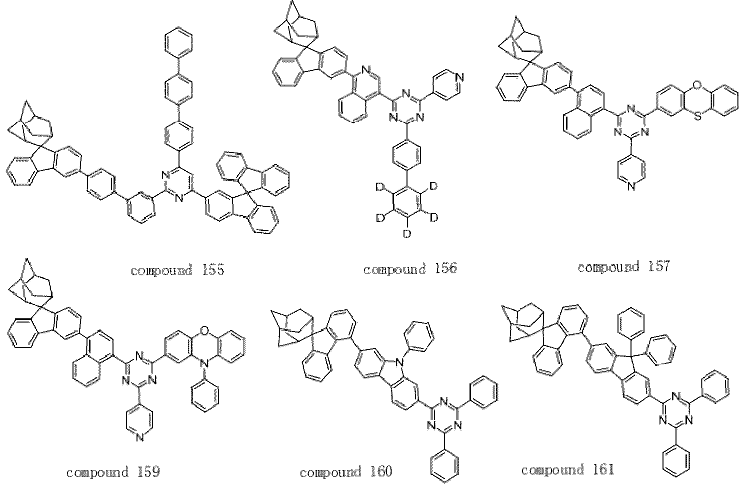

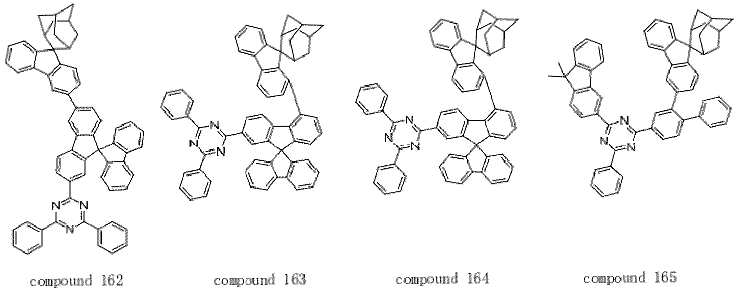

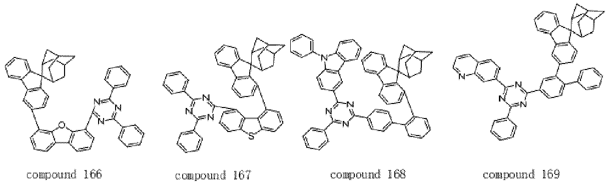

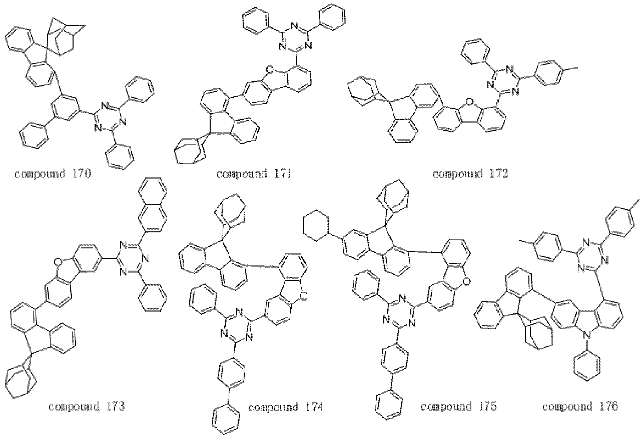

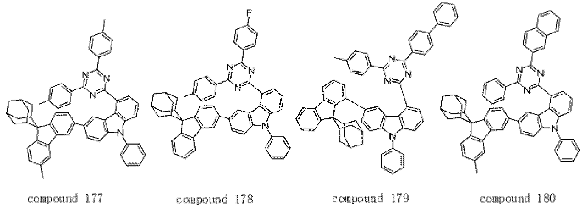

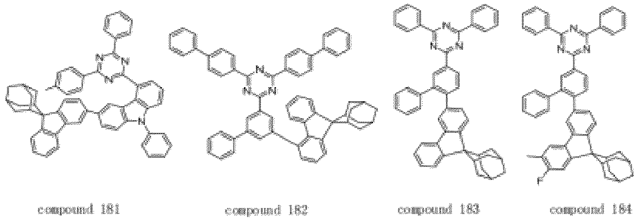

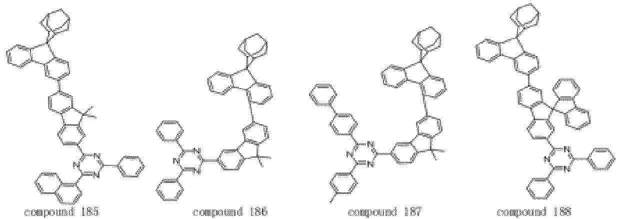

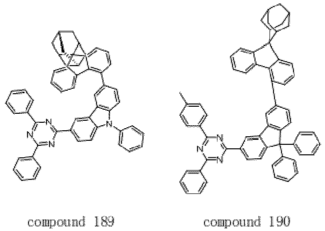

This present disclosure also provides an electronic element for implementing photoelectric conversion or electro-optical conversion. The electronic element includes an anode and a cathode disposed oppositely, and a functional layer disposed between the anode and the cathode; the functional layer contains the nitrogen-containing compound of the present disclosure.

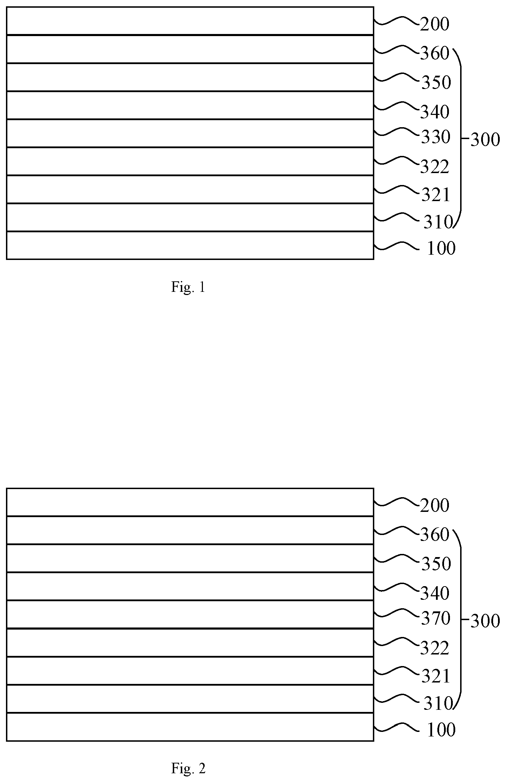

For example, the electronic element is an organic electroluminescent device. As shown in FIG. 1, the organic electroluminescent device includes an anode 100 and a cathode 200 disposed oppositely, and a functional layer 300 disposed between the anode 100 and the cathode 200; and the functional layer 300 contains the nitrogen-containing compound provided by the present disclosure.

Optionally, the functional layer 300 includes an electron transporting layer 350, and the electron transporting layer 350 contains the nitrogen-containing compound provided by the present disclosure. The electron transporting layer 350 may be composed of the nitrogen-containing compound provided by the present disclosure, or may be composed of the nitrogen-containing compound provided by the present disclosure and other materials.

Optionally, the electron transporting layer 350 also contains LiQ.

In one embodiment of the present disclosure, the organic electroluminescent device may include an anode 100, a hole transporting layer 321, an electron blocking layer 322, an organic electroluminescent layer 330 as the energy conversion layer, an electron transporting layer 350 and cathode 200 that are sequentially stacked.

Optionally, the organic electroluminescent device is a red light device.

Optionally, the anode 100 includes the anode material. Preferably, it is a material having a large work function that facilitates hole injection into the functional layer. Specific examples of anode materials include: metals such as nickel, platinum, vanadium, chromium, copper, zinc and gold, or their alloys; metal oxides such as zinc oxide, indium oxide, indium tin oxide (ITO) and indium zinc oxide (IZO); combination of metals and oxides such as ZnO:Al or SnO₂: Sb; or conductive polymers such as poly(3-methylthiophene), poly[3,4-(ethylene-1,2-dioxy)thiophene] (PEDT), polypyrrole and polyaniline, but not limited thereto. It preferably includes a transparent electrode containing indium tin oxide (ITO) as the anode.

Optionally, the hole transporting layer 321 may contian one or more hole transporting materials. The hole transporting material may be selected from carbazole polymers, carbazole-linked triarylamine compounds, or other types of compounds, which is not specially limited in the present disclosure. For example, the hole transporting layer 321 is composed of the compound TPD.

Optionally, the electron blocking layer 322 may contian one or more electron blocking materials. The electron blocking material may be selected from carbazole polymers or other types of compounds, which is not specially limited in the present disclosure. For example, the electron blocking layer 322 is composed of the compound TCTA.

Optionally, the organic light-emitting layer 330 may be composed of a single light-emitting material, and may also comprise a host material and a guest material. Alternatively, the organic light-emitting layer 330 is composed of a host material and a guest material. The holes injected into the organic light-emitting layer 330 and the electrons injected into the organic light-emitting layer 330 may combine in the organic light-emitting layer 330 to form excitons, and the excitons transfer energy to the host material, the host material transfers energy to the guest material, which in turn enables the guest material to emit light.

The host material of the organic light-emitting layer 330 may be a metal chelate compound, a bisstyryl derivative, an aromatic amine derivative, a dibenzofuran derivative, or other types of materials, which is not specially limited in the present disclosure. In an embodiment of the present disclosure, the host material of the organic light-emitting layer 330 may be CBP.

The guest material of the organic light-emitting layer 330 may be a compound having a condensed aryl ring or a derivative thereof, a compound having a heteroaryl ring or a derivative thereof, an aromatic amine derivative or other materials, which is not specially limited in the present disclosure. In an embodiment of the present disclosure, the guest material of the organic light-emitting layer 330 may be Ir(piq)₂(acac).

Optionally, the cathode 200 includes the cathode material, which is a material having a small work function that facilitates electron injection into the functional layer. Specific examples of cathode materials include: metals such as magnesium, calcium, sodium, potassium, titanium, indium, yttrium, lithium, gadolinium, aluminum, silver, tin and lead, or their alloys; or multilayer materials such as LiF/Al, Liq/ Al, LiO₂/Al, LiF/Ca, LiF/Al, and BaF₂/Ca, but not limited thereto. It is preferable to include a metal electrode containing silver and magnesium as the cathode.

Optionally, as shown in FIG. 1, a hole injecting layer 310 may also be provided between the anode 100 and the hole transporting layer 321 to enhance the ability to inject holes into the hole transporting layer 321. The hole injecting layer 310 may be selected from benzidine derivatives, starburst arylamine compounds, phthalocyanine derivatives, or other materials, which is not specially limited in the present disclosure. For example, the hole injecting layer 310 may be composed of HAT-CN.

Optionally, the hole transporting layer 321 may include one or more hole transporting materials, and the hole transporting materials may be selected from carbazole polymers, carbazole-linked triarylamine compounds, or other types of compounds, which is not specially limited in the present disclosure.. For example, in an embodiment of the present disclosure, the hole transporting layer 321 is composed of the compound TPD.

Optionally, the electron blocking layer 322 may contain one or more electron blocking materials. The electron blocking material may be selected from carbazole-linked triarylamine compounds, TCTA or other types of compounds, which is not specially limited in the present disclosure.

Optionally, as shown in FIG. 1, an electron injecting layer 360 may also be provided between the cathode 200 and the electron transporting layer 340 to enhance the ability to inject electrons into the electron transporting layer 350. The electron injecting layer 360 may include an inorganic material such as an alkali metal sulfide or an alkali metal halide, or may include a complex compound of an alkali metal and an organic compound. For example, the electron injecting layer 360 may include Ytterbium (Yb).

Optionally, a hole blocking layer 340 may be further provided between the organic electroluminescent layer 330 and the electron transporting layer 350.

For example, the electronic element may be a photoelectric conversion device. As shown in FIG. 2, the photoelectric conversion device may include an anode 100 and a cathode 200 disposed oppositely, and a functional layer 300 disposed between the anode 100 and the cathode 200; the functional layer 300 contains the nitrogen-containing compound provided in the present disclosure.

Optionally, the functional layer 300 includes an electron transporting layer 350, and the electron transporting layer 350 contains the nitrogen-containing compound provided by the present disclosure. The electron transporting layer 350 may be composed of the nitrogen-containing compound provided by the present disclosure, or may be composed of the nitrogen-containing compound provided by the present disclosure and other materials. Optionally, the electron transporting layer contains LiQ.

Optionally, as shown in FIG. 2, the photoelectric conversion device may include an anode 100, a hole transporting layer 321, an electron blocking layer 322, a photoelectric conversion layer 370 as the energy conversion layer, an electron transporting layer 350 and a cathode 200 that are sequentially stacked. The nitrogen-containing compound provided in the present disclosure may be applied to the electron transporting layer 350 of the photoelectric conversion device, which can effectively improve the luminous efficiency and lifetime of the photoelectric conversion device and increase the open circuit voltage of the photoelectric conversion device.

Optionally, a hole injecting layer 310 may also be provided between the anode 100 and the hole transporting layer 321.

Optionally, an electron injecting layer 360 may also be provided between the cathode 200 and the electron transporting layer 350.

Optionally, a hole blocking layer 340 may also be provided between the photoelectric conversion layer 370 and the electron transporting layer 350.

Optionally, the photoelectric conversion device may be a solar cell, especially an organic thin film solar cell. According to a specific embodiment, the solar cell includes an anode 100, a hole transporting layer 321, an electron blocking layer 322, a photoelectric conversion layer 370, an electron transporting layer 350 and a cathode 200 that are sequentially stacked, wherein the electron transporting layer 350 contains the nitrogen-containing compound of the present disclosure.

The present disclosure further provides an electronic device, which includes the electronic elements described in the present disclosure.

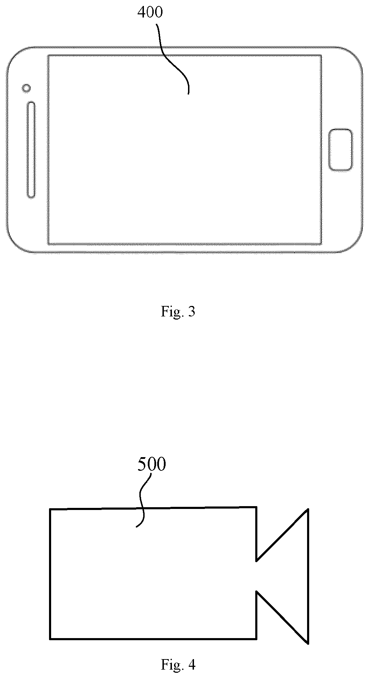

For example, as shown in FIG. 3, the electronic device provided in the present disclosure is a first electronic device 400, and the first electronic device 400 includes any of the organic electroluminescent devices described in the foregoing organic electroluminescent device embodiments. The electronic device may be a display device, a lighting device, an optical communication device or other types of electronic devices, for example, it may include, but is not limited to, a computer screen, a mobile phone screen, a television, an electronic paper, an emergency lighting lamp and an optical module, etc. Since the first electronic device 400 has the above organic electroluminescent device, it has the same beneficial effects, which will not be repeated here in this application.

For another example, as shown in FIG. 4, the electronic device is a second electronic device 500 comprising the above photoelectric conversion device. The second electronic device 500 may be a solar power generation device, a light detector, a fingerprint recognition device, an optical module, a CCD camera, or other types of electronic devices. Since the second electronic device has the above photoelectric conversion device, it has the same beneficial effects, which will not be repeated in this application.

Hereinafter, the present disclosure will be described in further detail through examples. However, the following examples are merely exemplary of the present disclosure, and do not limit the present disclosure.

In the following, unless otherwise specified, MC refers to dichloromethane, and rt refers to room temperature.

### Synthesis of compounds

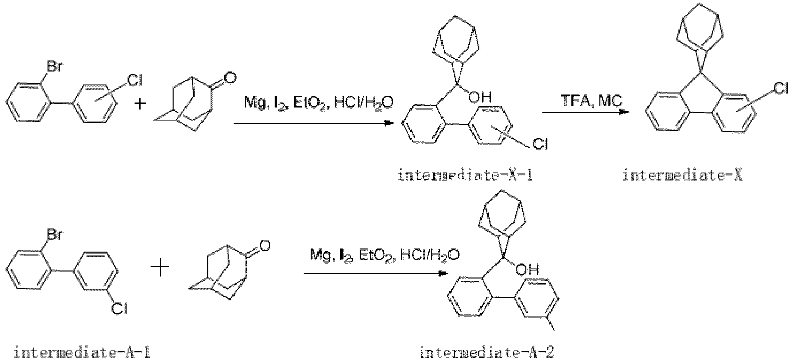

In a dry round-bottom flask, a magnesium bar (13.54 g, 564 mmol) and ether (100 mL) were placed under the protection of nitrogen gas, and iodine (100 mg) was added. Then, the solution of intermediate-A-1 (48.00 g, 187.0 mmol, CAS no. 154407-17-7) dissolved into diethyl ether (200 mL) was slowly dropped into to the flask. After dropped, the temperature was raised to 35°C and the reaction solution was stirred for 3 hours. The temperature of the reaction solution was lowered to 0°C, the solution of amantadone (22.45g, 149mmol) dissoved into diethyl ether (200 mL) was slowly added dropwise thereto. After the dropwise addition, the temperature was raised to 35°C, and stirred for 6 hours. Then, the reaction solution was cooled to room temperature, and 5wt% hydrochloric acid was added into the reaction solution until pH< 7, and stirring was carried out for 1 hour. Diethyl ether (200 mL) was added into the reaction solution for extraction. The organic phases were combined, dried with anhydrous magnesium sulfate, filtered, and the solvent was removed under reduced pressure. The crude product thus obtained was purified by silica gel column chromatography using n-heptane as the mobile phase to obtain a solid intermediate-A-2 (42g, yield 84%).

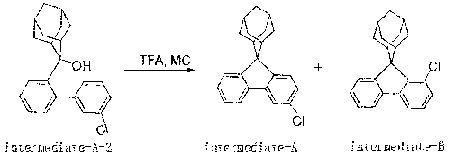

In a round-bottom flask, intermediate-A-2 (42, 123.9mmol), trifluoroacetic acid (42.29g, 371.8mmol) and dichloromethane (500mL) were added, and stirred for 2 hours under the protection of nitrogen gas. Then, an aqueous solution of sodium hydroxide was added into the reaction solution until pH=8, followed by liquid separation, the organic phase was dried with anhydrous magnesium sulfate, filtered, and the solvent was removed under reduced pressure. The crude product thus obtained was purified by silica gel column chromatography using dichloromethane/n-heptane (1:2) to obtain intermediate-A as a white solid (18.50g, yield 46%) and intermediate-B (16.0g, yield 40%).

### NMR data of Intermediate-A :

¹HNMR(400MHz, CD₂Cl₂): 8.11(d,1H), 8.03(d,1H), 7.41-7.63(m,2H), 7.37-7.39(m,1H), 7.30-7.33(m,1H), 7.23-7.24(m,1H), 2.88-2.93(m,2H), 2.81-2.85(m,2H), 2.19(s,2H), 1.99(s,2H), 1.77-1.83(m,4H), 1.54(s,2H).

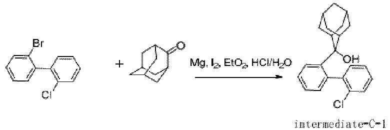

In a dry round-bottom flask, a magnesium bar (13.54 g, 564 mmol) and ether (100 mL) were placed under the protection of nitrogen gas, and iodine (100 mg) was added. Then, 2'-bromo-2-chlorobiphenyl (50.00g, 187.0mmol) dissoved into diethyl ether (200mL) was slowly dropped into to the flask. After dropped, the temperature was raised to 35°C and the reaction sloution was stirred for 3 hours. The temperature of the reaction solution was lowered to 0°C, the solution of amantadone (22.45g, 149mmol) dissolved into diethyl ether (200mL) was slowly added dropwise thereto. After the dropwise addition, the temperature was raised to 35°C, and stirred for 6 hours. Then, the reaction solution was cooled to room temperature, and 5wt% hydrochloric acid was added into the reaction sloution until pH< 7, and the stirring was carried out for 1 hour. Diethyl ether (200 mL) was added into the reaction solution for extraction. The organic phases were combined, dried with anhydrous magnesium sulfate, filtered, and the solvent was removed under reduced pressure. The crude product thus obtained was purified by silica gel column chromatography using ethyl acetate/n-heptane (1:2) as the mobile phase to obtain a white solid intermediate-C-1 (43g, yield 68%).

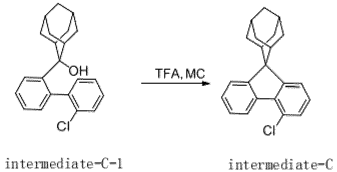

In a round-bottom flask, intermediate-C-1 (43, 126.9mmol), trifluoroacetic acid (36.93, 380.6mmol) and dichloromethane (300mL) were added, and stirred for 2 hours under the protection of nitrogen gas. Then, an aqueous solution of sodium hydroxide was added into the reaction solution until pH=8, followed by liquid separation, the organic phase was dried with anhydrous magnesium sulfate, filtered, and the solvent was removed under reduced pressure. The crude product thus obtained was purified by silica gel column chromatography using dichloromethane/n-heptane (1:2) to obtain intermediate-C as a white solid (39.2, yield 96.3%).

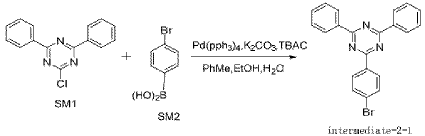

In a three-necked flask, SM1 (30g, 112.05mmol), SM2 (22.50g, 112.05mmol), tetrakis(triphenylphosphine) palladium (6.47g, 5.60mmol), potassium carbonate (46.39g, 336.7mmol), tetrabutylammonium chloride (1.56g, 5.60mmol), toluene (240mL), ethanol (120mL) and deionized water (60mL) were added, the reaction solution was heated to 78°C under the protection of nitrogen gas, and stirred for 8 hours; the reaction solution was cooled to room temperature, toluene (150 mL) was added for extraction, the organic phases were combined, the combined organic phase was dried with anhydrous magnesium sulfate, filtered to obtain a filtrate, and the filtrate was concentrated under reduced pressure to obtain a crude product; the crude product thus obtained was purified by silica gel column chromatography using n-heptane as the mobile phase, and then recrystallized and purified with a dichloromethane/ethyl acetate system (1:3) to obtain intermediate-2-1 (34.8g, yield 80%) ).

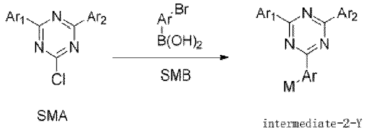

In one embodiment, intermediate-2-Y as listed in Table 1 below (see Table 1 for M and Ar) was synthesized with reference to the synthesis method for intermediate-2-1, Y can be 2, 3, 4, 5, 6, 7, 8, 9 to 29, etc., the difference was that a different compound SMA was used instead of SM1 for the preparation of intermediate-2-1, and a different compound SMB was used instead of SM2 for the preparation of intermediate-2-1. For example, the compound SMA may be SM1, 2-chloro-4,6-di(naphthalen-2-yl)-1,3,5-triazine, 2-(4-biphenyl)-4-chloro- 6-benzene-1,3,5-triazine, 2,4-bis((1,1'-biphenyl)-4-yl)-6-chloro-1,3,5-triazine, 2-chloro -4-(1-naphthyl)-6-phenyl-1,3,5-triazine, 2-((1,1'-biphenyl)-3-yl)-4-chloro-6-phenyl-1,3,5-triazine, 2-chloro-4,6-di-p-tolyl-1,3,5-triazine, but not limited thereto. The compound SMB can be 3'-bromo-3-biphenylboronic acid, 3-bromophenylboronic acid, 4-bromophenylboronic acid, 4-bromo-1-naphthylboronic acid, 4'-bromo-4-biphenylboronic acid, (3 -bromonaphthalene-2-yl)boronic acid, 2-bromo-phenylboronic acid, (8-bromonaphthalene-2-yl)boronic acid, intermediate-1-B, intermediate-1-C, 5-chloro-3-phenylphenylboronic acid, 7-bromo-9,9-dimethylfluorene-2-boronic acid, etc., but not limited thereto.

### Synthesis of intermediate 1-B:

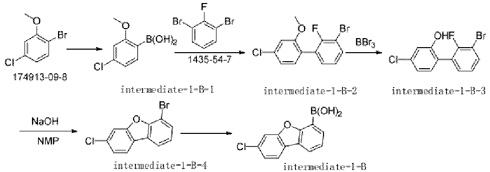

A three-necked flask containing THF (200ml) was charged with 2-bromo-5-chloroanisole (20g, 90.33mmol), and n-butyl lithium (6.07g, 94.85mmol) was added dropwise at -80°C. After addition, the temperature of the reaction solution was maintained for 1 hour, and then trimethyl borate (14.07g, 135.5mmol) was added dropwise.. The temperature of the reaction solution was maintained for 1 hour, then raised to room temperature, and the reaction solution was stirred overnight. Hydrochloric acid (2mol/L) was added into the reaction solution to adjust the pH to neutral, and then filtered to obtain a white crude product, which was beaten with n-heptane to obtain intermediate-1-B-1 as a white solid (11.7g, yield 70%).

In a round-bottom flask, intermediate-1-B-1 (11.0g, 59.04mmol), 1,3-dibromo-2-fluorobenzene (14.98g, 59.04mmol), tetrakis(triphenylphosphine)palladium (3.41g, 2.95mmol), potassium carbonate (16.32g, 118.09mmol), tetrabutylammonium chloride (0.67g, 2.95mmol), toluene (90mL), ethanol (50mL) and deionized water (30mL) were added, the temperature was raised to 78°C under the protection of nitrogen gas, and stirred for 8 hours; the reaction solution was cooled to room temperature, toluene (300 mL) was added for extraction, the organic phases were combined, dried with anhydrous magnesium sulfate, filtered, and the solvent was removed under reduced pressure; the crude product thus obtained was purified by silica gel column chromatography using n-heptane as the mobile phase, and then recrystallized and purified with a dichloromethane/ethyl acetate (3:1) system to obtain intermediate-1-B-2 (14.08g, yield 76%).

Intermediate-1-B-2 (14.0g, 44.37mmol) and dichloromethane (30mL) were stirred at room temperature under the protection of nitrogen gas for 10min, then the temperature was lowered to 0°C, and a dichloromethane solution of boron tribromide (16.67g, 66.56mmol) was added, kept at 0°C for 20min, and the temperature was raised to room temperature to react for 3 hours. After the reaction was complete, the reaction solution was inactivated with methanol (30 mL) at -78°C, and then water (30 mL) was added to concentrate and dried to obtain intermediate-1-B-3 (10.70 g, yield 80%).

Intermediate-1-B-3 (10.7 g, 35.48 mmol), sodium hydroxide (2.13 g, 52.23 mmol), and NMP (100 mL) were added into a flask, the reaction solution was heated to reflux for 3 hours, and the reaction solution was concentrated and dried to obtain a crude product. The crude product thus obtained was purified by silica gel column chromatography using n-heptane as the mobile phase. The product was then recrystallized and purified with a dichloromethane/n-heptane (1:3) system to obtain intermediate-1-B-4 (7.99g, yield 80%).

A three-necked flask containing THF (200ml) was charged with intermediate-1-B-4 (7.99g, 28.38mmol), followed by dropwise addition of n-butyllithium (1.90g, 29.80mmol) at -80°C. After addition, the temperature of the reaction solution was maintained for 1 hour and then trimethyl borate (4.42 g, 42.57 mmol) was added dropwise. The temperature of the reaction solution was maintained for 1 hour, then raised to room temperature, and the reaction solution was stirred overnight. Hydrochloric acid (2mol/L) was added into the reaction solution to adjust the pH to neutral, and then filtered to obtain a white crude product, which was beaten with n-heptane to obtain intermediate-1-B as a white solid(4.89g, yield 70%).

### Synthesis of intermediate 1-C:

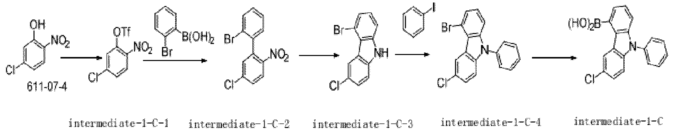

2-Nitro-5-chlorophenol (20.0g, 115.24mmol) and dichloromethane (30mL) were stirred at room temperature for 10min under the protection of nitrogen gas, then pyridine (27.34g, 345.7mmol) was added, and cooled to -5°C, followed by addition of trifluoromethane sulfonic anhydride (48.77g, 172.86mmol), the reaction solution was kept at -5°C for 20min, raised to room temperature to react for 2 hours. After the reaction was complete, hydrochloric acid (2mol/L) was added into the the reaction solution to adjust the pH to neutral, and beaten with n-heptane to obtain intermediate-1-C-1 as a white solid (26.41g, yield 75%).

In a round-bottom flask, intermediate-1-C-1 (26g, 85.07mmol), o-bromophenylboronic acid (17.08 g, 85.07mmol), tetrakis(triphenylphosphine)palladium (4.92g, 4.25mmol), potassium carbonate (23.51g, 170.15mmol), tetrabutylammonium chloride (0.97g, 4.25mmol), toluene (240mL), ethanol (120mL) and deionized water (50mL) were added, the temperature was raised to 78°C under the protection of nitrogen gas, and stirred for 10 hours; the reaction solution was cooled to room temperature, toluene (300 mL) was added for extraction, the organic phases were combined, dried with anhydrous magnesium sulfate, filtered, and the solvent was removed under reduced pressure; the crude product thus obtained was purified by silica gel column chromatography using n-heptane as the mobile phase, and then recrystallized and purified with a dichloromethane/ethyl acetate (3:1) system to obtain intermediate-1-C-2 (14.08g, yield 53%).

Intermediate-1-C-2 (14.08, 45.05mmol), triphenylphosphine (42.26g, 180.2mmol), o-dichlorobenzene (150mL) were added into a flask, the reaction solution was heated to reflux at 180°C for 3 hours. The reaction solution was then concentrated and dried to obtain a crude product. The crude product thus obtained was purified by silica gel column chromatography using dichloromethane/ethyl acetate (1:3) as the mobile phase to obtain intermediate-1-C-3 (9.86g, yield 78%).

Intermediate-1-C-3 (14.08g, 35.15mmol), iodobenzene (7.31g, 35.85mmol), cuprous iodide (1.34g, 7.03mmol), potassium carbonate (10.68g, 77.33mmol), 1,10-phenanthroline (2.53g, 14.06mmol), 18-crown ether-6 (0.93g, 3.52mmol), N,N-dimethylformamide (140mL) were added into a flask, the reaction solution was heated to reflux (130°C) for 10 hours to react. The reaction solution was cooled to room temperature, dichloromethane (300 mL) was added for extraction, the organic phases were combined, dried with anhydrous magnesium sulfate, filtered, and the solvent was removed under reduced pressure; the crude product thus obtained was purified by silica gel column chromatography using n-heptane as the mobile phase, and then recrystallized and purified with a dichloromethane/ethyl acetate (3:1) system to obtain intermediate-1-C-4 (10.02g, yield 80%).

A three-necked flask containing THF (10ml) was charged with intermediate-1-C-4 (10.0g, 27.35mmol), and n-butyllithium (2.39g, 37.42mmol) was added dropwise at -80°C. After the addition, the temperature of the reaction solution was maintained for 1 hour and then trimethyl borate (5.55 g,53.46mmol) was added dropwise. The temperature of the reaction solution was maintained for 1 hour, then raised to room temperature, and the reaction solution was stirred overnight. Hydrochloric acid (2mol/L) was added into the reaction solution to adjust the pH to neutral, and then filtered to obtain a white crude product, which was beaten with n-heptane to obtain intermediate-1-C as a white solid (6.12g, yield 70%).

The combination of each compound SMA and SMB can produce its uniquely corresponding intermediate-2-Y. The intermediate-2-Y thus prepared is shown in Table 1.

**Table 1: Structural formula, mass and yield of intermediate 2-Y**

| SMA | SMB | intermediate-2-Y | mass (g) | yield (%) |
|---|---|---|---|---|
| 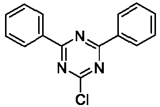 | 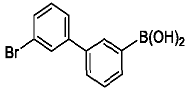 | 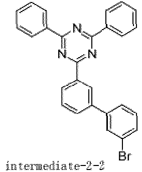 | 39.02 | 75 |
| | 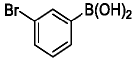 | 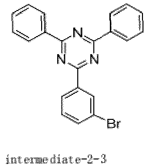 | 33.06 | 76 |
| | 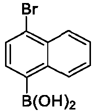 | 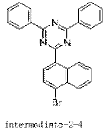 | 34.86 | 71 |
| | 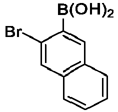 | 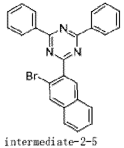 | 35.85 | 73 |
| | 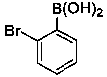 | 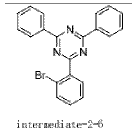 | 33.06 | 76 |
| 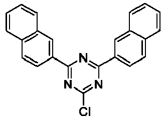 | 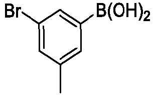 | 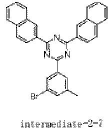 | 29.87 | 75 |
| | 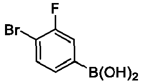 | 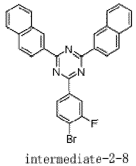 | 29.00 | 75 |
| | 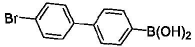 | 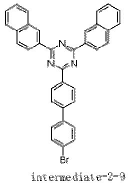 | 33.60 | 73 |
| 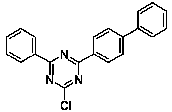 | 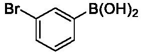 | 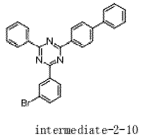 | 29.57 | 73 |
| | 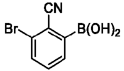 | 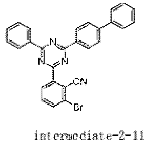 | 30.38 | 75 |
| | 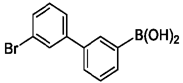 | 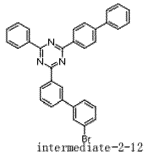 | 34.41 | 72 |
| | 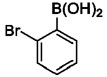 | 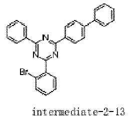 | 29.16 | 72 |
| | 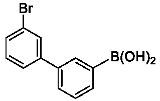 | 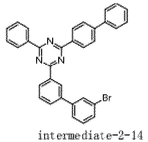 | 33.47 | 71 |
| 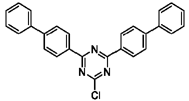 | 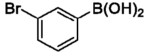 | 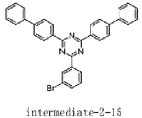 | 28.18 | 73 |
| | 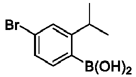 | 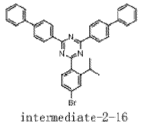 | 28.95 | 75 |
| | 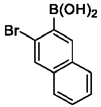 | 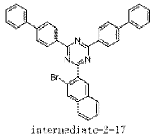 | 29.94 | 71 |
| | 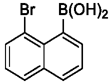 | 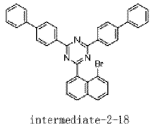 | 29.52 | 70 |
| 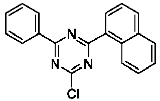 | 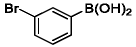 | 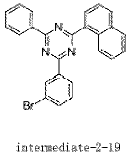 | 29.79 | 72 |
| | 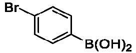 | 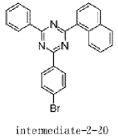 | 30.20 | 73 |
| | 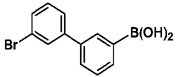 | 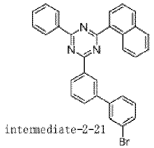 | 34.96 | 72 |
| 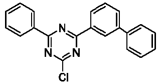 | 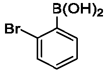 | 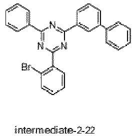 | 29.57 | 73 |
| | 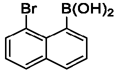 | 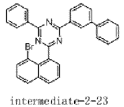 | 31.41 | 70 |
| 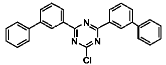 | 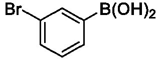 | 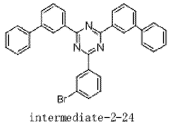 | 28.18 | 73 |
| | 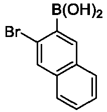 | 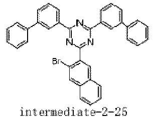 | 29.52 | 70 |
| | 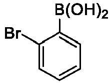 | 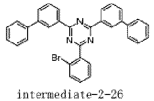 | 27.02 | 70 |
| 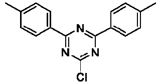 | 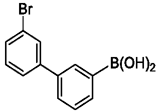 | 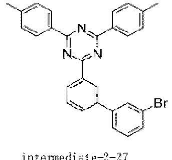 | 34.95 | 70 |
| | 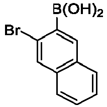 | 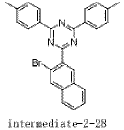 | 34.05 | 72 |
| 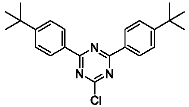 | 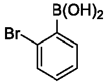 | 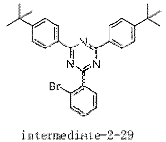 | 29.55 | 70 |
| 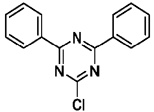 | 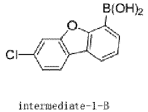 | 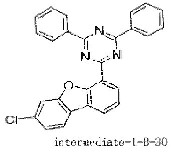 | 28.35 | 71 |
| 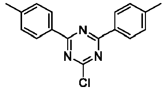 | 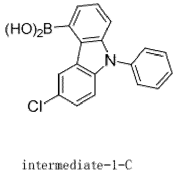 | 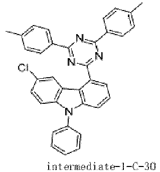 | 27.35 | 72 |
| 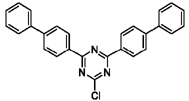 | 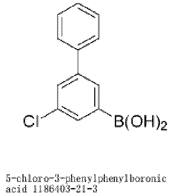 | 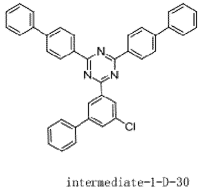 | 27.03 | 72 |
| 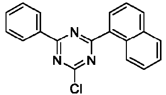 | 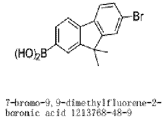 | 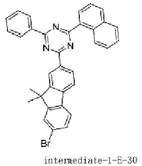 | 26.93 | 71 |

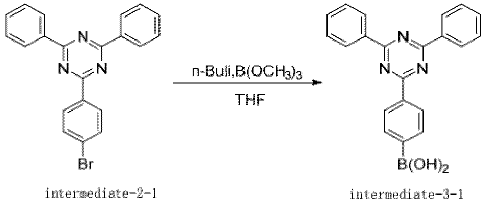

A three-necked flask containing THF (1L) was charged with intermediate-2-1 (20g, 51.51mmol), and n-butyllithium (3.46g, 4.08mmol) was added dropwise at -78°C. After the addition, the temperature of the reaction solution was maintained for 1 hour and then trimethyl borate (8.02g,77.26mmol) was added dropwise. The temperature of the reaction solution was maintained for 1 hour, then raised to room temperature, and the reaction solution was stirred overnight. Hydrochloric acid (2mol/L) was added into the reaction solution to adjust the pH to neutral, and then filtered to obtain a white crude product, which was beaten with n-heptane to obtain intermediate-3-1 as a white solid (11.82g, yield 65%).

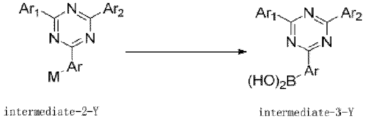

In one embodiment, the intermediate-3-Y was synthesized with reference to the synthesis method of intermediate-3-1, with the difference that different intermediate-2-Y was used instead of intermediate-2-1, and each intermediate-2-Y can produce its unique intermediate-3-Y. The thus prepared intermediate-3-Y was shown in Table 2.

**Table 2: Structural formula, mass and yield of intermediate 3-Y**

| intermediate-2-Y | intermediate-3-Y | mass (g) | yield (%) |
|---|---|---|---|
| 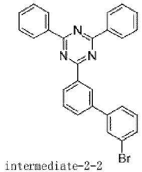 | 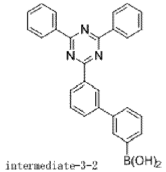 | 13.56 | 63 |
| 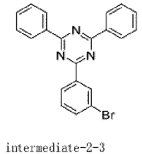 | 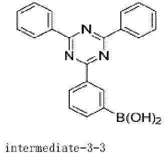 | 10.35 | 64 |
| 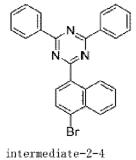 | 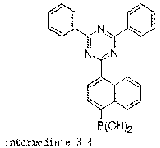 | 13.40 | 65 |
| 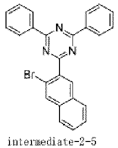 | 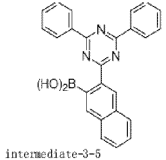 | 11.28 | 63 |
| 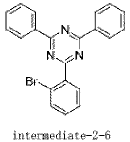 | 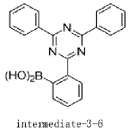 | 11.82 | 65 |
| 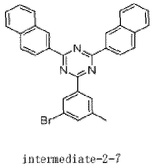 | 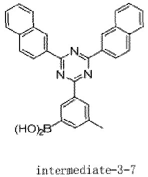 | 11.69 | 63 |
| 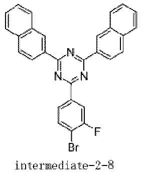 | 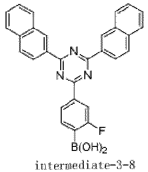 | 12.06 | 65 |
| 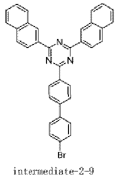 | 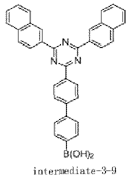 | 11.81 | 63 |
| 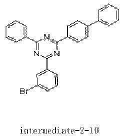 | 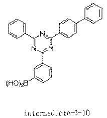 | 11.64 | 63 |
| 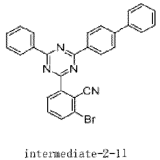 | 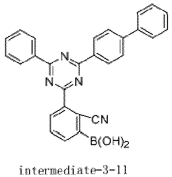 | 12.01 | 65 |
| 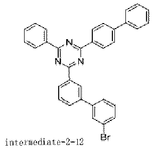 | 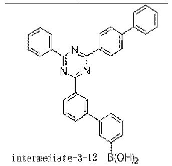 | 11.22 | 60 |
| 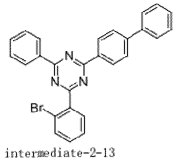 | 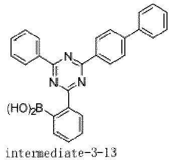 | 11.83 | 64 |
| 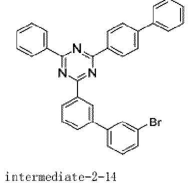 | 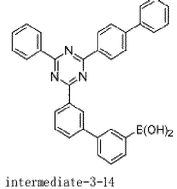 | 11.96 | 64 |
| 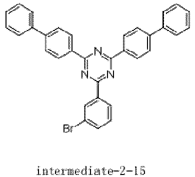 | 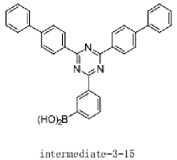 | 11.22 | 60 |
| 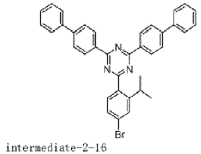 | 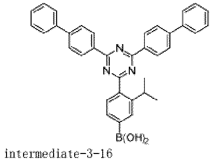 | 11.78 | 63 |
| 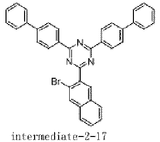 | 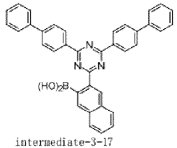 | 11.76 | 63 |
| 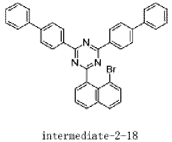 | 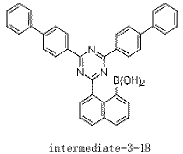 | 14.20 | 65 |
| 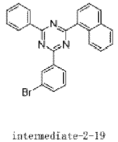 | 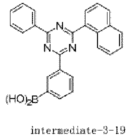 | 11.96 | 65 |
| 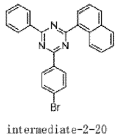 | 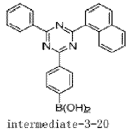 | 11.59 | 63 |
| 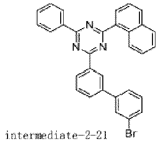 | 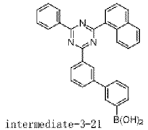 | 11.55 | 62 |
| 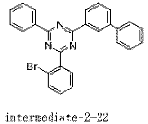 | 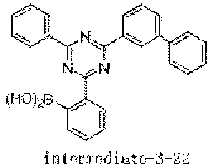 | 12.01 | 62 |
| 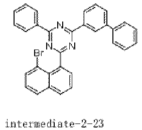 | 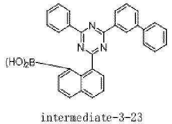 | 11.74 | 63 |
| 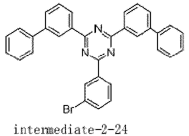 | 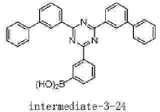 | 12.15 | 65 |
| 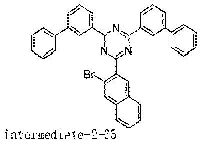 | 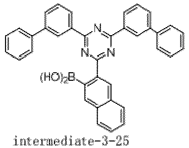 | 11.85 | 63 |
| 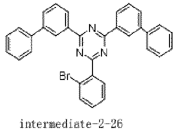 | 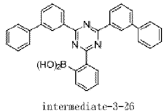 | 11.78 | 63 |
| 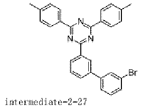 | 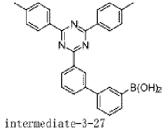 | 12.07 | 65 |
| 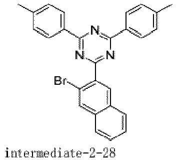 | 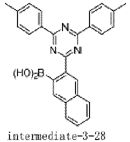 | 11.65 | 63 |
| 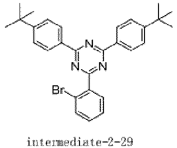 | 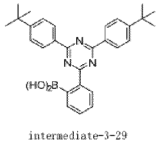 | 11.35 | 62 |
| 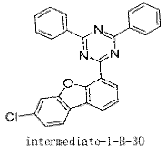 | 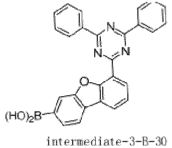 | 10.52 | 60 |
| 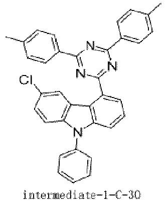 | 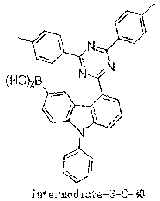 | 11.32 | 61 |
| 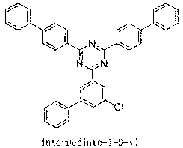 | 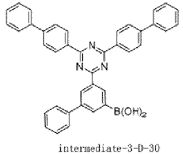 | 10.32 | 63 |
| 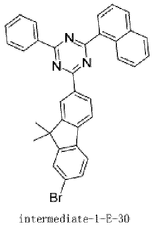 | 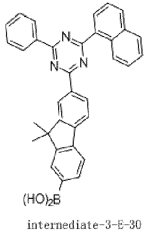 | 9.88 | 60 |

### Synthesis of compound 1

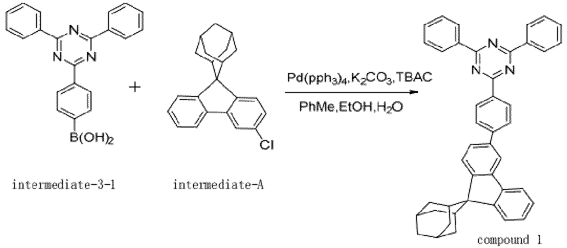

In a three-necked flask, intermediate-3-1 (10g,28.31mmol), intermediate-A (9.08g,28.31mmol), tetrakis(triphenylphosphine)palladium (1.63g, 1.41mmol), potassium carbonate (11.72g, 84.84mmol), tetrabutylammonium chloride (0.39g, 1.41mmol), toluene (80mL), ethanol (40mL) and deionized water (20mL) were added, the reaction resolution was heated to 78°C under nitrogen the protection of nitrogen gas, and stirred for 8 hours; the reaction solution was cooled to room temperature, toluene (150 mL) was added for extraction, the organic phases were combined, the combined organic phase was dried with anhydrous magnesium sulfate, filtered to obtain the filtrate, and the filtrate was concentrated under reduced pressure to obtain the crude product; the crude product thus obtained was purified by silica gel column chromatography using n-heptane as the mobile phase, and then recrystallized and purified with a dichloromethane/ethyl acetate system (1:4) to obtain compound 1 (13.95g, yield: 83%), m/z=594.3 [M+H]⁺.

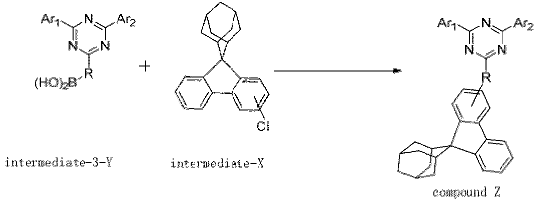

In one embodiment, compound Z listed in Table 3 was synthesized with reference to the method for compound 1, with the difference was that intermediate-3-Y was used instead of intermediate-3-1, and intermediate-X was used instead of intermediate-A, and each combination of intermediate-3-Y and intermediate-X produce the unique compound Z as shown in Table 3.

**Table 3 Structure, preparation and characterization data of compounds**

| Intermediate-3-Y | Intermediate-X | Compound Z | mass (g) | yield (%) | Mass spectrum (m/z) |
|---|---|---|---|---|---|
| 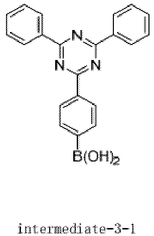 | 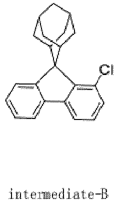 | 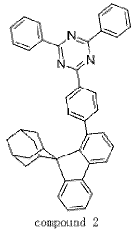 | 13.44 | 80 | 593.2 |
| | 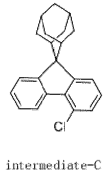 | 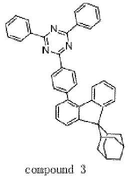 | 13.61 | 81 | 593.2 |
| 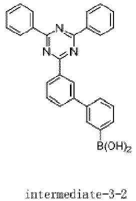 | 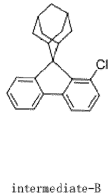 | 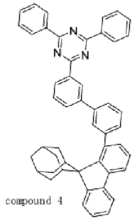 | 12.62 | 81 | 669.3 |
| | 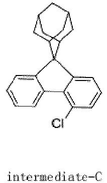 | 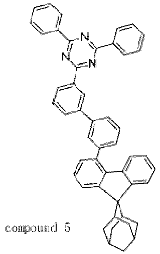 | 12.94 | 83 | 669.3 |
| 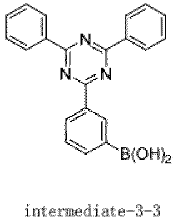 | 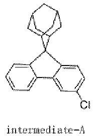 | 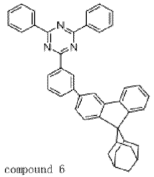 | 13.44 | 80 | 593.2 |
| | 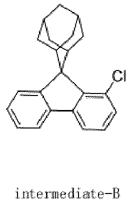 | 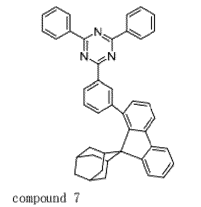 | 13.61 | 81 | 593.2 |
| | 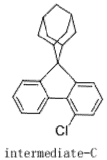 | 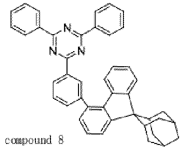 | 13.44 | 80 | 593.2 |
| 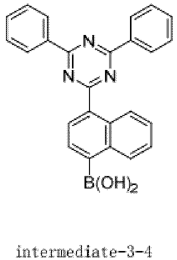 | 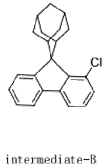 | 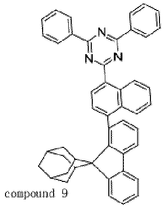 | 12.77 | 80 | 643.3 |
| | 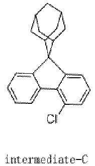 | 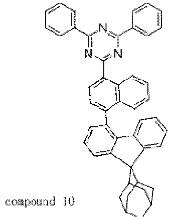 | 12.93 | 81 | 643.3 |
| 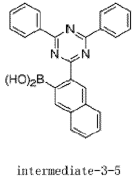 | 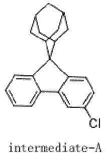 | 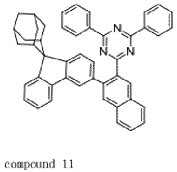 | 13.09 | 82 | 643.3 |
| | 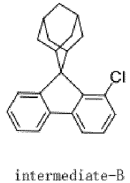 | 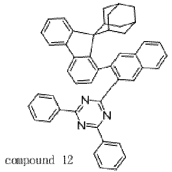 | 12.93 | 81 | 643.3 |
| | 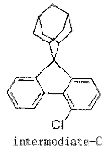 | 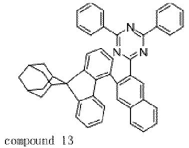 | 12.77 | 80 | 643.3 |
| 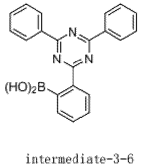 | 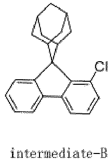 | 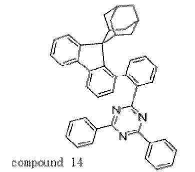 | 13.94 | 83 | 593.2 |
| | 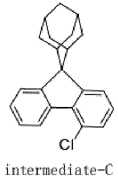 | 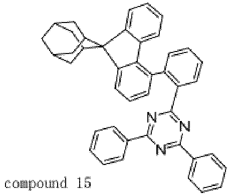 | 13.60 | 81 | 593.2 |
| 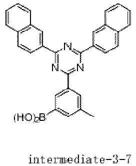 | 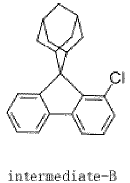 | 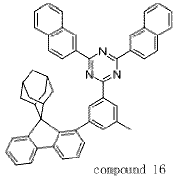 | 12.70 | 83 | 707.3 |
| 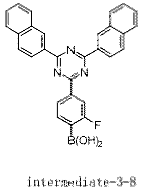 | 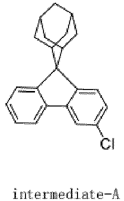 | 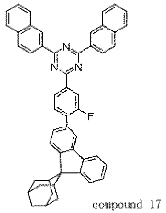 | 12.69 | 83 | 711.3 |
| 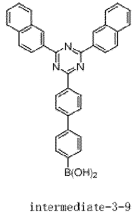 | 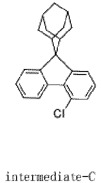 | 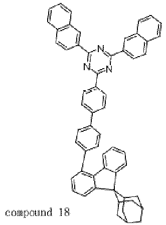 | 11.63 | 80 | 769.3 |
| 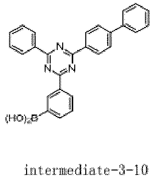 | 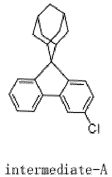 | 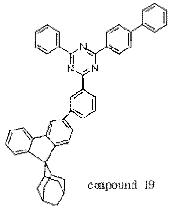 | 12.61 | 81 | 669.3 |
| 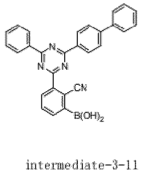 | 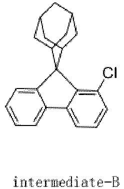 | 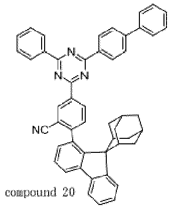 | 12.94 | 83 | 694.3 |
| 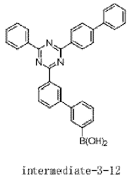 | 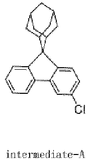 | 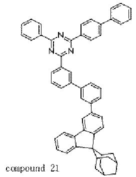 | 11.79 | 80 | 745.3 |
| 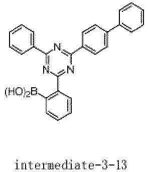 | 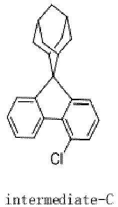 | 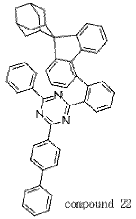 | 12.47 | 80 | 669.3 |
| 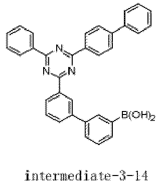 | 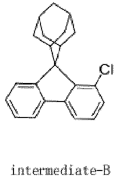 | 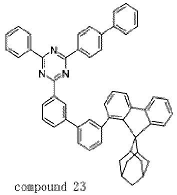 | 11.94 | 81 | 745.3 |
| 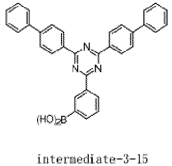 | 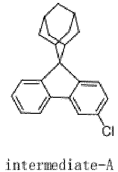 | 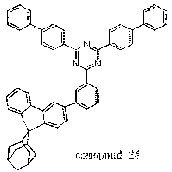 | 12.24 | 83 | 745.3 |
| 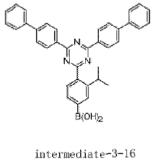 | 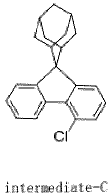 | 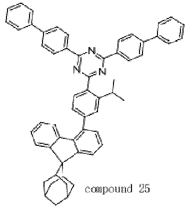 | 10.86 | 80 | 787.3 |
| 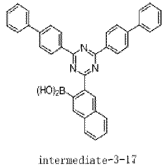 | 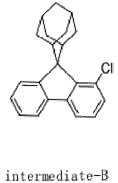 | 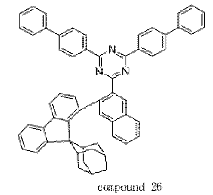 | 11.59 | 81 | 795.3 |
| 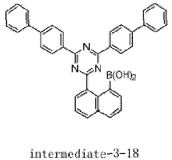 | 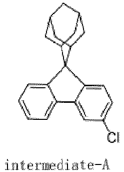 | 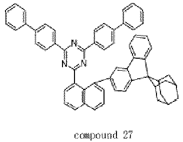 | 11.45 | 80 | 795.3 |
| 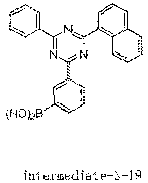 | 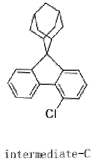 | 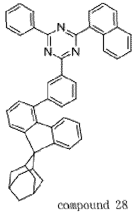 | 12.92 | 81 | 643.3 |
| 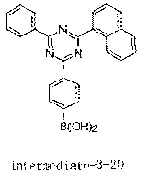 | 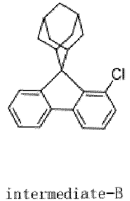 | 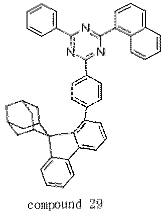 | 12.76 | 80 | 643.3 |
| 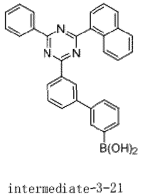 | 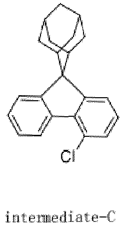 | 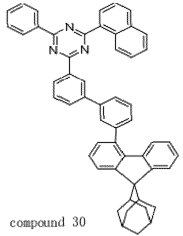 | 12.00 | 80 | 719.3 |
| 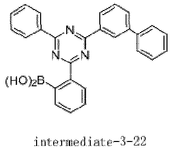 | 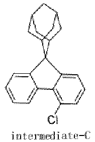 | 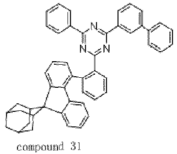 | 12.62 | 81 | 669.3 |
| 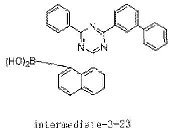 | 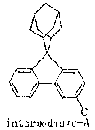 | 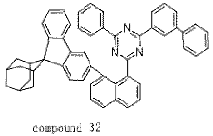 | 12.15 | 81 | 719.3 |
| 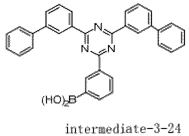 | 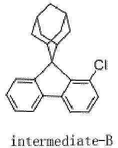 | 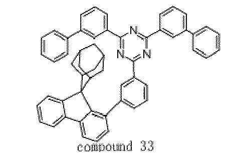 | 11.94 | 81 | 745.3 |
| 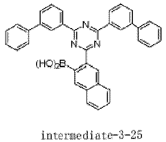 | 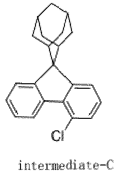 | 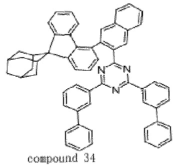 | 11.45 | 80 | 795.3 |
| 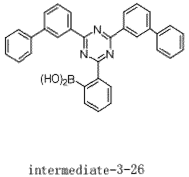 | 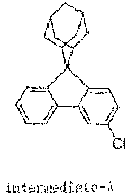 | 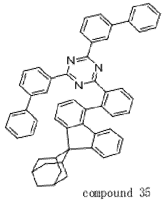 | 11.94 | 81 | 745.3 |
| 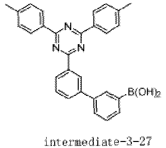 | 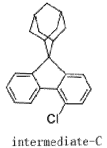 | 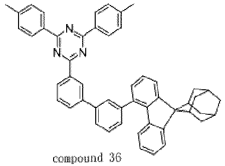 | 12.66 | 83 | 697.3 |
| 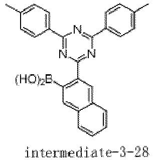 | 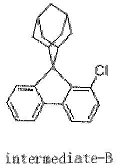 | 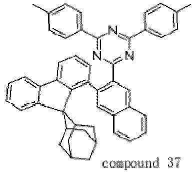 | 13.48 | 83 | 671.3 |
| 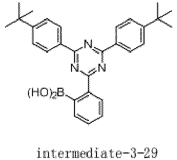 | 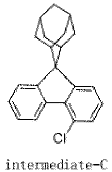 | 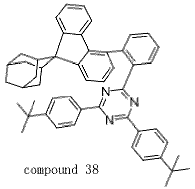 | 13.03 | 80 | 705.4 |
| 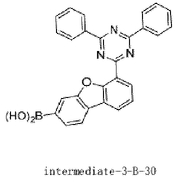 | 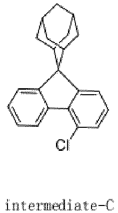 | 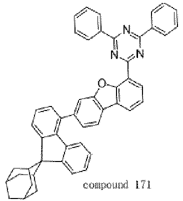 | 12.38 | 80 | 684.2 |
| 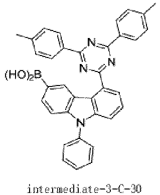 | 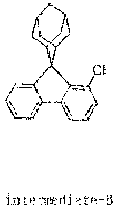 | 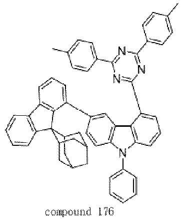 | 13.02 | 81 | 787.3 |
| 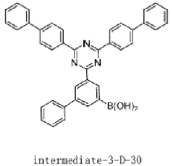 | 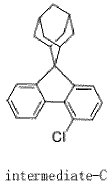 | 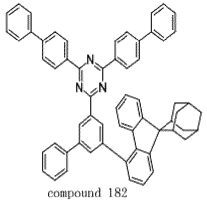 | 12.89 | 80 | 822.3 |
| 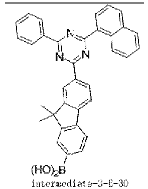 | 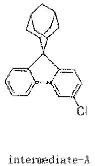 | 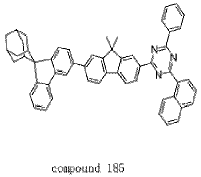 | 11.56 | 80 | 760.3 |

### Synthesis of compound 39:

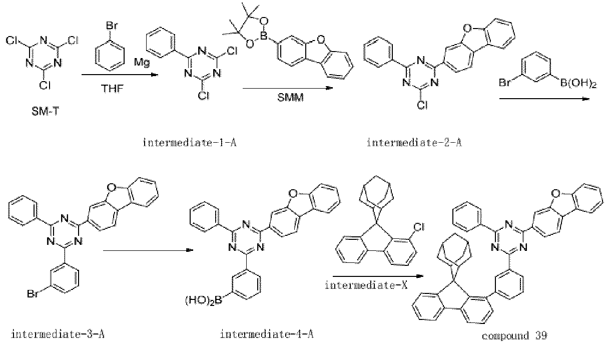

### Preparation of Intermediate-1-A:

In a 3-L reaction flask, 2,4,6-trichloro-1,3,5-triazine (100g, 542.27mmol) and 800mL of anhydrous tetrahydrofuran were added, and the reaction solution was stirred at 0°C under nitrogen. 97.93mL (1 mol/L) phenyl magnesium bromide (which can be obtained by the reaction of bromobenzene and metallic magnesium) was added into the reaction solution, naturally raised to room temperature, and stirred for 1 hour. A 2mol/L aqueous hydrochloric acid solution was added to the above reaction solution, and then washed with dichloromethane and ultrapure water. After drying over anhydrous magnesium sulfate and filtering through silica gel, the filtrate was concentrated under reduced pressure, and then recrystallized with dichloromethane and n-heptane to obtain intermediate-1-A (98 g, yield 80%).

### Preparation of intermediate-2-A:

In a 3-L reaction flask, intermediate-1-A (98g, 433.44mmol), SMM (127.51g, 433.44mmol), 1000ml of anhydrous tetrahydrofuran, palladium acetate (2.92g, 13.0mmol), 2-dicyclohexylphosphine-2,4, 6-Triisopropylbiphenyl (12.39g, 26.00mmol), potassium acetate (127.61g, 1300.31mmol) were added, and the reaction solution was stirred at reflux for 2 hours under nitrogen. The reaction solution was cooled to room temperature, extracted with dichloromethane and ultrapure water, washed, dried with anhydrous magnesium sulfate, filtered, and the filtrate was concentrated under reduced pressure, and then recrystallized with dichloromethane and n-heptane to obtain intermediate-2-A (124.06g, yield 80%).

### Preparation of intermediate-3-A:

In a 1-L reaction flask, intermediate-2-A(76.68g, 214.30mmol), m-bromophenylboronic acid (43.03g, 214.31mmol), 620ml of 1,4-dioxane were added, and the reaction solution was stirred at at 60°C under nitrogen atmosphere, followed by addition of tetrakis(triphenyl)phosphine palladium (12.38g, 10.72mmol) and 50ml of an aqueous solution of potassium carbonate (59.24g, 428.62mmol). The reaction solution was heated to reflux and stirred overnight, which was then cooled to room temperature, the solid was washed with methanol and ultrapure water, and recrystallized with toluene to obtain intermediate-3-A (82 g, yield 80%).

### Preparation of intermediate-4-A:

A three-necked flask containing THF (1L) was charged with intermediate-3-A (82g, 171.4mmol), and n-butyllithium (11.6g, 179.9mmol) was added dropwise at -78°C. After the addition, the temperature of the reaction solution was maintained for 1h, and then trimethyl borate (27.51g, 257.1mmol) was added dropwise. The temperature of the reaction solution was maintained for 1h, then raised to room temperature, and the reaction solution was stirred overnight. Hydrochloric acid (2mol/L) was added into the reaction solution to adjust the pH to neutral, and then filtered to obtain a white crude product, which was beaten with n-heptane 3 times to obtain intermediate-4-A as a white solid (45.5g, yield 60%).

In a three-necked flask, intermediate-4-A (10g, 22.55mmol), intermediate-A (7.23g, 22.55mmol), tetrakis(triphenylphosphine)palladium (1.30g, 1.13mmol), potassium carbonate (9.34g, 67.67mmol), tetrabutylammonium chloride (0.31g, 1.13mmol), toluene (80mL), ethanol (40mL) and deionized water (20mL) were added, the reaction solution was heated to 78°C under the protection of nitrogen gas, and stirred for 8 hours. The reaction solution was cooled to room temperature, toluene (150 mL) was added for extraction, the organic phases were combined, the combined organic phase was dried with anhydrous magnesium sulfate, filtered, and the filtrate thus obtained was concentrated under reduced pressure to obtain a crude product; the crude product was purified by silica gel column chromatography using n-heptane as the mobile phase, and then recrystallized and purified with a dichloromethane/ethyl acetate (1:5) system to obtain compound 39 (12.33g, yield 80%), m/z=684.3[M+H]⁺.

### Synthesis of compound 115:

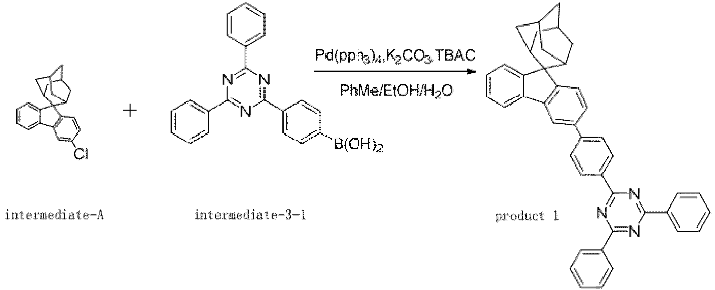

In a round-bottom flask, intermediate-A (3.92g, 12.2mmol), intermediate-3-1 (4.32g, 12.2mmol), tetrakis(triphenylphosphine) palladium (0.70g, 0.61mmol), potassium carbonate (3.37g, 24.4 mmol), tetrabutylammonium chloride (0.13g, 0.61mmol), toluene (32mL), ethanol (16mL) and deionized water (8mL) were added, the reaction solution was heated to 78°C under the protection of nitrogen gas, and stirred for 18 hours. The reaction solution was cooled to room temperature, toluene (100 mL) was added for extraction, the organic phases were combined, dried with anhydrous magnesium sulfate, filtered, and the solvent was removed under reduced pressure; the crude product was purified by silica gel column chromatography using n-heptane as the mobile phase, and then recrystallized and purified with a dichloromethane/ethyl acetate system to obtain the white product 1 (6.01g, yield 83%).

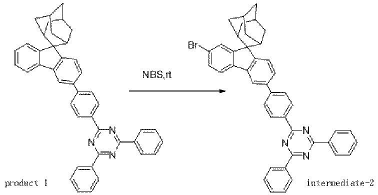

In a round-bottom flask, the white product 1 (4.07g, 11.2mmol), N-bromosuccinimide (2.3g, 13.4mmol) and dichloromethane (40mL) were added, and the reaction solution was stirred at room temperature for 2 hours under the protection of nitrogen gas; after liquid separation, the organic phase was dried with anhydrous magnesium sulfate, filtered, and the solvent was removed under reduced pressure to obtain intermediate-2 as a white solid (4.21 g, yield 55%).

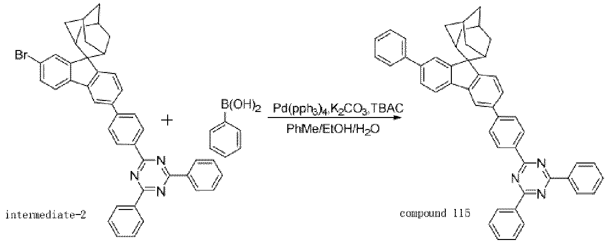

In a round-bottom flask, intermediate-2 (3.0g, 4.45mmol), phenylboronic acid (0.54g, 4.45mmol), tetrakis(triphenylphosphine)palladium (0.25g, 0.22mmol), potassium carbonate (1.23g, 8.91mmol), tetrabutyl Ammonium chloride (0.05g, 0.22mmol), toluene (24mL), ethanol (12mL) and deionized water (6mL) were added, the reaction solution was heated to 78°C under the protection of nitrogen gas, and stirred for 18 hours. The reaction solution was cooled to room temperature, toluene (100 mL) was added for extraction, the organic phases were combined, dried with anhydrous magnesium sulfate, filtered, and the solvent was removed under reduced pressure; the crude product was purified by silica gel column chromatography using n-heptane as the mobile phase, and then recrystallized and purified with a dichloromethane/ethyl acetate system to obtain compound 115 (2.74 g, yield 92%), m/z=670.3[M+H]⁺.

### Synthesis of compound 116:

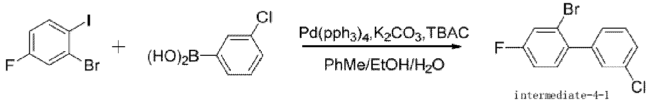

In a three-necked flask, 2-bromo-4-fluoro-1-iodobenzene (10g, 33.23mmol), p-chlorophenylboronic acid (5.20g, 33.23mmol), tetrakis(triphenylphosphine)palladium (1.92g, 1.66mmol), potassium carbonate ( 9.19g, 1.66mmol), tetrabutylammonium chloride (0.38g, 1.66mmol), toluene (80mL), ethanol (40mL) and deionized water (20mL) were added, the reaction solution was heated to 78°C under the protection of nitrogen gas, and stirred for 8 hours. The reaction solution was cooled to room temperature, toluene (100 mL) was added for extraction, the organic phases were combined, dried with anhydrous magnesium sulfate, filtered, and the solvent was removed under reduced pressure; the crude product was purified by silica gel column chromatography using n-heptane as the mobile phase, and then recrystallized and purified with a dichloromethane/ethyl acetate system to obtain a white intermediate-4-1 (7.56 g, yield 79%).

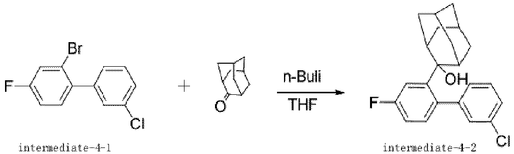

A three-necked flask containing THF (1L) was charged with intermediate-4-1 (5g, 17.5mmol), and n-butyllithium (1.27g, 19mmol) was added dropwise at -80°C. After the addition, the temperature of the reaction solution was maintained for 1h, and then adamantone (1.98g,13.2mmol) was added dropwise. The temperature of the reaction solution was maintained for 1h, then raised to room temperature, and the reaction solution was stirred overnight. Hydrochloric acid (2mol/L) was added into the reaction solution to adjust the pH to neutral, and then filtered to obtain a white crude product, which was beaten with n-heptane to obtain intermediate-4-2 as a white solid (4.32g, yield 70%).

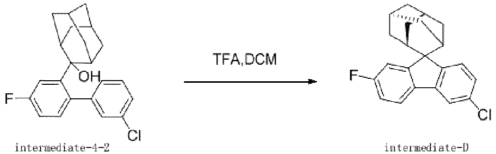

In a round-bottom flask, intermediate-4-2 (4.0g,11.2mmol), trifluoroacetic acid (3.83g,33.62mmol) and dichloromethane (40mL) were added, and stirred for 2 hours under the protection of nitrogen gas. Then, an aqueous solution of sodium hydroxide was added into the reaction solution until pH=8, followed by liquid separation, the organic phase was dried with anhydrous magnesium sulfate, filtered, and the solvent was removed under reduced pressure. The crude product obtained was purified with recrystallization using dichloromethane/n-heptane (1:2) to obtain intermediate-D (2.15g, yield 57%).

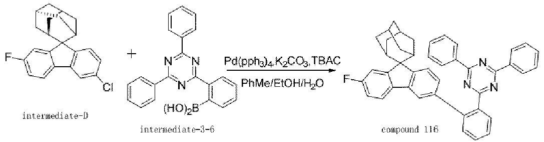

In a round-bottom flask, intermediate-D (2.0g, 5.90mmol), intermediate-3-6 (2.08g, 5.90mmol), tetrakis(triphenylphosphine)palladium (0.34g, 0.29mmol), potassium carbonate (1.63g, 11.80mmol) ), tetrabutylammonium chloride (0.06g, 0.29mmol), toluene (16mL), ethanol (8mL) and deionized water (4mL) were added, the reaction solution was heated to 78°C under the protection of nitrogen gas, and stirred for 8 hours. The reaction solution was cooled to room temperature, toluene (100 mL) was added for extraction, the organic phases were combined, dried with anhydrous magnesium sulfate, filtered, and the solvent was removed under reduced pressure; the crude product was purified by silica gel column chromatography using n-heptane as the mobile phase, and then recrystallized and purified with a dichloromethane/ethyl acetate system to obtain compound 116 (2.65 g, yield 73%), m/z=611.3[M+H]⁺.

### Synthesis of compound 121:

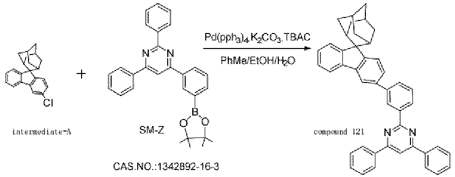

In a round-bottom flask, intermediate-A (1.96g, 6.1mmol), SM-Z (2.16g, 6.1mmol), tetrakis(triphenylphosphine) palladium (0.35g, 0.30mmol), potassium carbonate (1.69g, 12.2mmol), four Butylammonium chloride (0.65g, 0.30mmol), toluene (16mL), ethanol (8mL) and deionized water (4mL) were added, the reaction solution was heated to 78°C under the protection of nitrogen gas, and stirred for 8 hours. The reaction solution was cooled to room temperature, toluene (100 mL) was added for extraction, the organic phases were combined, dried with anhydrous magnesium sulfate, filtered, and the solvent was removed under reduced pressure; the crude product was purified by silica gel column chromatography using n-heptane as the mobile phase, and then recrystallized and purified with a dichloromethane/ethyl acetate system to obtain compound 121 (3.0 g, yield 83%), m/z=593.2[M+H]⁺.

**NMR data of some compounds is shown in Table 4 below**

**Table 4**

| **compound** | **NMR data** |
|---|---|
| compound 11 | ¹HNMR (400MHz,CD₂Cl₂) : 9.03-9.01(m,5H),8.73(s,1H),8.52(s,1H),8.37-8.33(m,2H),7.94(d,1H), 7.86-7.66(m,8H), 7.60-7.56(m,1H),7.40-7.18(m,3H),7.06(d,1H),3.10 (d,2H), 3.00 (d, 2H), 2.38 (s, 1H), 2.25 (s, 1H), 2.07 (s, 2H), 1.97 (t, 4H), 1.86 (s, 2H). |
| compound 21 | ¹HNMR(400MHz,CD₂Cl₂):9.10(s,1H),8.90(d,2H),8.74(d,1H),8.59(d,2H),8.50(s,1H), 8.37(s,1H),7.86(d,2H),7.72(d,1H),7.67-7.58(m,5H),7.54-7.38(m,11H),7.32-7.28(m,1H),7.20(d,1H), 3.05 (d, 2H), 2.95 (d, 2H), 2.35 (s, 1H), 2.21 (s, 1H), 2.05 (s, 2H), 1.94 (t, 4H), 1.85 (s, 2H). |
| compound 39 | ¹HNMR(400MHz,CD₂Cl₂): 9.05(s,1H), 8.80(d,2H), 8.77(d,1H), 8.69(s,1H), 8.60(d,1H),8.53(d,1H),7.96-7.90(m,2H), 7.72-7.48(m,8H), 7.45-7.38(m,4H), 7.30(d,1H), 7.25-7.20(m, 1H), 3.15 (d, 2H), 2.98 (d, 2H), 2.35 (s, 1H), 2.26(s, 1H), 2.08 (s, 2H), 1.96 (t, 4H), 1.87 (s, 2H). |

### Preparation and evaluation of organic electroluminescent devices

### Example 1

### Red organic electroluminescent device

The anode 1 ITO substrate with a thickness of 1500Å was cut into a size of 40mm (length) × 40mm (width) × 0.7mm (thickness), and the photolithography process was used to prepare it into an experimental substrate with cathode, anode and insulating layer patterns. Ultraviolet ozone and O₂:N₂ plasma was used for surface treatment to increase the work function of the anode (experimental substrate), and the surface of the ITO substrate was cleaned with organic solvent to remove scum and oil on the surface of the ITO substrate.

Compound HAT-CN (see below for the structural formula) was vacuum vapor-deposited on the experimental substrate to form a hole injecting layer (HIL) with a thickness of 100Å; and compound TPD was vacuum vapor-deposited on the hole injection layer to form a hole transporting layer (HIL) with a thickness of 1200Å.

On the hole transporting layer (HTL), compound TCTA (see below for the structural formula) was vapor-deposited and served as an electron blocking layer (EBL) with a thickness of 100Å .

On the electron blocking layer (EBL), compound 4,4'-N,N'-dicarbazole-biphenyl (referred to as "CBP" for short) (see below for the structural formula) was vapor-deposited and served as the core group, Ir(piq)2(acac) was doped (see below for the structural formula), and vapor deposition was carried out at a film thickness ratio of 20:1 to form an organic electroluminescent layer (EML) with a thickness of 330Å.

On the organic electroluminescent layer (EML), compound 1 and LiQ (see below for the structural formula) was doped by vapor-deposition, which was carried out at a film thickness ratio of 1:1, to act as a electron transporting layer (ETL) with a vapor-depositing thickness of 350Å.

A 15Å ytterbium (Yb) was vapor-deposited on the electron transporting layer (ETL) to form an electron injecting layer (EIT), and then silver (Ag) and magnesium (Mg) with a film thickness ratio of 10:1 were used as the cathode. The thickness of the cathode was 120Å.

Compound CP-1 (see below for structural formula) was vapor-deposited on the cathode as an organic covering layer (CPL). The thickness of the organic capping layer is 630 Å.

The vapor-deposited device was encapsulated with ultraviolet hardening resin in a nitrogen glove box (the content of water and oxygen was strictly controlled) to prevent the device from being corroded by external moisture or other substances.

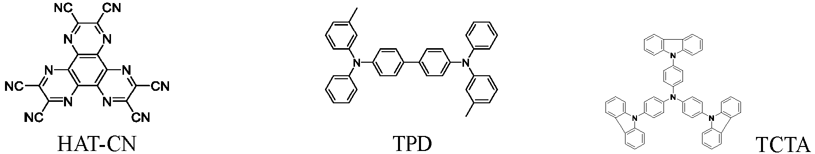

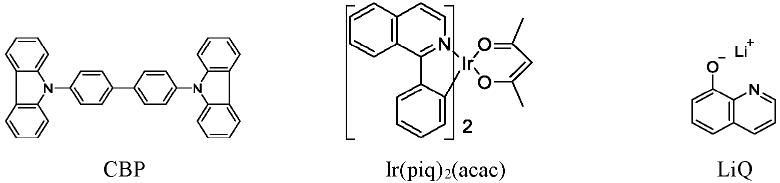

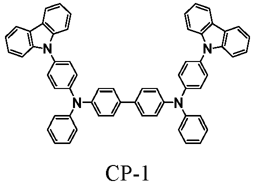

### Examples 2-46

The compounds in Table 5 were used instead of compound 1, and the red organic electroluminescent device was made in the same method as Example 1.

### Comparative Example 1

Compound A was used instead of compound 1, and the red organic electroluminescent device was made in the same method as Example 1.

### Comparative Example 2

Compound B was used instead of compound 1, and the red organic electroluminescent device was made in the same method as Example 1.

### Comparative Example 3

Compound C was used instead of compound 1, and the red organic electroluminescent device was made in the same method as Example 1.

### Comparative Example 4

Compound D was used instead of compound 1, and the red organic electroluminescent device was made in the same method as Example 1.

### Comparative Example 5

Compound Alq3 (CAS NO. :2085-33-8) was used instead of compound 1, and the red organic electroluminescent device was made in the same method as Example 1.

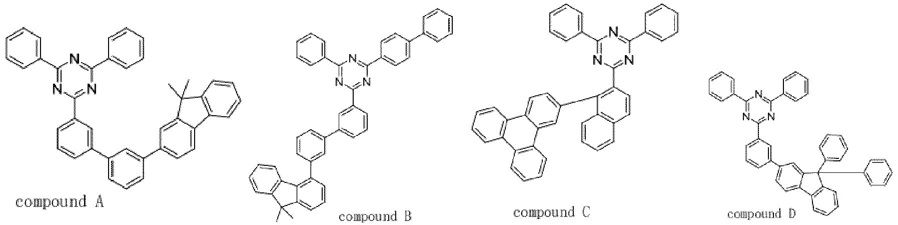

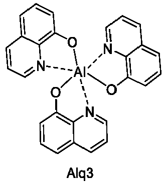

For the organic electroluminescent device prepared as above, the performance of the device was analyzed under the condition of 10 mA/cm², and the results are shown in Table 5.

**Table 5 Performance test results of red organic electroluminescent devices**

| | Electron transporting layer | Operating voltage V | Luminous efficiency cd/A | Color coordin ate CIEx | External quantum efficiency EQE% | T95 lifetime (15mA/cm²) |
|---|---|---|---|---|---|---|
| Example 1 | compound 1 | 3.84 | 34.1 | 0.68 | 24.5 | 435 |
| Example 2 | compound 2 | 3.86 | 33.4 | 0.68 | 23.6 | 438 |
| Example 3 | compound 3 | 3.83 | 33.6 | 0.68 | 23.8 | 446 |
| Example 4 | compound 4 | 3.88 | 33.9 | 0.68 | 24.1 | 430 |
| Example 5 | compound 5 | 3.90 | 33.8 | 0.68 | 23.9 | 435 |
| Example 6 | compound 6 | 3.86 | 33.5 | 0.68 | 23.7 | 433 |
| Example 7 | compound 7 | 3.81 | 33.3 | 0.68 | 23.4 | 445 |
| Example 8 | compound 8 | 3.85 | 34.0 | 0.68 | 24.4 | 435 |
| Example 9 | compound 9 | 3.88 | 33.8 | 0.68 | 23.9 | 447 |
| Example 10 | compound 10 | 3.83 | 33.7 | 0.68 | 23.8 | 430 |
| Example 11 | compound 11 | 3.86 | 34.1 | 0.68 | 24.4 | 445 |
| Example 12 | compound 12 | 3.91 | 33.6 | 0.68 | 23.8 | 432 |
| Example 13 | compound 13 | 3.87 | 33.8 | 0.68 | 23.8 | 430 |
| Example 14 | compound 14 | 3.91 | 33.9 | 0.68 | 24.2 | 433 |
| Example 15 | compound 15 | 3.87 | 33.4 | 0.68 | 23.5 | 438 |
| Example 16 | compound 16 | 3.86 | 33.7 | 0.68 | 23.9 | 442 |
| Example 17 | compound 17 | 3.81 | 33.8 | 0.68 | 24.0 | 442 |
| Example 18 | compound 18 | 3.85 | 34.1 | 0.68 | 24.4 | 440 |
| Example 19 | compound 19 | 3.87 | 33.8 | 0.68 | 24.1 | 437 |
| Example 20 | compound 20 | 3.83 | 33.6 | 0.68 | 23.6 | 445 |
| Example 21 | compound 21 | 3.86 | 33.5 | 0.68 | 23.4 | 441 |
| Example 22 | compound 22 | 3.86 | 33.7 | 0.68 | 23.8 | 439 |
| Example 23 | compound 23 | 3.88 | 34.1 | 0.68 | 24.3 | 438 |
| Example 24 | compound 24 | 3.87 | 33.7 | 0.68 | 23.7 | 440 |
| Example 25 | compound 25 | 3.90 | 34.2 | 0.68 | 24.4 | 442 |
| Example 26 | compound 26 | 3.86 | 33.9 | 0.68 | 24.0 | 443 |
| Example 27 | compound 27 | 3.84 | 33.5 | 0.68 | 23.4 | 440 |
| Example 28 | compound 28 | 3.88 | 33.8 | 0.68 | 24.0 | 444 |
| Example 29 | compound 29 | 3.81 | 34.3 | 0.68 | 24.4 | 440 |
| Example 30 | compound 30 | 3.82 | 34.1 | 0.68 | 24.2 | 445 |
| Example 31 | compound 31 | 3.85 | 33.8 | 0.68 | 23.9 | 438 |
| Example 32 | compound 32 | 3.87 | 33.3 | 0.68 | 23.4 | 435 |
| Example 33 | compound 33 | 3.83 | 33.8 | 0.68 | 23.8 | 432 |
| Example 34 | compound 34 | 3.83 | 33.6 | 0.68 | 23.6 | 438 |
| Example 35 | compound 35 | 3.87 | 33.9 | 0.68 | 24.1 | 442 |
| Example 36 | compound 36 | 3.81 | 33.8 | 0.68 | 23.7 | 437 |
| Example 37 | compound 37 | 3.85 | 33.7 | 0.68 | 23.6 | 439 |
| Example 38 | compound 38 | 3.87 | 33.9 | 0.68 | 24.1 | 438 |
| Example 39 | compound 39 | 3.88 | 34.3 | 0.68 | 24.5 | 442 |
| Example 40 | compound 115 | 3.85 | 33.3 | 0.68 | 24.2 | 442 |
| Example 41 | compound 116 | 3.86 | 34.1 | 0.68 | 24.3 | 431 |
| Example 42 | compound 121 | 3.88 | 33.9 | 0.68 | 24.4 | 437 |
| Example 43 | compound 171 | 3.86 | 33.9 | 0.68 | 24.5 | 441 |
| Example 44 | compound 176 | 3.85 | 34.1 | 0.68 | 24.1 | 439 |
| Example 45 | compound 182 | 3.86 | 33.6 | 0.68 | 23.8 | 435 |
| Example 46 | compound 185 | 3.87 | 33.8 | 0.68 | 24.2 | 438 |
| Comparative Example 1 | compound A | 4.13 | 27.4 | 0.68 | 20.5 | 327 |
| Comparative Example 2 | compound B | 4.17 | 27.2 | 0.68 | 19.9 | 325 |
| Comparative Example 3 | compound C | 4.11 | 27.7 | 0.68 | 20.7 | 310 |
| Comparative Example 4 | compound D | 4.12 | 27.3 | 0.68 | 19.9 | 320 |
| Comparative Example 5 | Alq³ | 4.00 | 28.5 | 0.68 | 21.7 | 350 |

From Table 5, it can be seen that in the OLED devices where compounds were used as the electron transporting layer, compared with the Comparative Examples, the performances of the organic electroluminescent devices prepared by Examples 1-46 have been improved. Among them, compared with Comparative Example 1, the operating voltage of the organic electroluminescent devices prepared by Example 1-46 was reduced by at least 0.22V, the external quantum efficiency (EQE) was improved by at least 14.1%, and the luminous efficiency (Cd/A) was improved by at least 21.5. %, the lifetime (T95) was improved by at least 31.5%; compared with Comparative Example 2, the operating voltage of the organic electroluminescent devices prepared by Example 1-46 was reduced by at least 0.25V, the external quantum efficiency (EQE) was improved by at least 17.6%, the luminous efficiency (Cd/A) was improved by at least 22.4%, the lifetime (T95) was improved by at least 32.3%; compared with Comparative Example 3, the operating voltage of the organic electroluminescent devices prepared by Example 1-46 was reduced by at least 0.2V, the external quantum efficiency (EQE) was improved by at least 13.0%, the luminous efficiency (Cd/A) was improved by at least 38.7%, lifetime (T95) was improved by at least 31.5%; compared with Comparative Example 4, the operating voltage of the organic electroluminescent devices prepared by Example 1-46 was reduced by at least 0.21V, the external quantum efficiency (EQE) was improved by at least 17.6%, the luminous efficiency (Cd/A) was improved by at least 22%, and the lifetime (T95) was improved by at least 34.4%; compared with Comparative Example 5, the operating voltage of the organic electroluminescent devices prepared by Example 1-46 was reduced by at least 0.09V, the external quantum efficiency (EQE) was improved by at least 7.8%, the luminous efficiency (Cd/A) was improved by at least 16.8%, the lifetime (T95) was improved by at least 22.9%. Therefore, using the nitrogen-containing compound of the present disclosure in the electron transporting layer can prepare an organic electroluminescent device with high luminous efficiency and long lifetime.

The nitrogen-containing compound provided in the present disclosure has a molecular structure combining a nitrogen-containing heterocyclic ring with 1, 3 or 4-position adamantane fluorene. In one aspect, the nitrogen-containing heterocyclic compound can reduce the energy level injection into the barrier, thereby reducing the operating voltage of the organic electroluminescent device. At the same time, the nitrogen-containing heterocyclic structure is relatively stable, not easy to decompose, and has strong high temperature resistance, which can extend the service life of electronic elements, such as organic electroluminescence devices and photoelectric conversion devices. In another aspect, the adamantane-fluorene group has a large molecular weight, which can effectively increase the glass transition temperature of the material, and the large steric hindrance of its structure makes the material difficult to crystallize or aggregate, and the material has a better lifespan in electronic elements. Not only that, on the basis of the nitrogen-containing heterocyclic ring, the introduction of the adamantane-fluorene group as an electron injecting and transporting group makes the nitrogen-containing heterocyclic compound have electron-rich characteristics, and the polarity of the entire molecule is enhanced, which is more conducive to the directional arrangement of the material molecules, thereby enhancing the injection and transportation of electrons, enhancing the electronic conductivity of electron transporting materials. At the same time, the luminous efficiency of the organic electroluminescent device using the nitrogen-containing compound can be improved, and the conversion efficiency of the photoelectric conversion device using the nitrogen-containing compound can be improved.

## Claims

1. A nitrogen-containing compound having a structure of Chemical Formula 1: 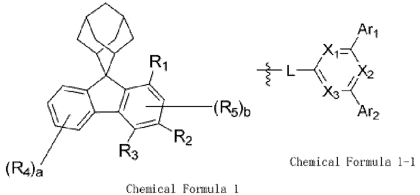
wherein 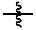 represents a chemical bond;
X₁, X₂, and X₃ are the same or different, and are each independently selected from C or N, and at least one is N;
R₁, R₂ and R₃ are each independently selected from hydrogen or a group represented by Chemical Formula 1-1, and one and only one of R₁, R₂ and R₃ has the group represented by Chemical Formula 1-1; when R₁, R₂ or R₃ is selected from hydrogen, said R₁, R₂ or R₃ may be replaced by R₅;
R₄, R₅ are the same or different, and are each independently selected from deuterium, halogen, a heteroaryl having 3 to 20 carbon atoms, an aryl having 6 to 20 carbon atoms, a trialkylsily having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, and an alkylthio having 1 to 10 carbon atoms, an aryloxy having 6 to 18 carbon atoms, an arylthio having 6 to 18 carbon atoms, a phosphoryloxy having 6 to 18 carbon atoms;
a is selected from 0, 1, 2, 3, or 4; when a is greater than 1, any two R₄ are the same or different;
b is selected from 0, 1, 2, or 3, when b is greater than 1, any two R₅ are the same or different;
L is selected from a single bond, a substituted or unsubstituted arylene having 6 to 30 carbon atoms, or a substituted or unsubstituted heteroarylene having 3 to 30 carbon atoms;
Ar₁ and Ar₂ are each independently selected from the following substituted or unsubstituted groups: an alkyl group having 1 to 20 carbon atoms, a cycloalkyl group having 3 to 20 carbon atoms, an aryl having 6 to 30 carbon atoms, a heteroaryl having 3 to 30 carbon atoms;
the substituents of L are selected from deuterium, halogen, cyano, a heteroaryl having 3 to 20 carbon atoms, an aryl having 6 to 20 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, and an alkylthio having 1 to 10 carbon atoms, an aryloxy having 6 to 18 carbon atoms, an arylthio having 6 to 18 carbon atoms, a phosphoryloxy having 6 to 18 carbon atoms;
the substituents in Ar₁ and Ar₂ are the same or different, and are each independently selected from deuterium, halogen, a heteroaryl having 3 to 20 carbon atoms, an aryl having 6 to 20 carbon atoms, and a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms.

2. The nitrogen-containing compound of claim 1, wherein L is selected from a single bond, a substituted or unsubstituted arylene having 6 to 25 ring-forming carbon atoms, and a substituted or unsubstituted heteroarylene having 5 to 18 ring-forming carbon atoms.

3. The nitrogen-containing compound according to claim 1, wherein L is selected from a single bond, a substituted or unsubstituted arylene having 6 to 25 carbon atoms, and a substituted or unsubstituted heteroarylene having 5 to 20 carbon atoms.

4. The nitrogen-containing compound according to claim 1, wherein L is selected from a single bond, a substituted or unsubstituted phenylene, a substituted or unsubstituted naphthylene, and a substituted or unsubstituted phenanthrylene, a substituted or unsubstituted anthracenylene, a substituted or unsubstituted fluorenylene, a substituted or unsubstituted biphenylene, a substituted or unsubstituted terphenylene, a substituted or unsubstituted pyridylene, a substituted or unsubstituted dibenzofuranyl, a substituted or unsubstituted dibenzothienyl, a substituted or unsubstituted N-phenylcarbazolylidene;
preferably, the substituents in L are selected from deuterium, halogen, cyano, an aryl having 6 to 12 carbon atoms, a heteroaryl group having 5 to 18 carbon atoms, an alkyl having 1 to 5 carbon atoms, a cycloalkyl having 3 to 10 carbon atoms.

5. The nitrogen-containing compound of claim 1, wherein L is selected from a single bond or the group consisting of groups of the chemical formula j-1 to j-13: 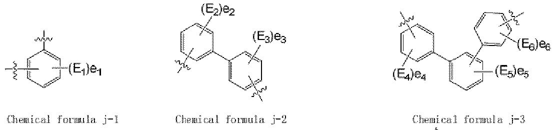 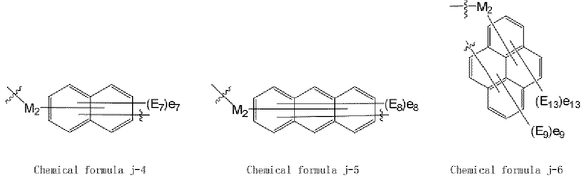 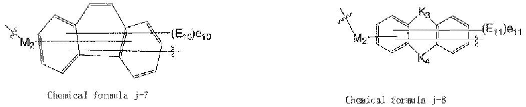 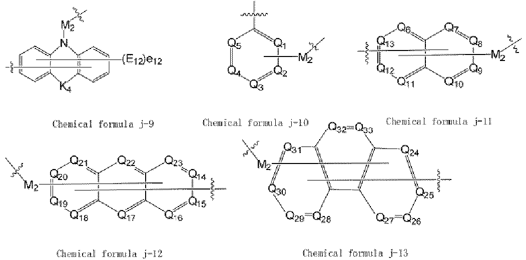
wherein M₂ is selected from a single bond or 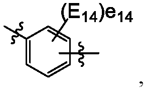
Q₁ to Q₅ are each independently selected from N or C(F₅), and at least one of Q₁ to Q₅ is selected from N; when two or more of Q₁ to Q₅ are selected from C(F₅), any two of F₅ are the same or different;
Q₆ to Q₁₃ are each independently selected from N or C(F₆), and at least one of Q₆ to Q₁₃ is selected from N; when two or more of Q₆ to Q₁₃ are selected from C(F₆), any two of F₆ are the same or different;
Q₁₄ to Q₂₃ are each independently selected from N or C(F₇), and at least one of Q₁₄ to Q₂₃ is selected from N; when two or more of Q₁₄ to Q₂₃ are selected from C(F₇), any two of F₇ are the same or different;
Q₂₄ to Q₃₃ are each independently selected from N or C(F₈), and at least one of Q₂₄ to Q₃₃ is selected from N; when two or more of Q₂₄ to Q₃₃ are selected from C(F₈), any two of F₈ are the same or different;
E₁ to E₁₄, F₅ to F₈ are each independently selected from deuterium, halogen, cyano, a heteroaryl having 3 to 18 carbon atoms, an aryl having 6 to 18 carbon atoms and optionally substituted by deuterium, fluorine, chlorine or cyano, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms;
any one of F₅ to F₈ may also be independently selected from hydrogen;
eᵣ is the number of substituents Eᵣ, r is any integer from 1 to 14; when r is selected from 1, 2, 3, 4, 5, 6, 9, 13 or 14, eᵣ is selected from 0, 1, 2, 3 or 4; when r is selected from 7 or 11, eᵣ is selected from 0, 1, 2, 3, 4, 5 or 6; when r is 12, eᵣ is selected from 0, 1, 2, 3, 4, 5, 6 or 7; when r is selected from 8 or 10, eᵣ is selected from 0, 1, 2, 3, 4, 5, 6, 7 or 8; when eᵣ is greater than 1, any two of Eᵣ are the same or different;
K₃ is selected from O, S, Se, N(E₁₅), C(E₁₆E₁₇), Si(E₁₆E₁₇); wherein, E₁₅, E₁₆, E₁₇ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocycloalkenyl group having 4 to 10 carbon atoms, or the above E₁₆ and E₁₇ are connected to each other to form a ring with the atom that they are commonly connected to;
K₄ is selected from a single bond, O, S, Se, N(E₁₈), C(E₁₉E₂₀), Si(E₁₉E₂₀); wherein, E₁₈, E₁₉, E₂₀ are each independently selected from an aryl having 6 to 18 carbon atoms, a heteroaryl having 3 to 18 carbon atoms, an alkyl having 1 to 10 carbon atoms, alkenyl having 2 to 6 carbon atoms, alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, or the above E₁₉ and E₂₀ are connected to each other to form a ring with the atom that they are commonly connected to.

6. The nitrogen-containing compound of claim 1, wherein L is selected from a single bond or the group consisting of the following groups:

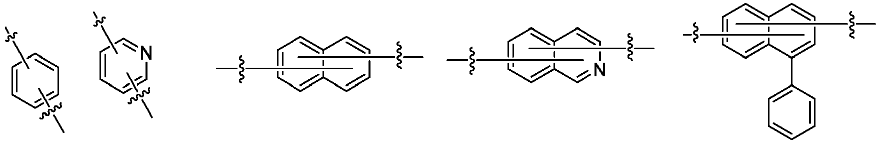

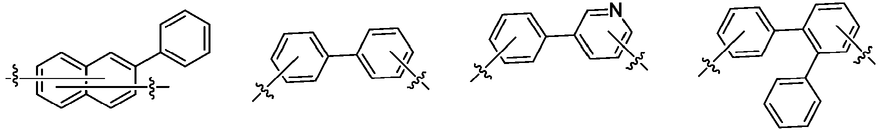

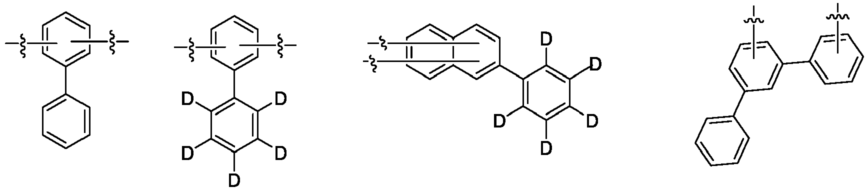

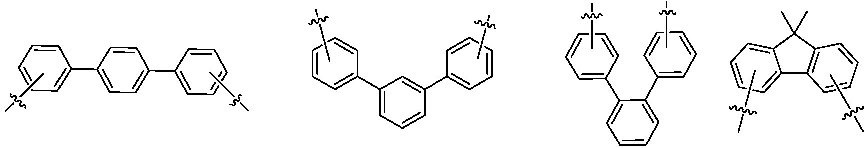

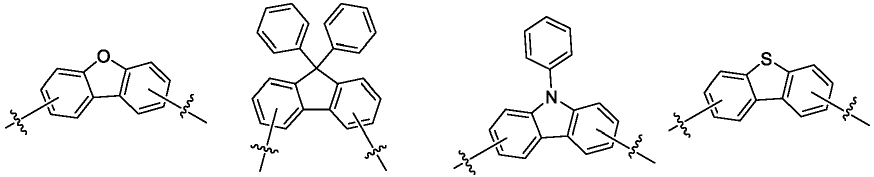

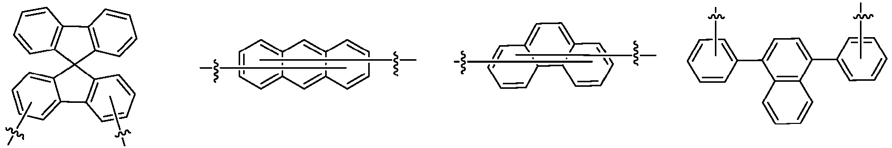

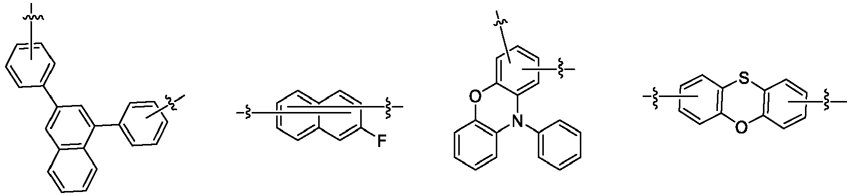

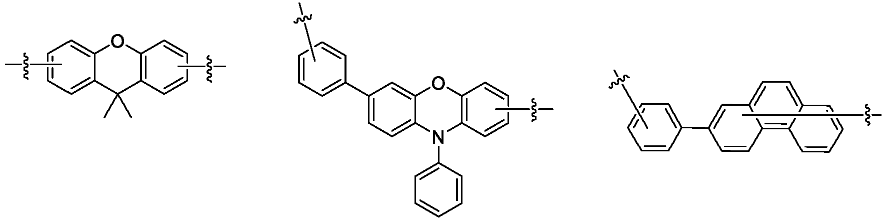

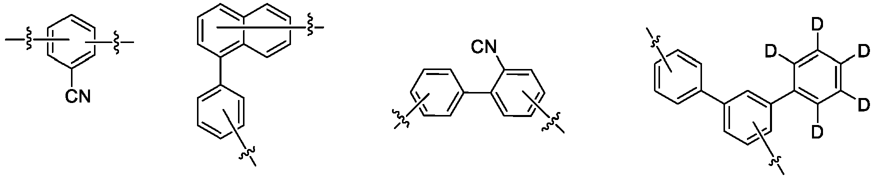

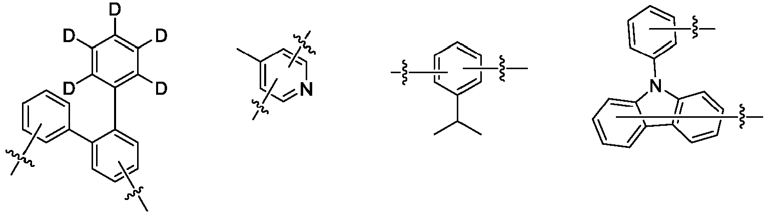

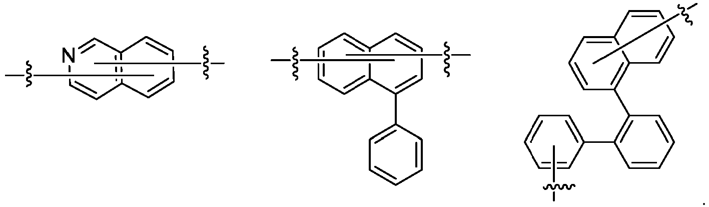

7. The nitrogen-containing compound of claim 1, wherein Ar₁ and Ar₂ are each independently selected from the following substituted or unsubstituted groups: an aryl having 6 to 25 ring-forming carbon atoms, or a heteroaryl having 5 to 20 ring-forming carbon atoms.

8. The nitrogen-containing compound of claim 1, wherein Ar₁ and Ar₂ are each independently selected from substituted or unsubstituted aryl having 6 to 25 carbon atoms, or substituted or unsubstituted heteroaryl having 5 to 24 carbon atoms;
preferably, the substituents in Ar₁ and Ar₂ are each independently selected from deuterium, halogen, an aryl having 6 to 12 carbon atoms, a heteroaryl group having 3 to 20 carbon atoms, an alkyl having 1 to 5 carbon atoms, a cycloalkyl having 3 to 10 carbon atoms.

9. The nitrogen-containing compound of claim 1, wherein Ar₁ and Ar₂ are each independently selected from substituents represented by the following chemical formula i-1 to 1-15: 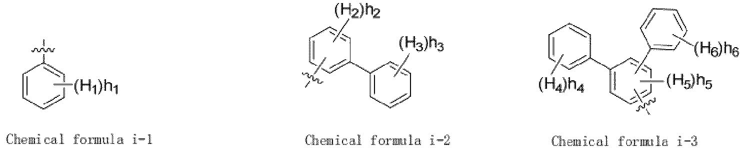 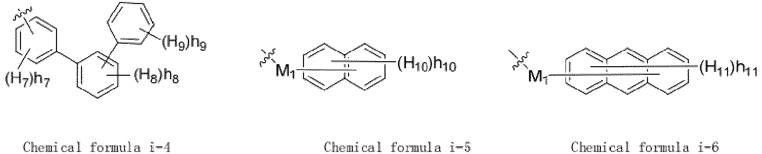 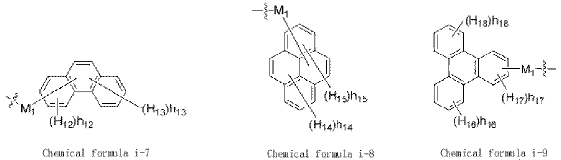 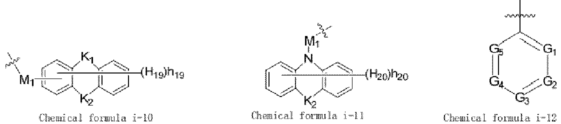 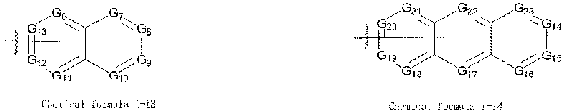 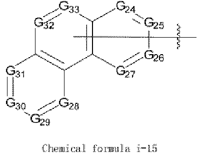
wherein M₁ is selected from a single bond or 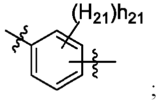
G₁ to G₅ are each independently selected from N or C(F₁), and at least one of G₁ to G₅ is selected from N; when two or more of G₁ to G₅ are selected from C(F₁), any two F₁ are the same or different;
G₆ to G₁₃ are each independently selected from N or C(F₂), and at least one of G₆ to G₁₃ is selected from N; when two or more of G₆ to G₁₃ are selected from C(F₂), any two F₂ are the same or different;
G₁₄ to G₂₃ are each independently selected from N or C(F₃), and at least one of G₁₄ to G₂₃ is selected from N; when two or more of G₁₄ to G₂₃ are selected from C(F₃), any two F₂ are the same or different;
G₂₄ to G₃₃ are each independently selected from N or C(F₄), and at least one of G₂₄ to G₃₃ is selected from N; when two or more of G₂₄ to G₃₃ are selected from C(F₄), any two F₂ are the same or different;
H₁ to H₂₁, F₁ to F₄ are each independently selected from deuterium, halogen, a heteroaryl having 3 to 18 carbon atoms, a trialkylsilyl having 3 to 12 carbon atoms, an arylsilyl having 8 to 12 carbon atoms, an alkyl having 1 to 10 carbon atoms, a haloalkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, an alkoxy having 1 to 10 carbon atoms, an alkylthio having 1 to 10 carbon atoms, an aryloxy having 6 to 18 carbon atoms, an arylthio having 6 to 18 carbon atoms, a phosphoryloxy having 6 to 18 carbon atoms;
any one of H₄ to H₂₀ may also be independently selected from an aryl having 6 to 18 carbon atoms;
any one of F₁ to F₄ may also be independently selected from hydrogen and an aryl having 6 to 18 carbon atoms;
hₖ is the number of substituents Hₖ, k is any integer from 1 to 21; wherein, when k is selected from 5 or 17, hₖ is selected from 0, 1, 2 or 3; when k is selected from 2, 7, 8, 12, 15, 16, 18, or 21, hₖ is selected from 0, 1, 2, 3, or 4; when k is 1, 3, 4, 6, 9 or 14, hₖ is selected from 0, 1, 2, 3, 4, or 5; when k is 13, hₖ is selected from 0, 1, 2, 3, 4, 5, or 6; when k is selected from 10 or 19, hₖ is selected from 0, 1, 2, 3, 4, 5, 6 or 7; when k is selected from 20, hₖ is selected from 0, 1, 2, 3, 4, 5, 6, 7 or 8; when k is 11, hₖ is selected from 0, 1, 2, 3, 4, 5, 6, 7, 8, or 9;
When hₖ is greater than 1, any two Hk are the same or different;
K₁ is selected from O, S, Se, N (H₂₂), C (H₂₃H₂₄), Si (H₂₃H₂₄); wherein, H₂₂, H₂₃, and H₂₄ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 10 carbon atoms, a heterocyclenyl having 4 to 10 carbon atoms, or the above H₂₃ and H₂₄ are connected to each other to form a ring with the atom they are commonly connected to;
K₂ is selected from a single bond, O, S, Se, N(H₂₅), C(H₂₆H₂₇), Si(H₂₆H₂₇);
wherein, H₂₅, H₂₆, H₂₇ are each independently selected from an aryl having 6 to 18 carbon atoms, a heteroaryl having 3 to 18 carbon atoms, an alkyl having 1 to 10 carbon atoms, an alkenyl having 2 to 6 carbon atoms, an alkynyl having 2 to 6 carbon atoms, a cycloalkyl having 3 to 10 carbon atoms, a heterocycloalkyl having 2 to 10 carbon atoms, a cycloalkenyl having 5 to 1 0 carbon atoms, a heterocyclenyl having 4 to 10 carbon atoms, or the above H₂₆ and H₂₇ are connected to each other to form a ring with the atom they are commonly connected to.

10. The nitrogen-containing compound of claim 1, wherein Ar₁ and Ar₂ are each independently selected from the group consisting of the following groups:

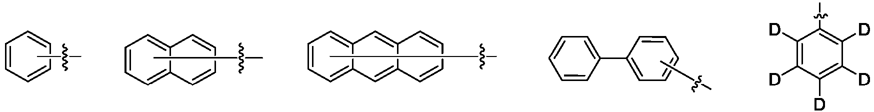

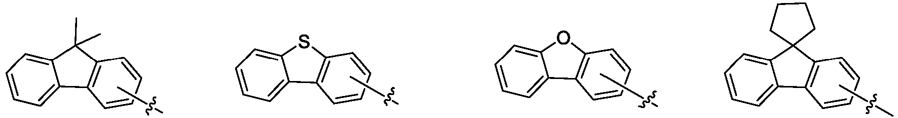

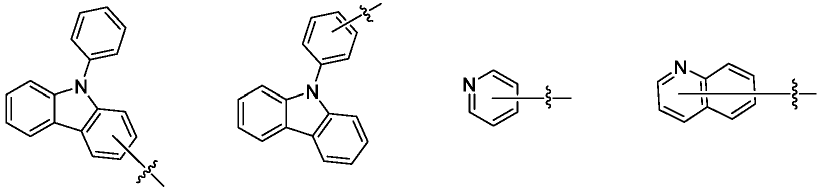

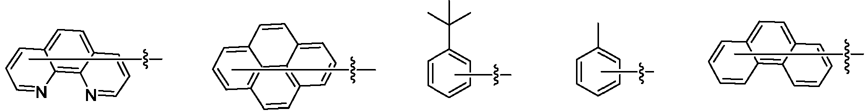

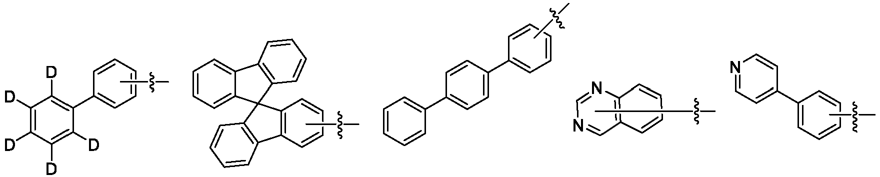

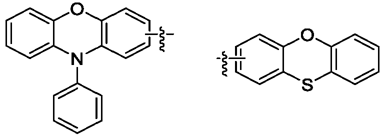

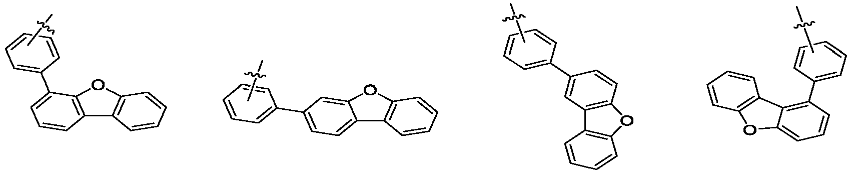

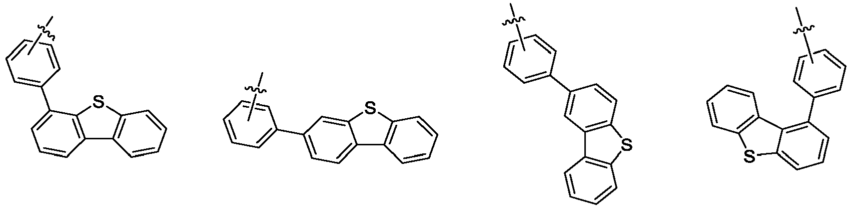

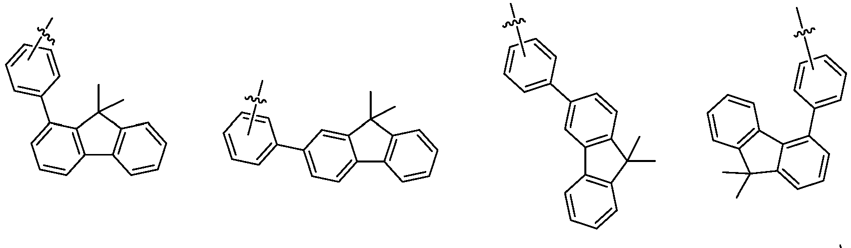

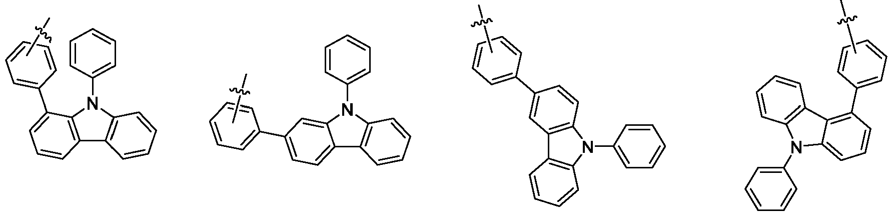

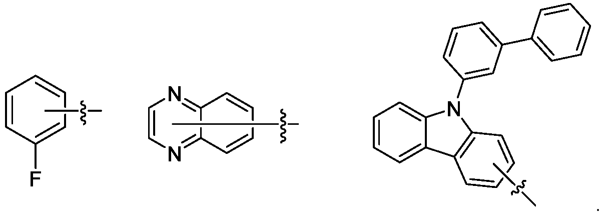

11. The nitrogen-containing compound of claim 1, wherein the nitrogen-containing compound are each independently selected from the group consisting of the following compounds:

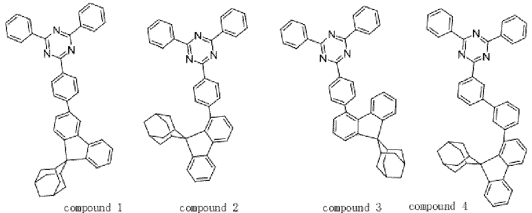

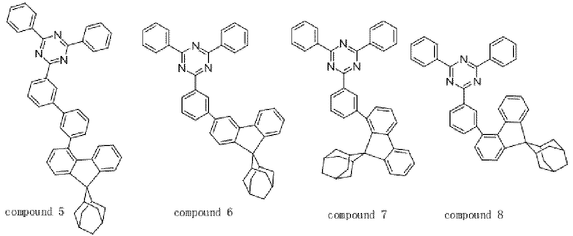

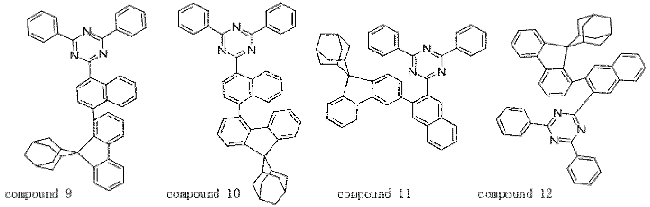

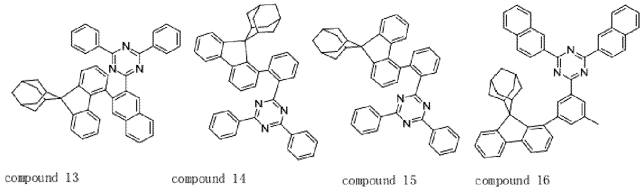

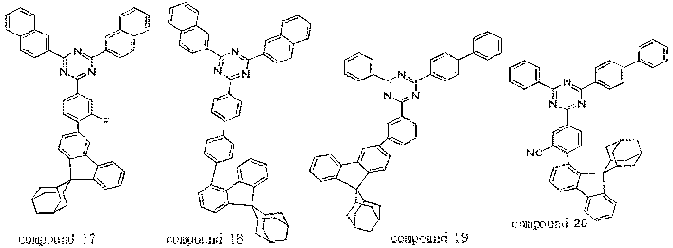

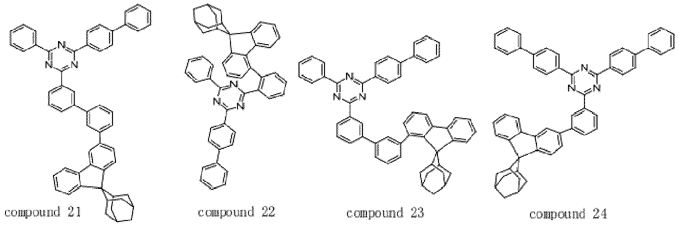

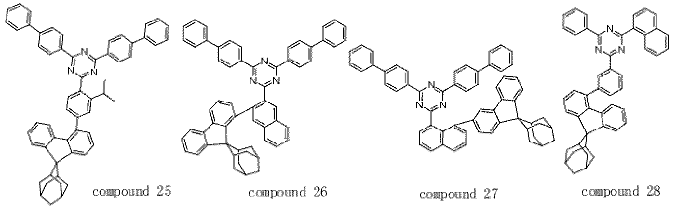

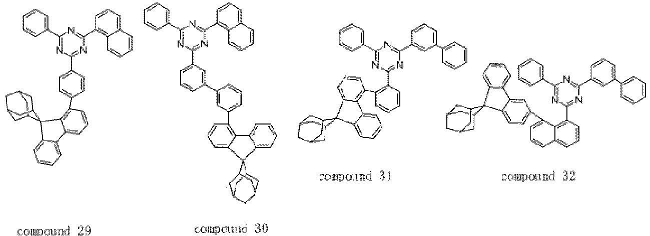

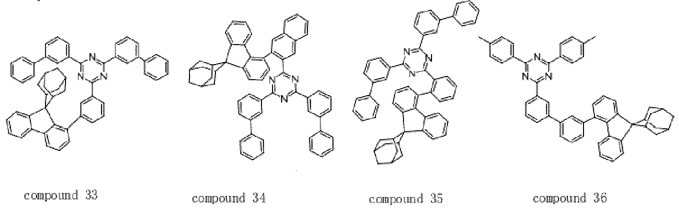

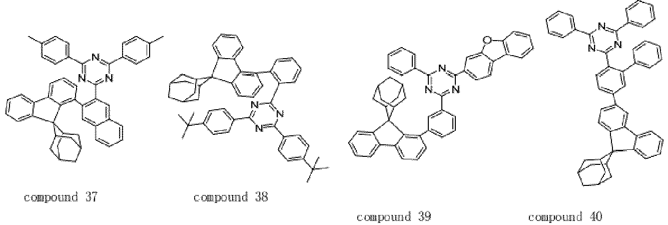

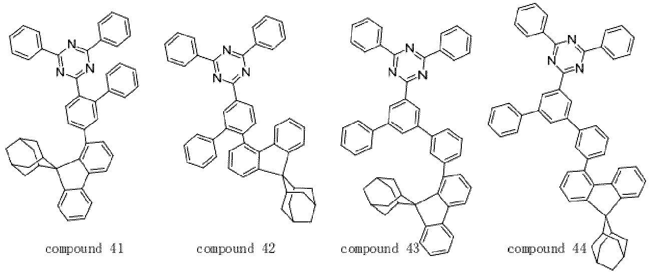

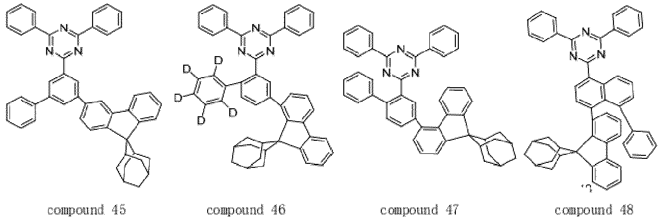

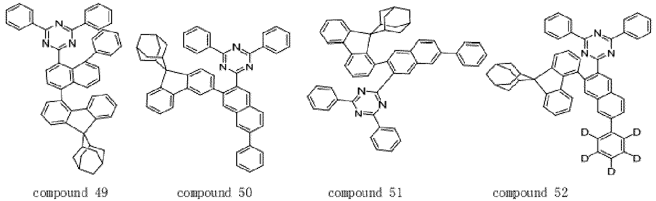

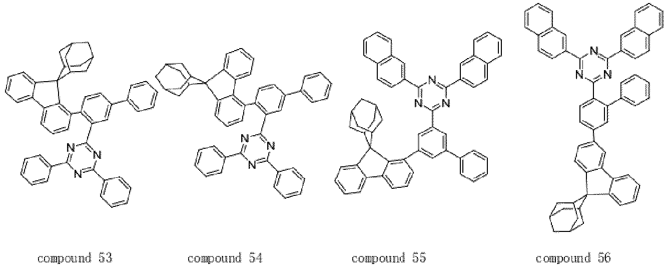

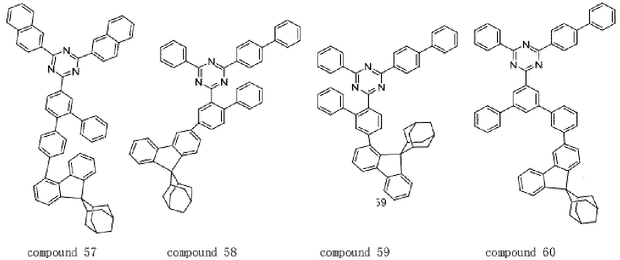

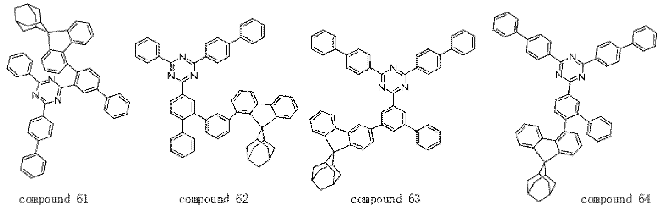

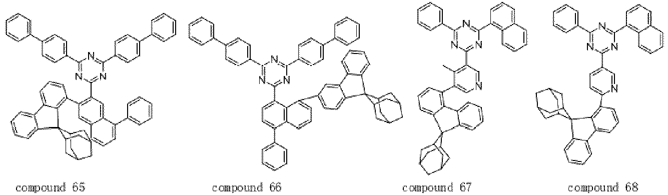

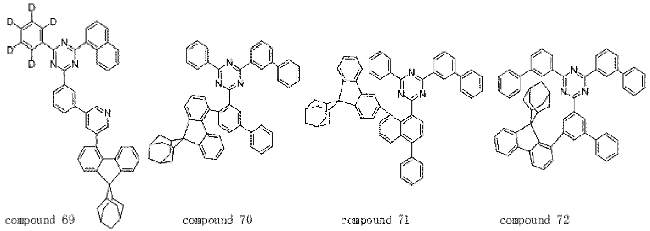

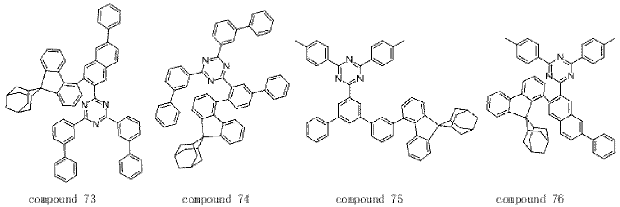

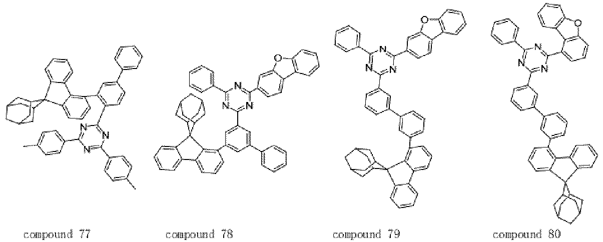

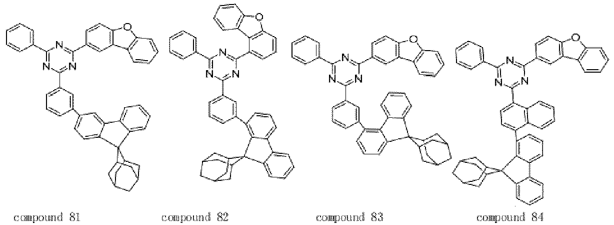

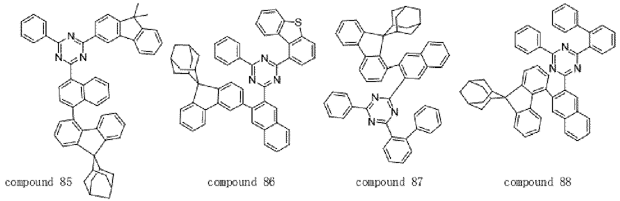

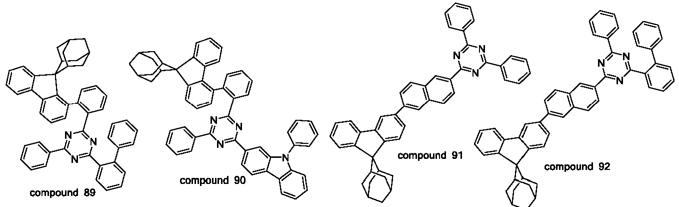

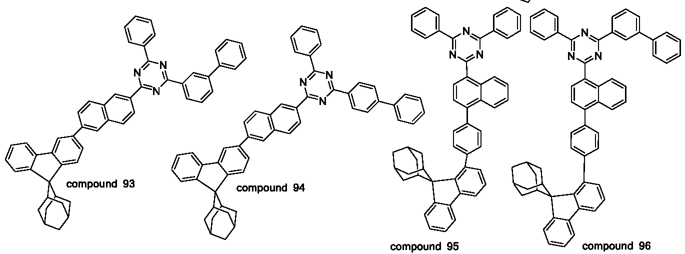

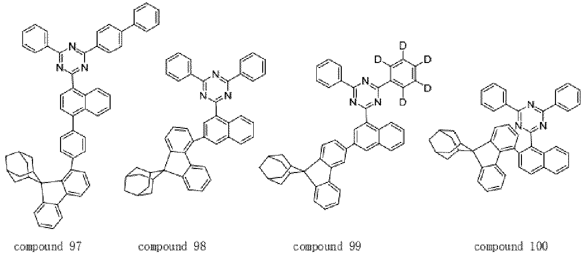

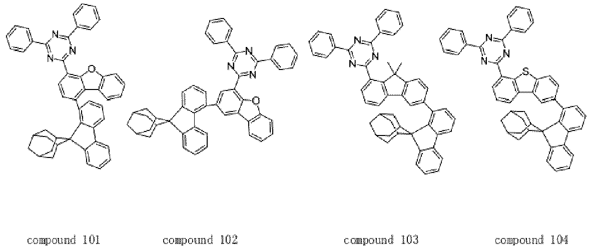

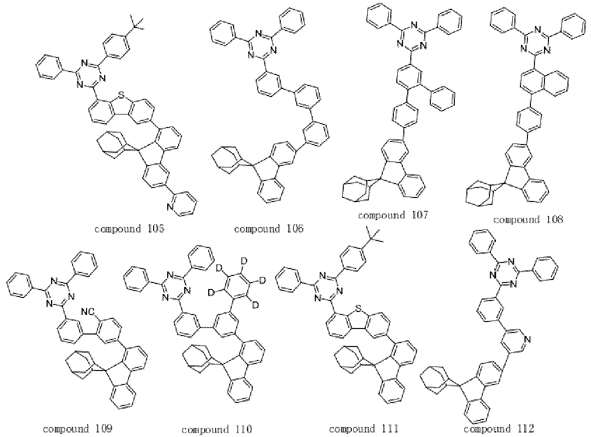

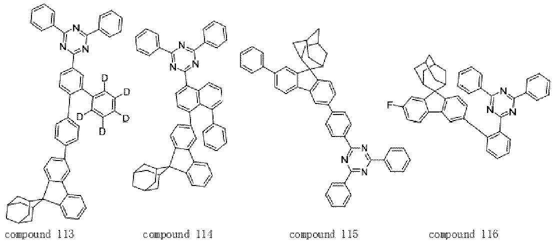

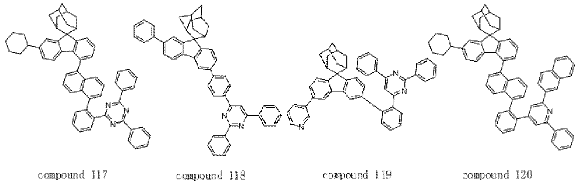

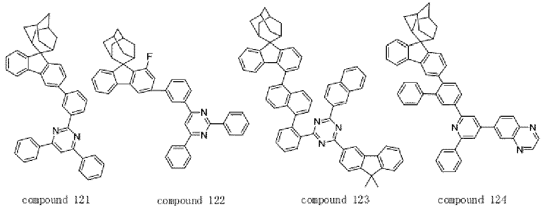

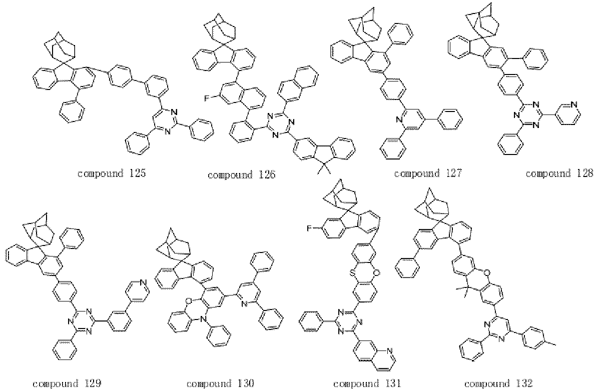

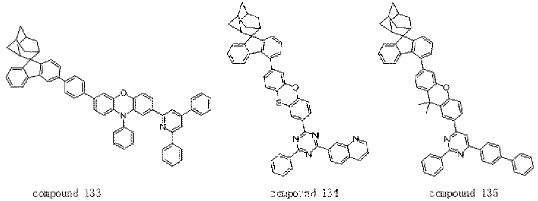

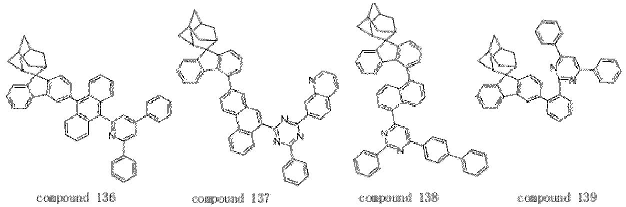

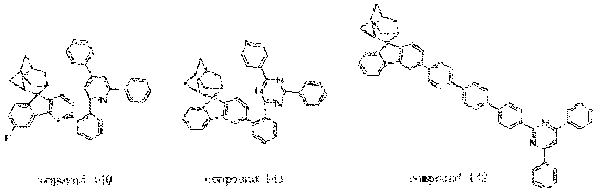

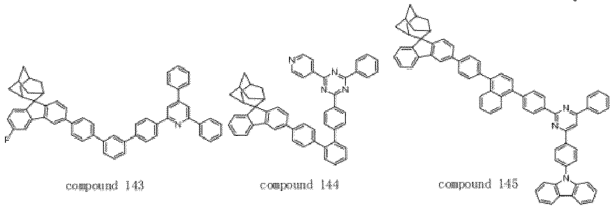

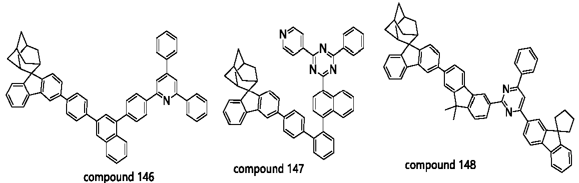

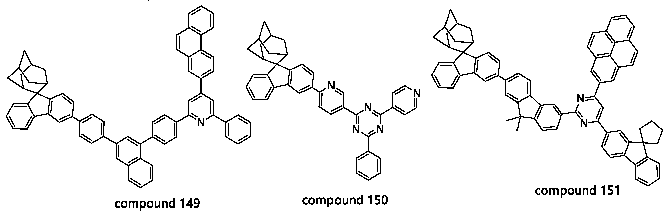

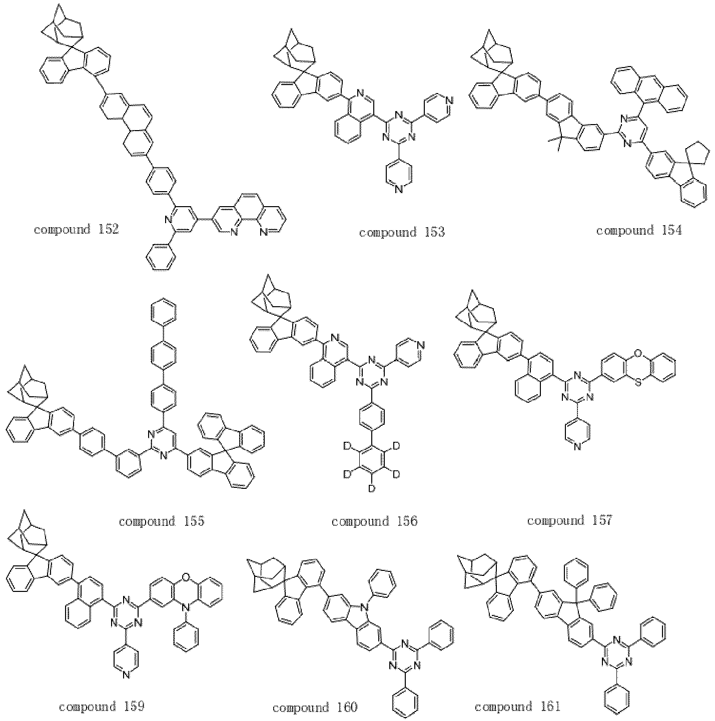

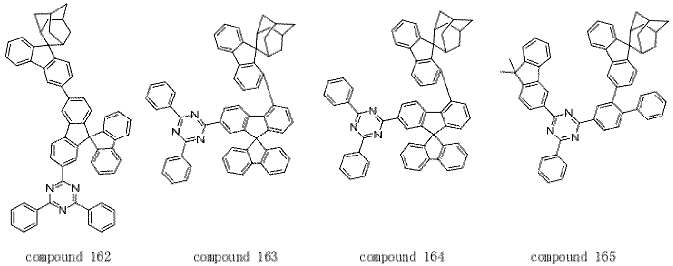

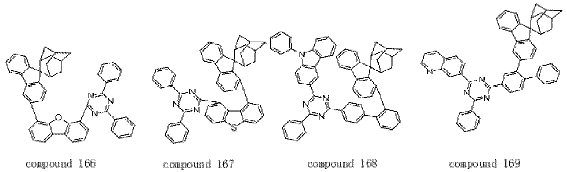

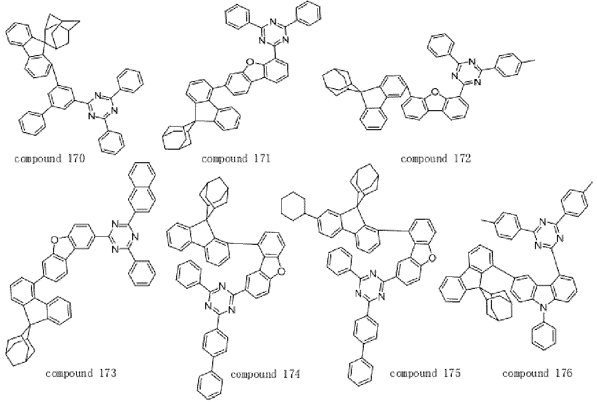

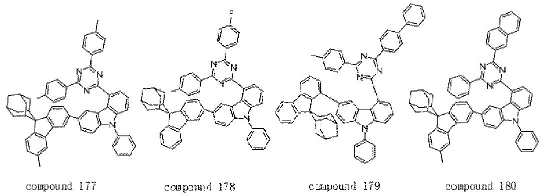

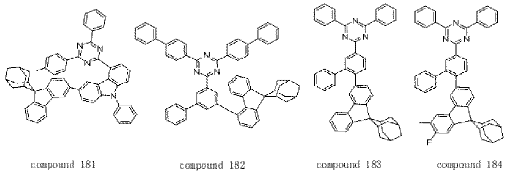

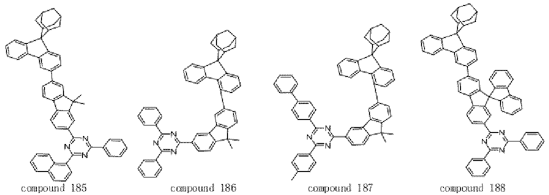

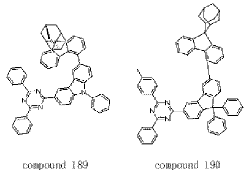

12. An electronic element, wherein the electronic element comprises an anode and a cathode disposed oppositely, and a functional layer arranged between the anode and the cathode;
the functional layer comprises the nitrogen-containing compound according to any one of claims 1 to 11;
preferably, the functional layer comprises an electron transporting layer, and the electron transporting layer comprises the nitrogen-containing compound;
preferably, the electron transporting layer further comprises LiQ;
preferably, the electronic element is an organic electroluminescence device or a photoelectric conversion device;
preferably, the organic electroluminescent device is a red light device.

13. An electronic device including the electronic element of claim 12.

## Patentansprüche

1. Stickstoffhaltige Verbindung mit einer Struktur der chemischen Formel 1: 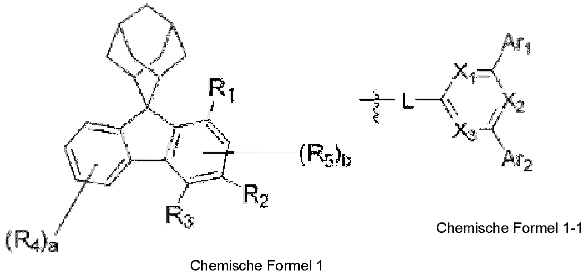 wobei 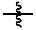 eine chemische Bindung darstellt;
X₁, X₂ und X₃ gleich oder verschieden sind und jeweils unabhängig aus C oder N ausgewählt sind und zumindest eines N ist;
R₁, R₂ und R₃ jeweils unabhängig aus Wasserstoff oder einer Gruppe, die durch die chemische Formel 1-1 dargestellt ist, ausgewählt sind und eines und nur eines von R₁, R₂ und R₃ die Gruppe aufweist, die durch die chemische Formel 1-1 dargestellt ist; wenn R₁, R₂ oder R₃ aus Wasserstoff ausgewählt ist, R₁, R₂ oder R₃ durch R₅ ersetzt werden kann;
R₄, R₅ gleich oder verschieden sind und jeweils unabhängig aus Deuterium, Halogen, einem Heteroaryl mit 3 bis 20 Kohlenstoffatomen, einem Aryl mit 6 bis 20 Kohlenstoffatomen, einem Trialkylsilyl mit 3 bis 12 Kohlenstoffatomen, einem Arylsilyl mit 8 bis 12 Kohlenstoffatomen, einem Alkyl mit 1 bis 10 Kohlenstoffatomen, einem Haloalkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen, einem Alkoxy mit 1 bis 10 Kohlenstoffatomen und einem Alkylthio mit 1 bis 10 Kohlenstoffatomen, einem Aryloxy mit 6 bis 18 Kohlenstoffatomen, einem Arylthio mit 6 bis 18 Kohlenstoffatomen, einem Phosphoryloxy mit 6 bis 18 Kohlenstoffatomen ausgewählt sind;
a aus 0, 1, 2, 3 oder 4 ausgewählt wird; wenn a größer als 1 ist, beliebige zwei R₄ gleich oder verschieden sind;
b aus 0, 1, 2 oder 3 ausgewählt wird, wenn b größer als 1 ist, beliebige zwei R₅ gleich oder verschieden sind;
L aus einer einzelnen Bindung, einem substituierten oder unsubstituierten Arylen mit 6 bis 30 Kohlenstoffatomen, oder einem substituierten oder unsubstituierten Heteroarylen mit 3 bis 30 Kohlenstoffatomen ausgewählt ist;
Ar₁ und Ar₂ jeweils unabhängig aus den folgenden substituierten oder unsubstituierten Gruppen ausgewählt sind: eine Alkylgruppe mit 1 bis 20 Kohlenstoffatomen, eine Cycloalkylgruppe mit 3 bis 20 Kohlenstoffatomen, ein Aryl mit 6 bis 30 Kohlenstoffatomen, ein Heteroaryl mit 3 bis 30 Kohlenstoffatomen;
die Substituenten von L aus Deuterium, Halogen, Cyano, einem Heteroaryl mit 3 bis 20 Kohlenstoffatomen, einem Aryl mit 6 bis 20 Kohlenstoffatomen, einem Trialkylsilyl mit 3 bis 12 Kohlenstoffatomen, einem Arylsilyl mit 8 bis 12 Kohlenstoffatomen, einem Alkyl mit 1 bis 10 Kohlenstoffatomen, einem Haloalkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen, einem Alkoxy mit 1 bis 10 Kohlenstoffatomen, und einem Alkylthio mit 1 bis 10 Kohlenstoffatomen, einem Aryloxy mit 6 bis 18 Kohlenstoffatomen, einem Arylthio mit 6 bis 18 Kohlenstoffatomen, einem Phosphoryloxy mit 6 bis 18 Kohlenstoffatomen ausgewählt sind;
die Substituenten in Ar₁ und Ar₂ gleich oder verschieden sind und jeweils unabhängig aus Deuterium, Halogen, einem Heteroaryl mit 3 bis 20 Kohlenstoffatomen, einem Aryl mit 6 bis 20 Kohlenstoffatomen und einem Trialkylsilyl mit 3 bis 12 Kohlenstoffatomen, einem Arylsilyl mit 8 bis 12 Kohlenstoffatomen, einem Alkyl mit 1 bis 10 Kohlenstoffatomen, einem Haloalkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen, einem Alkoxy mit 1 bis 10 Kohlenstoffatomen, einem Alkylthio mit 1 bis 10 Kohlenstoffatomen, einem Aryloxy mit 6 bis 18 Kohlenstoffatomen, einem Arylthio mit 6 bis 18 Kohlenstoffatomen, einem Phosphoryloxy mit 6 bis 18 Kohlenstoffatomen ausgewählt sind.

2. Stickstoffhaltige Verbindung nach Anspruch 1, wobei L aus einer einzelnen Bindung, einem substituierten oder unsubstituierten Arylen mit 6 bis 25 Kohlenstoffatomen und einem substituierten oder unsubstituierten Heteroarylen mit 5 bis 18 Kohlenstoffatomen ausgewählt ist.

3. Stickstoffhaltige Verbindung nach Anspruch 1, wobei L aus einer einzelnen Bindung, einem substituierten oder unsubstituierten Arylen mit 6 bis 25 Kohlenstoffatomen und einem substituierten oder unsubstituierten Heteroarylen mit 5 bis 20 Kohlenstoffatomen ausgewählt ist.

4. Stickstoffhaltige Verbindung nach Anspruch 1, wobei L aus einer einzelnen Bindung, einem substituierten oder unsubstituierten Phenylen, einem substituierten oder unsubstituierten Naphtylen und einem substituierten oder unsubstituierten Phenanthrylen, einem substituierten oder unsubstituierten Anthracenylen, einem substituierten oder unsubstituierten Fluorenylen, einem substituierten oder unsubstituierten Biphenylen, einem substituierten oder unsubstituierten Terphenylen, einem substituierten oder unsubstituierten Pyridylen, einem substituierten oder unsubstituiertem Dibenzofuranyl, einem substituierten oder unsubstituierten Dibenzothienyl, einem substituierten oder unsubstituierten N-Phenylcarbazolyliden ausgewählt ist;
wobei vorzugsweise die Substituenten in L aus Deuterium, Halogen, Cyano, einem Aryl mit 6 bis 12 Kohlenstoffatomen, einer Heteroarylgruppe mit 5 bis 18 Kohlenstoffatomen, einem Alkyl mit 1 bis 5 Kohlenstoffatomen, einem Cycloalkyl mit 3 bis 10 Kohlenstoffatomen ausgewählt sind.

5. Stickstoffhaltige Verbindung nach Anspruch 1, wobei L aus einer einzelnen Bindung oder der Gruppe bestehend aus Gruppen der chemischen Formel j-1 bis j-13 ausgewählt ist: 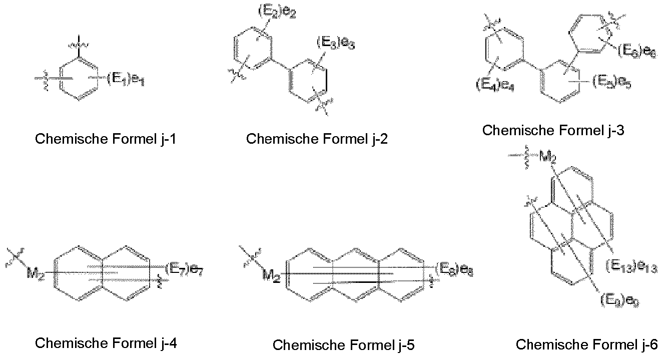 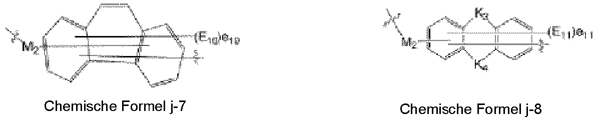 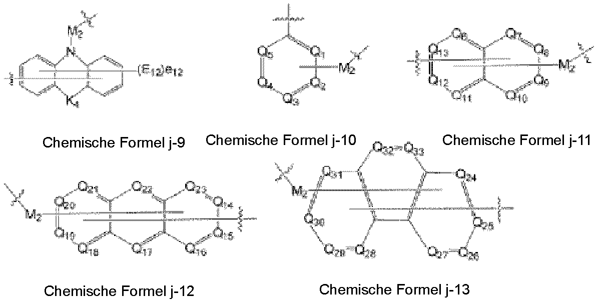
wobei M₂ aus einer einzelnen Bindung oder 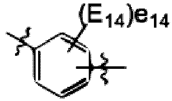 ausgewählt ist,
Q₁ bis Q₅ jeweils unabhängig aus N oder C(Fs) ausgewählt sind und zumindest eines von Q₁ bis Q₅ aus N ausgewählt ist; wenn zwei oder mehr von Q₁ bis Q₅ aus C(F₅) ausgewählt sind, beliebige zwei von F₅ gleich oder verschieden sind;
Q₆ bis Q₁₃ jeweils unabhängig aus N oder C(F₆) ausgewählt sind, und zumindest eines von Q₆ bis Q₁₃ aus N ausgewählt ist; wenn zwei oder mehr von Q₆ bis Q₁₃ aus C(F₆) ausgewählt sind, beliebige zwei von F₆ gleich oder verschieden sind;
Q₁₄ bis Q₂₃ jeweils unabhängig aus N oder C(F₇) ausgewählt sind, und zumindest eines von Q₁₄ bis Q₂₃ aus N ausgewählt ist; wenn zwei oder mehr von Q₁₄ bis Q₂₃ aus C(F₇) ausgewählt sind, beliebige zwei von F₇ gleich oder verschieden sind;
Q₂₄ bis Q₃₃ jeweils unabhängig aus N oder C(F₈) ausgewählt sind, und zumindest eines von Q₂₄ bis Q₃₃ aus N ausgewählt ist; wenn zwei oder mehr von Q₂₄ bis Q₃₃ aus C(F₈) ausgewählt sind, beliebige zwei von F₈ gleich oder verschieden sind;
E₁ bis E₁₄, F₅ bis F₈ jeweils unabhängig aus Deuterium, Halogen, Cyano, einem Heteroaryl mit 3 bis 18 Kohlenstoffatomen, einem Aryl mit 6 bis 18 Kohlenstoffatomen ausgewählt sind und optional durch Deuterium, Fluor, Chlor oder Cyano, einem Trialkylsilyl mit 3 bis 12 Kohlenstoffatomen, einem Arylsilyl mit 8 bis 12 Kohlenstoffatomen, einem Alkyl mit 1 bis 10 Kohlenstoffatomen, einem Haloalkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen, einem Alkoxy mit 1 bis 10 Kohlenstoffatomen, einem Alkylthio mit 1 bis 10 Kohlenstoffatomen, einem Aryloxy mit 6 bis 18 Kohlenstoffatomen, einem Arylthio mit 6 bis 18 Kohlenstoffatomen, einem Phosphoryloxy mit 6 bis 18 Kohlenstoffatomen substituiert sind;
ein beliebiges von F₅ bis F₈ auch unabhängig aus Wasserstoff ausgewählt werden kann;
eᵣ die Anzahl an Substituenten Eᵣ ist, r eine beliebige ganze Zahl von 1 bis 14 ist; wenn r aus 1, 2, 3, 4, 5, 6, 9, 13 oder 14 ausgewählt ist, eᵣ aus 0, 1, 2, 3 oder 4 ausgewählt ist; wenn r aus 7 oder 11 ausgewählt ist, eᵣ aus 0, 1, 2, 3, 4, 5 oder 6 ausgewählt ist; wenn r 12 ist, eᵣ aus 0, 1, 2, 3, 4, 5, 6 oder 7 ausgewählt ist; wenn r aus 8 oder 10 ausgewählt ist, eᵣ aus 0, 1, 2, 3, 4, 5, 6, 7 oder 8 ausgewählt ist; wenn eᵣ größer als 1 ist, beliebige zwei von Eᵣ gleich oder verschieden sind;
K₃ aus O, S, Se, N(E₁₅), C(E₁₆E₁₇), Si(E₁₆E₁₇) ausgewählt ist; wobei E₁₅, E₁₆, E₁₇ jeweils unabhängig aus einem Aryl mit 6 bis 18 Kohlenstoffatomen, einem Heteroaryl mit 3 bis 18 Kohlenstoffatomen, einem Alkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einer Heterocycloalkenylgruppe mit 4 bis 10 Kohlenstoffatomen ausgewählt ist, oder die vorherigen E₁₆ und E₁₇ miteinander verbunden sind, um einen Ring mit dem Atom zu bilden, mit dem sie für gewöhnlich verbunden sind;
K₄ aus einer einzelnen Bindung, O, S, Se, N(E₁₈), C(E₁₉E₂₀), Si(E₁₉E₂₀) ausgewählt sind; wobei E₁₈, E₁₉, E₂₀ jeweils unabhängig aus einem Aryl mit 6 bis 18 Kohlenstoffatomen, einem Heteroaryl mit 3 bis 18 Kohlenstoffatomen, einem Alkyl mit 1 bis 10 Kohlenstoffatomen, Alkenyl mit 2 bis 6 Kohlenstoffatomen, Alkynyl mit 2 bis 6 Kohlenstoffatomen, einem Cycloalkenyl mit 5 bis 10 Kohlenstoffatomen, einem Heterocyclenyl mit 4 bis 10 Kohlenstoffatomen ausgewählt ist oder die vorherigen E₁₉ und E₂₀ miteinander verbunden sind, um einen Ring mit dem Atom zu bilden, mit dem sie für gewöhnlich verbunden sind.

6. Stickstoffhaltige Verbindung nach Anspruch 1, wobei L aus einer einzelnen Bindung oder der Gruppe bestehend aus den folgenden Gruppen ausgewählt ist:

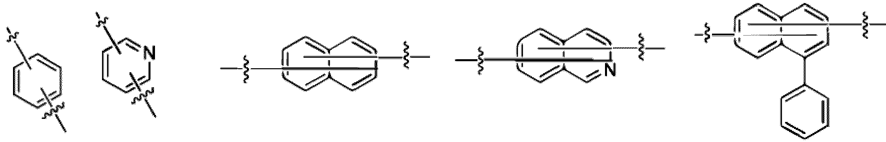

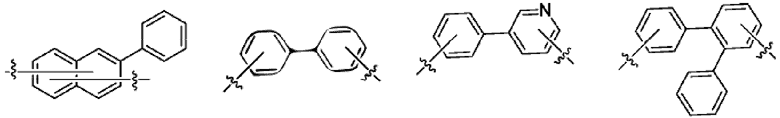

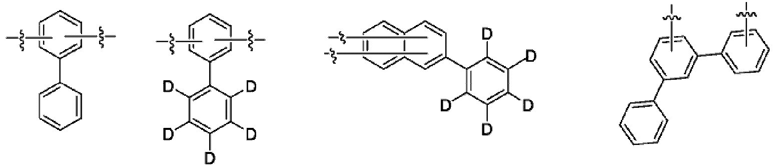

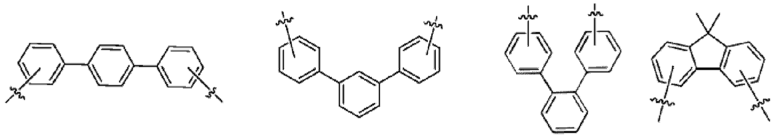

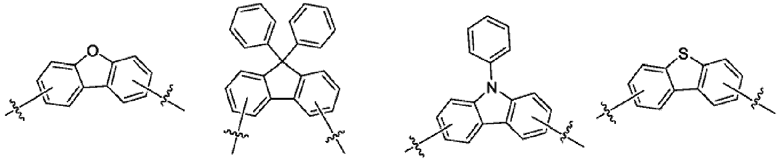

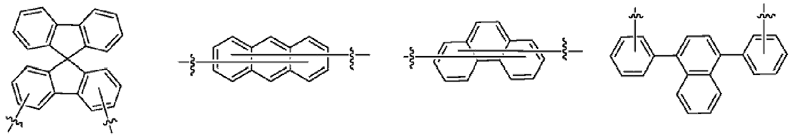

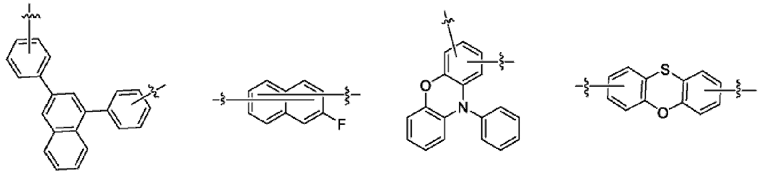

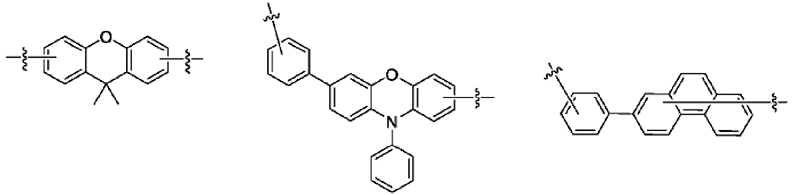

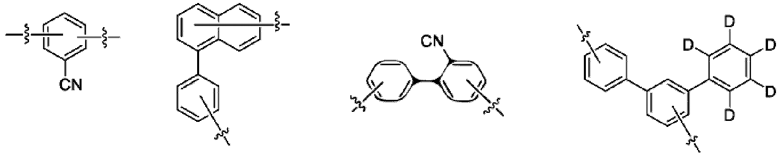

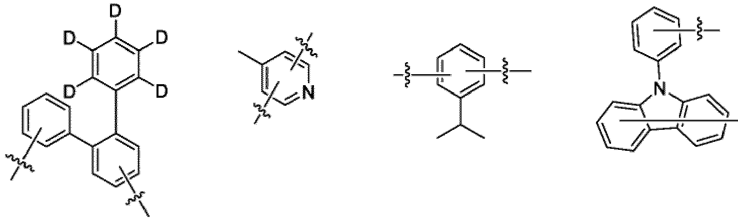

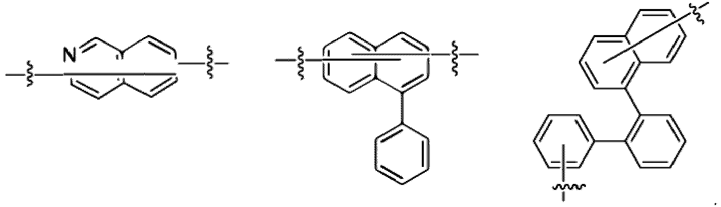

7. Stickstoffhaltige Verbindung nach Anspruch 1, wobei Ar₁ und Ar₂ jeweils unabhängig aus den folgenden substituierten oder unsubstituierten Gruppen ausgewählt sind: einem Aryl mit 6 bis 25 ringförmigen Kohlenstoffatomen, oder einem Heteroaryl mit 5 bis 20 ringförmigen Kohlenstoffatomen.

8. Stickstoffhaltige Verbindung nach Anspruch 1, wobei Ar₁ und Ar₂ jeweils unabhängig aus substituiertem oder unsubstituiertem Aryl mit 6 bis 25 Kohlenstoffatomen oder substituiertem oder unsubstituiertem Heteroaryl mit 5 bis 24 Kohlenstoffatomen ausgewählt sind;
wobei vorzugsweise die Substituenten Ar₁ und Ar₂ jeweils unabhängig aus Deuterium, Halogen, einem Aryl mit 6 bis 12 Kohlenstoffatomen, einer Heteroarylgruppe mit 3 bis 20 Kohlenstoffatomen, einem Alkyl mit 1 bis 5 Kohlenstoffatomen, einem Cycloalkyl mit 3 bis 10 Kohlenstoffatomen ausgewählt sind.

9. Stickstoffhaltige Verbindung nach Anspruch 1, wobei Ar₁ und Ar₂ jeweils unabhängig aus Substituenten ausgewählt sind, die durch die folgende chemische Formel i-1 bis i-15 dargestellt sind: 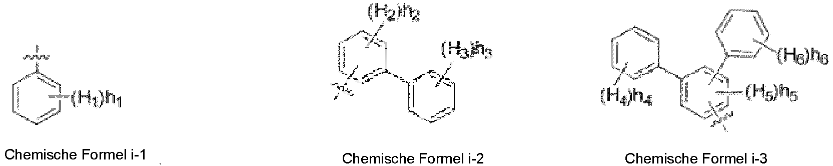 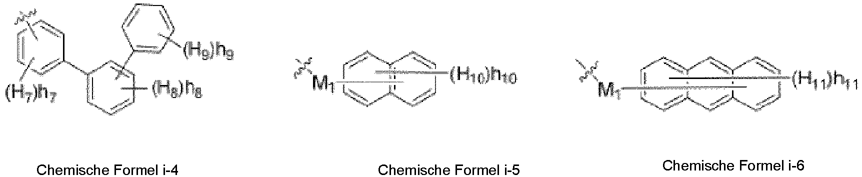 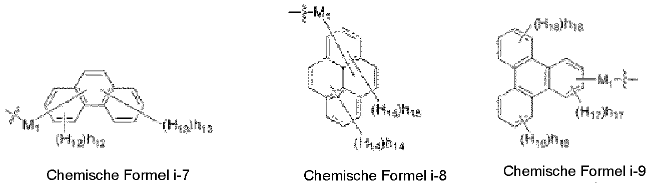 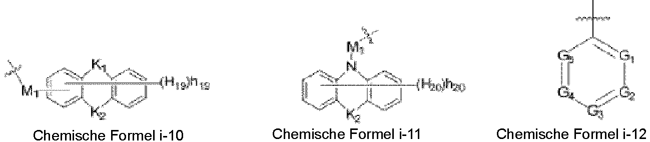 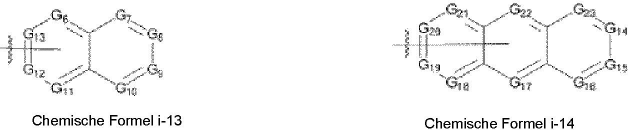 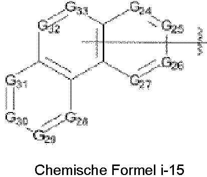
wobei M₁ aus einer einzelnen Bindung oder 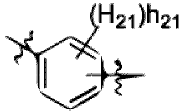 ausgewählt ist;
G₁ bis G₅ jeweils unabhängig aus N oder C(F₁) ausgewählt sind, und zumindest eines von G₁ bis G₅ aus N ausgewählt ist; wenn zwei oder mehr von G₁ bis G₅ aus C(F₁) ausgewählt sind, beliebige zwei F₁ gleich oder verschieden sind;
G₆ bis G₁₃ jeweils unabhängig aus N oder C(F₂) ausgewählt sind, und zumindest eines von G₆ bis G₁₃ aus N ausgewählt ist; wenn zwei oder mehr von G₆ bis G₁₃ aus C(F₂) ausgewählt sind, beliebige zwei F₂ gleich oder verschieden sind;
G₁₄ bis G₂₃ jeweils unabhängig aus N oder C(F₃) ausgewählt sind, und zumindest eines von G₁₄ bis G₂₃ aus N ausgewählt ist; wenn zwei oder mehr von G₁₄ bis G₂₃ aus C(F₃) ausgewählt sind, beliebige zwei F₂ gleich oder verschieden sind;
G₂₄ bis G₃₃ jeweils unabhängig ausgewählt sind aus N oder C(F₄), und zumindest eines von G₂₄ bis G₃₃ ausgewählt ist aus N; wenn zwei oder mehr von G₂₄ bis G₃₃ aus C(F₄) ausgewählt sind, beliebige zwei F₂ gleich oder verschieden sind;
H₁ bis H₂₁, F₁ bis F₄ jeweils unabhängig aus Deuterium, Halogen, einem Heteroaryl mit 3 bis 18 Kohlenstoffatomen, einem Trialkylsilyl mit 3 bis 12 Kohlenstoffatomen, einem Arylsilyl mit 8 bis 12 Kohlenstoffatomen, einem Alkyl mit 1 bis 10 Kohlenstoffatomen, einem Haloalkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen, einem Alkoxy mit 1 bis 10 Kohlenstoffatomen, einem Alkylthio mit 1 bis 10 Kohlenstoffatomen, einem Aryloxy mit 6 bis 18 Kohlenstoffatomen, einem Arylthio mit 6 bis 18 Kohlenstoffatomen, einem Phosphoryloxy mit 6 bis 18 Kohlenstoffatomen ausgewählt sind;
ein beliebiges von H₄ bis H₂₀ auch unabhängig aus einem Aryl mit 6 bis 18 Kohlenstoffatomen ausgewählt werden kann;
ein beliebiges von F₁ bis F₄ auch unabhängig aus Wasserstoff und einem Aryl mit 6 bis 18 Kohlenstoffatomen ausgewählt werden kann;
hₖ die Anzahl an Substituenten Hₖ ist, k eine beliebige ganze Zahl von 1 bis 21 ist; wobei, wenn k aus 5 oder 17 ausgewählt ist, hₖ aus 0, 1, 2 oder 3 ausgewählt ist; wenn k aus 2, 7, 8, 12, 15, 16, 18 oder 21 ausgewählt ist, hₖ aus 0, 1, 2, 3 oder 4 ausgewählt ist; wenn k 1, 3, 4, 6, 9 oder 14 ist, hₖ aus 0, 1, 2, 3, 4 oder 5 ausgewählt ist; wenn k 13 ist, hₖ aus 0, 1, 2, 3, 4, 5, oder 6 ausgewählt ist; wenn k aus 10 oder 19 ausgewählt ist, hₖ aus 0, 1, 2, 3, 4, 5, 6 oder 7 ausgewählt ist; wenn k aus 20 ausgewählt ist, hₖ aus 0, 1, 2, 3, 4, 5, 6, 7 oder 8 ausgewählt ist; wenn k 11 ist, hₖ aus 0, 1, 2, 3, 4, 5, 6, 7, 8 oder 9 ausgewählt ist;
wenn hₖ größer als 1 ist, beliebige zwei Hₖ gleich oder verschieden sind;
K₁ aus O, S, Se, N (H₂₂), C (H₂₃,H₂₄), Si (H₂₃H₂₄) ausgewählt ist; wobei H₂₂, H₂₃ und H₂₄ jeweils unabhängig aus einem Aryl mit 6 bis 18 Kohlenstoffatomen, einem Heteroaryl mit 3 bis 18 Kohlenstoffatomen, einem Alkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen ausgewählt sind oder die vorherigen H₂₃ und H₂₄ miteinander verbunden sind, um einen Ring mit dem Atom zu bilden, mit dem sie für gewöhnlich verbunden sind;
K₂ aus einer einzelnen Bindung, O, S, Se, N(H₂₅), C(H₂₆H₂₇), Si(H₂₆H₂₇) ausgewählt ist;
wobei H₂₅, H₂₆, H₂₇ jeweils unabhängig aus einem Aryl mit 6 bis 18 Kohlenstoffatomen, einem Heteroaryl mit 3 bis 18 Kohlenstoffatomen, einem Alkyl mit 1 bis 10 Kohlenstoffatomen, einem Alkenyl mit 2 bis 6 Kohlenstoffatomen, einem Alkynyl mit 2 bis 6 Kohlenstoffatomen, einem Cycloalkyl mit 3 bis 10 Kohlenstoffatomen, einem Heterocycloalkyl mit 2 bis 10 Kohlenstoffatomen, einem Cycloalkenyl mit 5 bis 10 Kohlenstoffatomen, einem Heterocyclenyl mit 4 bis 10 Kohlenstoffatomen ausgewählt sind oder die vorherigen H₂₆ und H₂₇ miteinander verbunden sind, um einen Ring mit dem Atom zu bilden, mit dem sie für gewöhnlich verbunden sind.

10. Stickstoffhaltige Verbindung nach Anspruch 1, wobei Ar₁ und Ar₂ jeweils unabhängig aus der Gruppe bestehend aus den folgenden Gruppen ausgewählt sind:

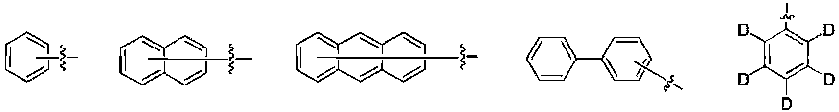

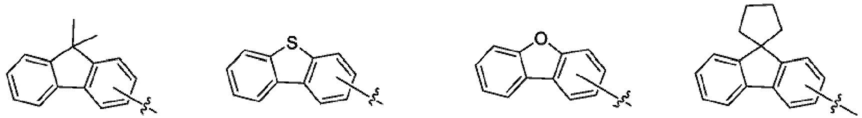

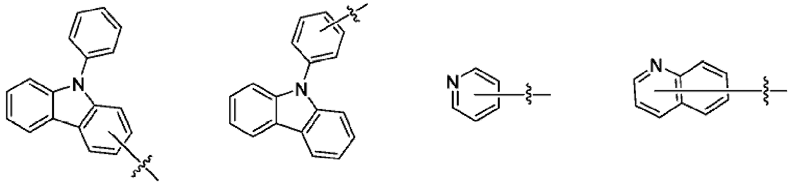

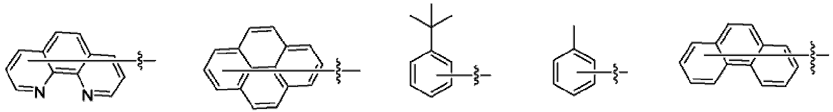

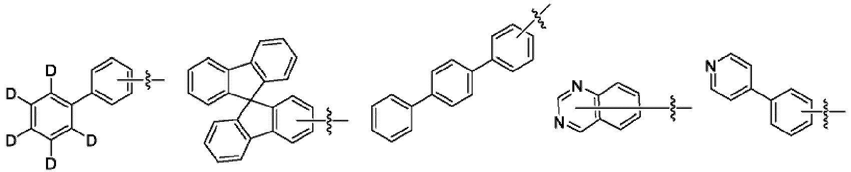

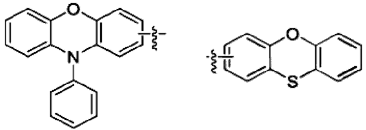

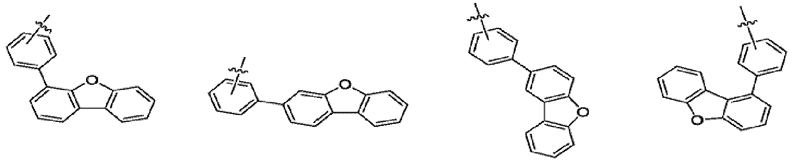

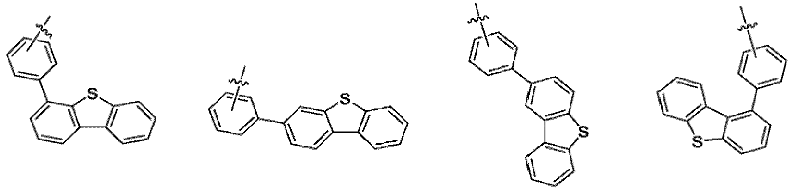

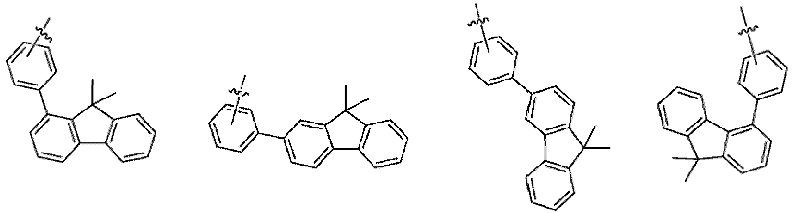

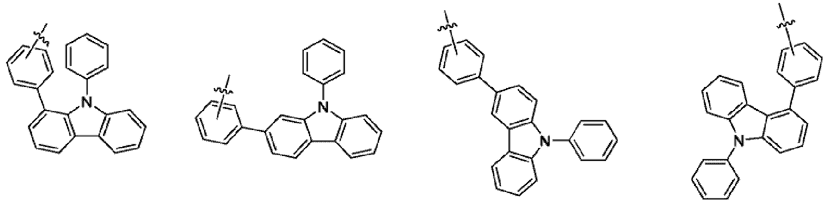

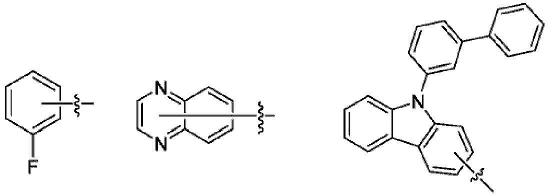

11. Stickstoffhaltige Verbindung nach Anspruch 1, wobei die stickstoffhaltige Verbindung jeweils aus der Gruppe bestehend aus den folgenden Verbindungen ausgewählt ist:

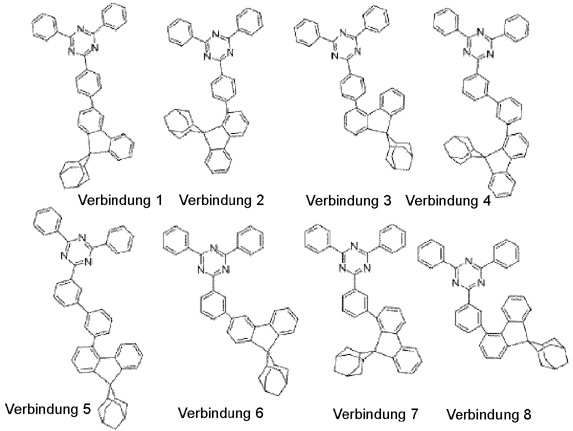

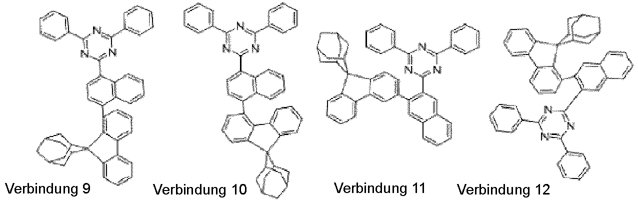

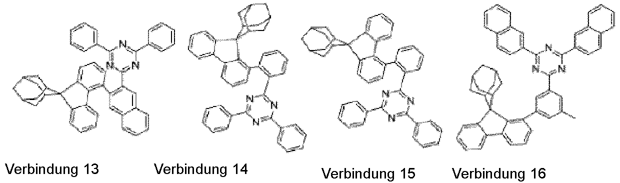

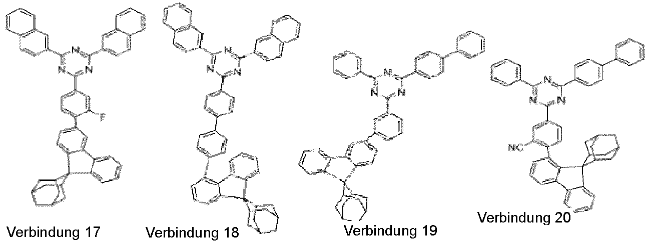

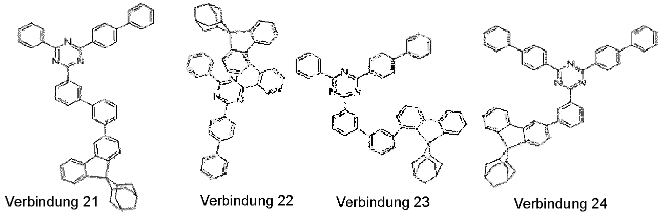

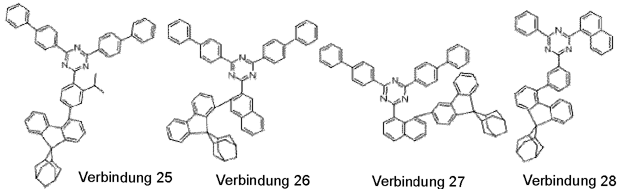

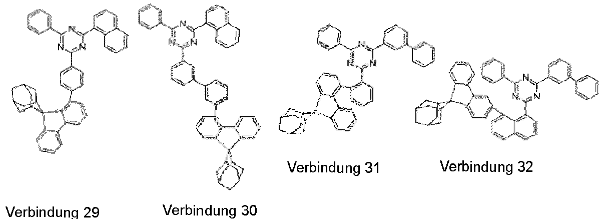

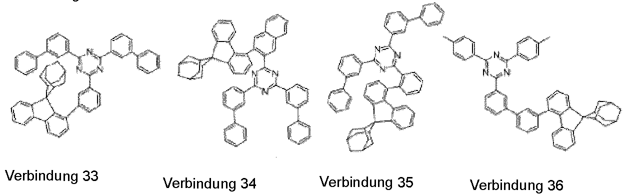

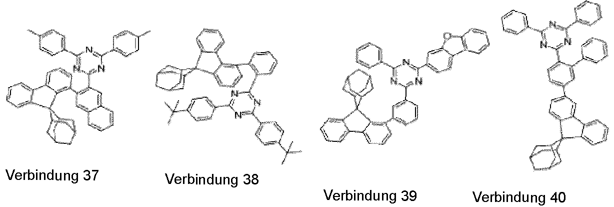

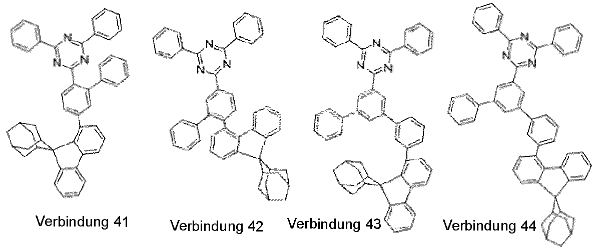

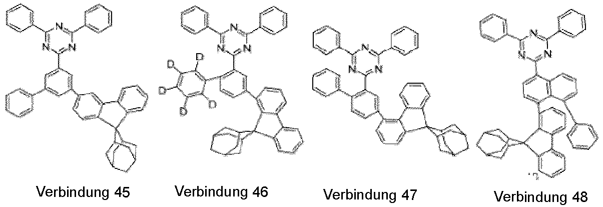

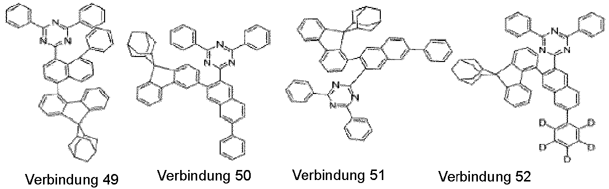

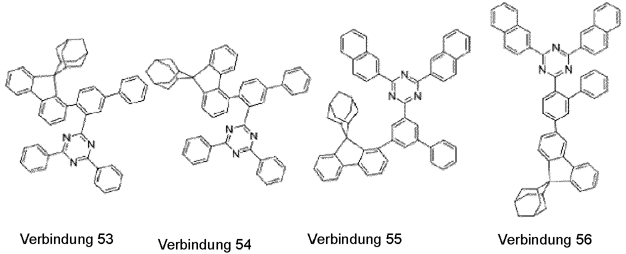

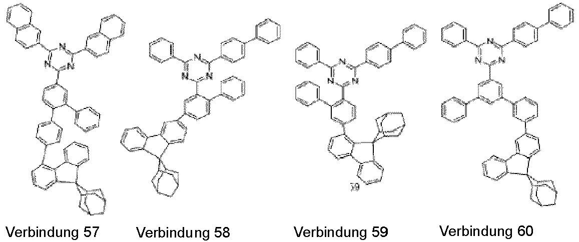

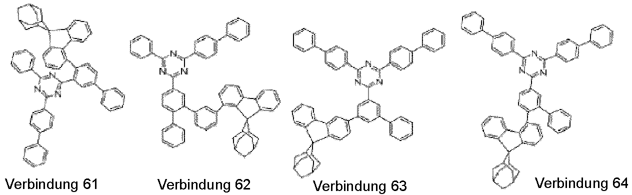

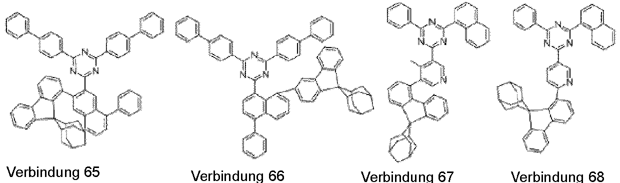

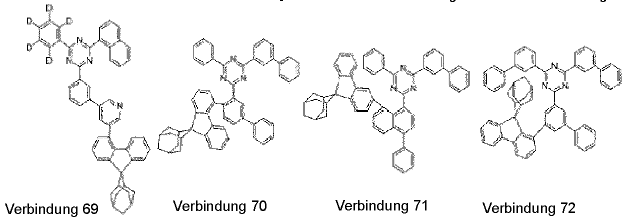

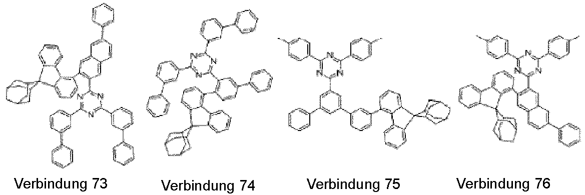

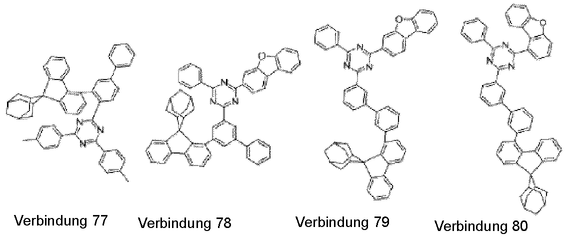

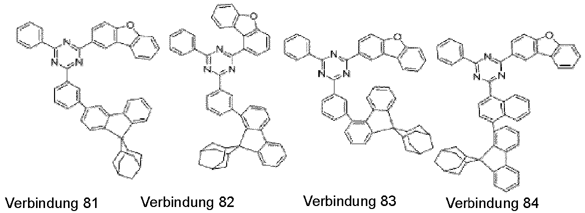

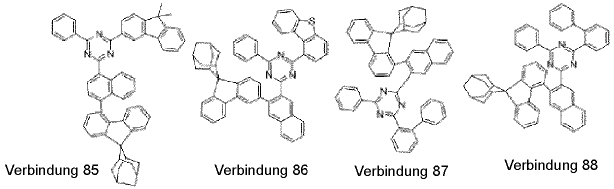

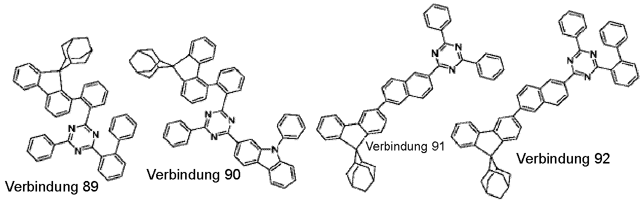

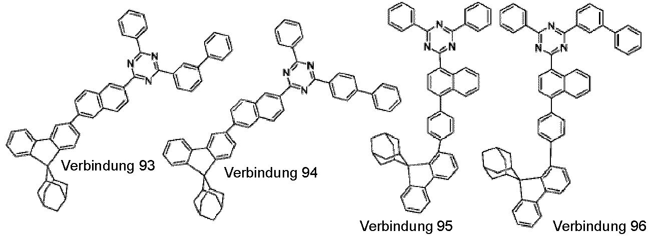

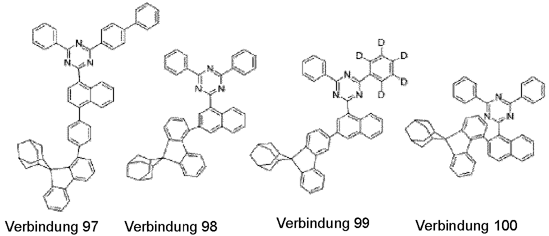

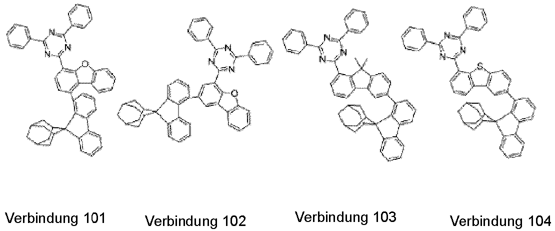

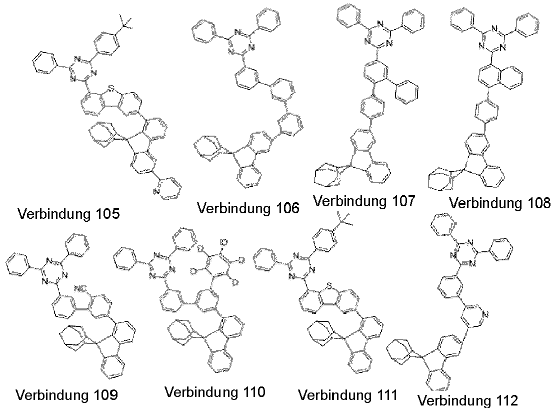

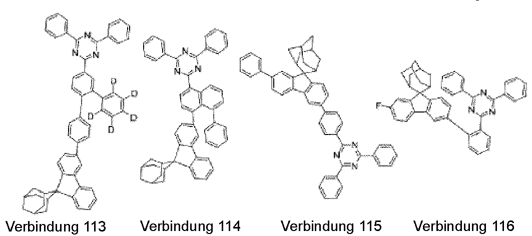

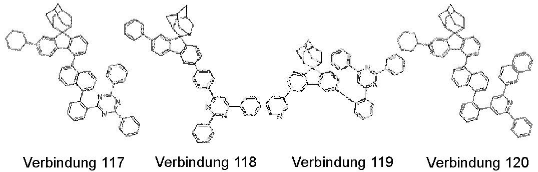

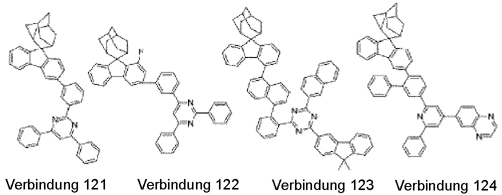

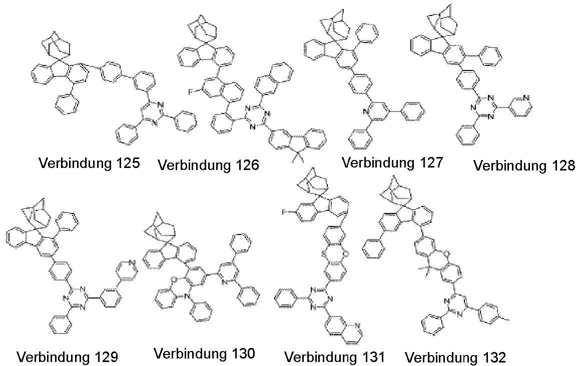

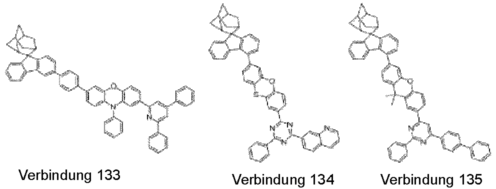

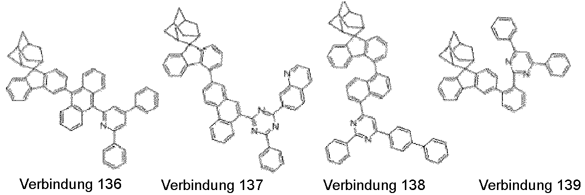

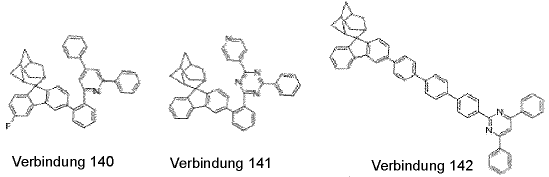

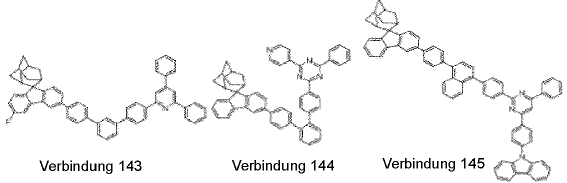

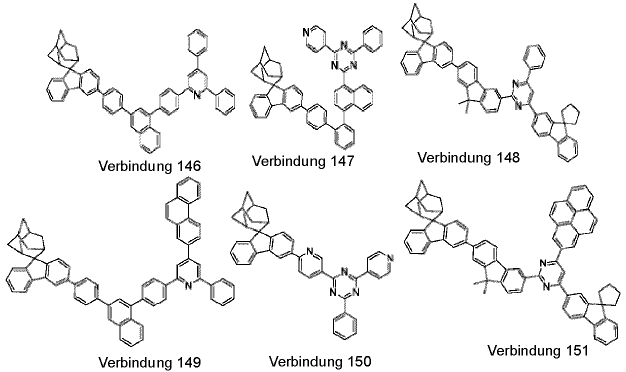

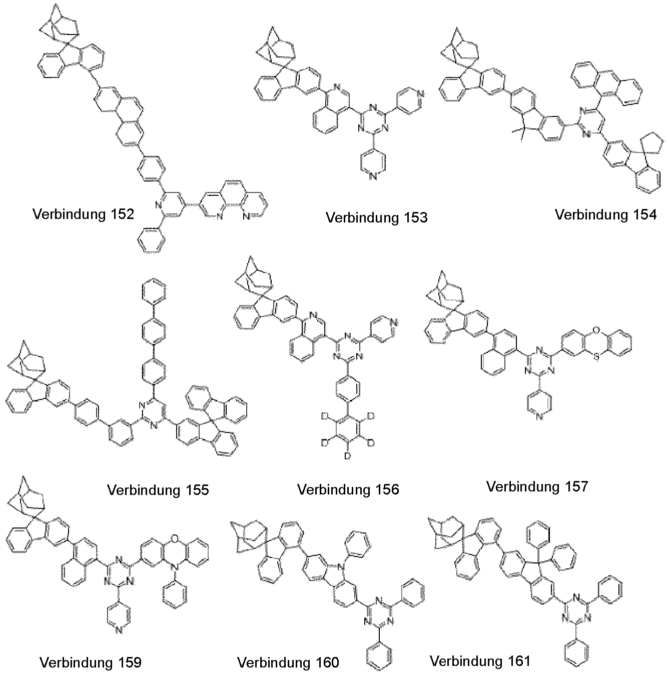

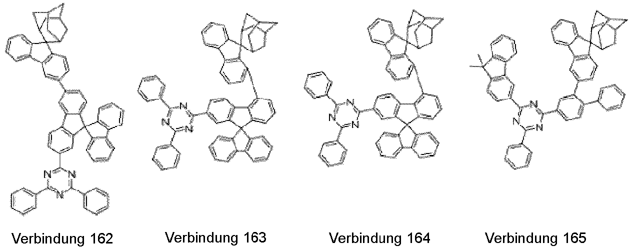

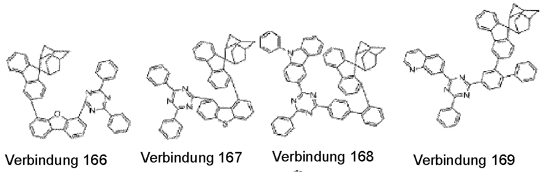

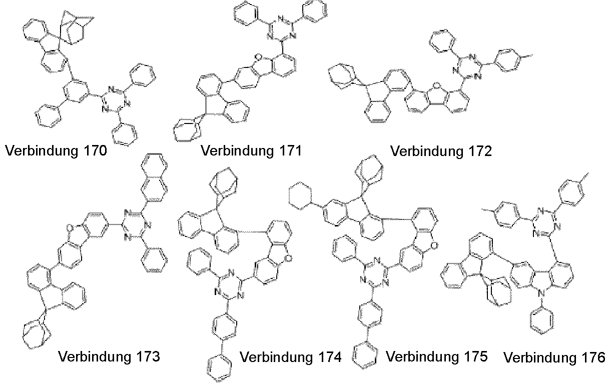

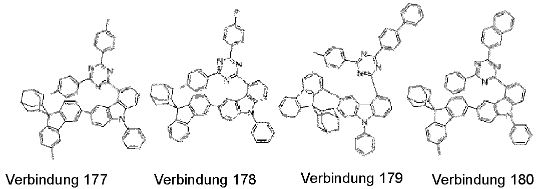

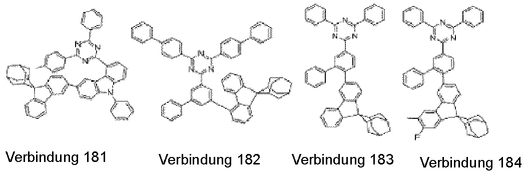

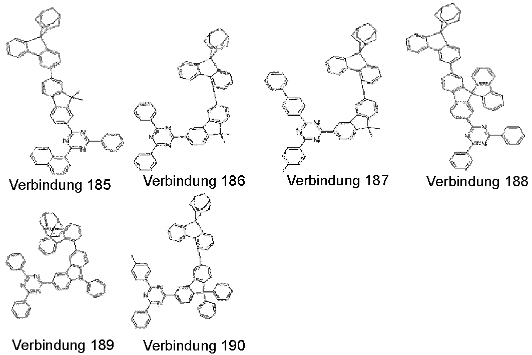

12. Elektronisches Element, wobei das elektronische Element eine Anode und eine Kathode, die entgegengesetzt angeordnet sind, und eine funktionelle Schicht, die zwischen der Anode und der Kathode angeordnet ist, umfasst;
wobei die funktionelle Schicht die stickstoffhaltige Verbindung nach einem der Ansprüche 1 bis 11 umfasst;
wobei vorzugsweise die funktionelle Schicht eine Elektronentransportschicht umfasst und die Elektronentransportschicht die stickstoffhaltige Verbindung umfasst;
wobei vorzugsweise die Elektronentransportschicht ferner LiQ umfasst;
wobei vorzugsweise das elektronische Element eine organische Elektrolumineszenzvorrichtung oder eine fotoelektrische Umwandlungsvorrichtung ist;
wobei vorzugsweise die organische Elektrolumineszenzvorrichtung eine Rotlichtvorrichtung ist.

13. Elektronische Vorrichtung, die das elektronische Element nach Anspruch 12 einschließt.

## Revendications

1. Composé contenant de l'azote présentant une structure de formule chimique 1 : 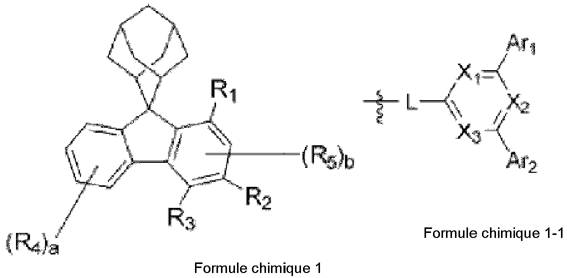
dans lequel 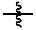 représente une liaison chimique ;
X₁, X₂, et X₃ sont identiques ou différents, et sont choisis chacun indépendamment parmi C ou N, et au moins un est N ;
R₁, R₂ et R₃ sont choisis chacun indépendamment parmi un hydrogène ou un groupe représenté par la formule chimique 1-1, et un et un seul de R₁, R₂ et R₃ présente le groupe représenté par la formule chimique 1-1 ;
lorsque R₁, R₂ ou R₃ R est choisi parmi l'hydrogène, ledit R₁, R₂ ou R₃ peut être remplacé par R₅ ;
R₄, R₅ sont identiques ou différents et sont choisis chacun indépendamment parmi le deutérium, un halogène, un hétéroaryle présentant 3 à 20 atomes de carbone, un aryle présentant 6 à 20 atomes de carbone, un trialkylsilyle présentant 3 à 12 atomes de carbone, un arylsilyle présentant 8 à 12 atomes de carbone, un alkyle présentant 1 à 10 atomes de carbone, un haloalkyle présentant 1 à 10 atomes de carbone, un alcényle présentant 2 à 6 atomes de carbone, un alcynyle ayant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, un alcoxy présentant 1 à 10 atomes de carbone et un alkylthio présentant 1 à 10 atomes de carbone, un aryloxy présentant 6 à 18 atomes de carbone, un arylthio présentant 6 à 18 atomes de carbone, un phosphoryloxy présentant 6 à 18 atomes de carbone ;
a est choisi parmi 0, 1, 2, 3 ou 4 ; lorsque a est supérieur à 1, deux R₄ quelconques sont identiques ou différents ;
b est choisi parmi 0, 1, 2 ou 3, lorsque b est supérieur à 1, deux R₅ quelconques sont identiques ou différents ;
L est choisi parmi une liaison simple, un arylène substitué ou non substitué présentant 6 à 30 atomes de carbone, ou un hétéroarylène substitué ou non substitué présentant 3 à 30 atomes de carbone ;
Ar₁ et Ar₂ sont choisis chacun indépendamment parmi les groupes substitués ou non substitués suivants : un groupe alkyle présentant 1 à 20 atomes de carbone, un groupe cycloalkyle présentant 3 à 20 atomes de carbone, un aryle présentant 6 à 30 atomes de carbone, un hétéroaryle présentant 3 à 30 atomes de carbone ;
les substituants de L sont choisis parmi le deutérium, un halogène, un cyano, un hétéroaryle présentant 3 à 20 atomes de carbone, un aryle présentant 6 à 20 atomes de carbone, un trialkylsilyle présentant 3 à 12 atomes de carbone, un arylsilyle présentant 8 à 12 atomes de carbone, un alkyle présentant 1 à 10 atomes de carbone, un haloalkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, un alcoxy présentant 1 à 10 atomes de carbone, et un alkylthio présentant 1 à 10 atomes de carbone, un aryloxy présentant 6 à 18 atomes de carbone, un arylthio présentant 6 à 18 atomes de carbone, un phosphoryloxy présentant 6 à 18 atomes de carbone ;
les substituants dans Ar₁ et Ar₂ sont identiques ou différents et sont choisis chacun indépendamment parmi le deutérium, un halogène, un hétéroaryle présentant 3 à 20 atomes de carbone, un aryle présentant 6 à 20 atomes de carbone et un trialkylsilyle présentant 3 à 12 atomes de carbone, un arylsilyle présentant 8 à 12 atomes de carbone, un alkyle présentant 1 à 10 atomes de carbone, un haloalkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, un alcoxy présentant 1 à 10 atomes de carbone, un alkylthio présentant 1 à 10 atomes de carbone, un aryloxy présentant 6 à 18 atomes de carbone, un arylthio présentant 6 à 18 atomes de carbone, un phosphoryloxy présentant 6 à 18 atomes de carbone.

2. Composé contenant de l'azote selon la revendication 1, dans lequel L est choisi parmi une liaison simple, un arylène substitué ou non substitué présentant 6 à 25 atomes de carbone formant un cycle, et un hétéroarylène substitué ou non substitué présentant 5 à 18 atomes de carbone formant un cycle.

3. Composé contenant de l'azote selon la revendication 1, dans lequel L est choisi parmi une liaison simple, un arylène substitué ou non substitué présentant 6 à 25 atomes de carbone, et un hétéroarylène substitué ou non substitué présentant 5 à 20 atomes de carbone.

4. Composé contenant de l'azote selon la revendication 1, dans lequel L est choisi parmi une liaison simple, un phénylène substitué ou non substitué, un naphtylène substitué ou non substitué, et un phénanthrylène substitué ou non substitué, un anthracénylène substitué ou non substitué, un fluorénylène substitué ou non substitué, un biphénylène substitué ou non substitué, un terphénylène substitué ou non substitué, un pyridylène substitué ou non substitué, un dibenzofuranyle substitué ou non substitué, un dibenzothiényle substitué ou non substitué, un N-phénylcarbazolylidène substitué ou non substitué ;
de préférence, les substituants dans L sont choisis parmi le deutérium, un halogène, un cyano, un aryle présentant 6 à 12 atomes de carbone, un groupe hétéroaryle présentant 5 à 18 atomes de carbone, un alkyle présentant 1 à 5 atomes de carbone, un cycloalkyle présentant 3 à 10 atomes de carbone.

5. Composé contenant de l'azote selon la revendication 1, dans lequel L est choisi parmi une liaison simple ou le groupe constitué de groupes de la formule chimique j-1 à j-13 : 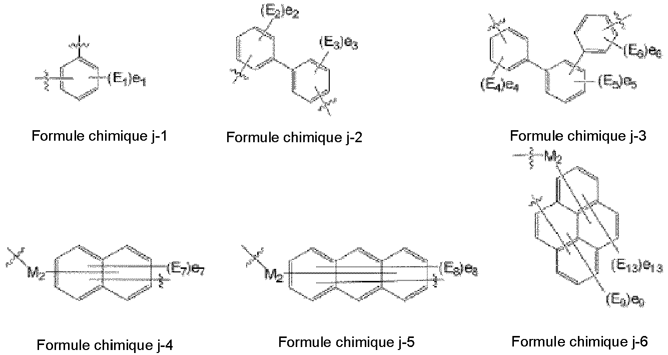 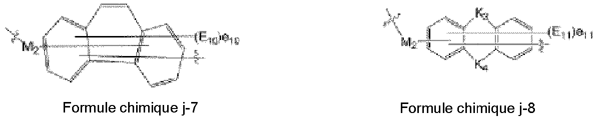 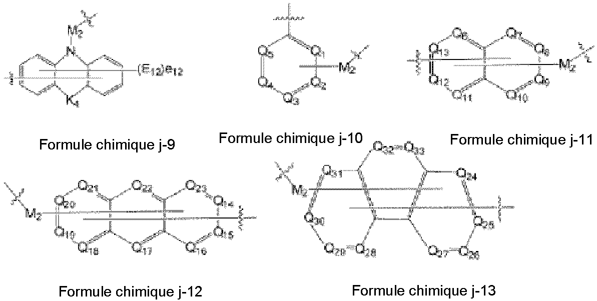
dans lequel M₂ est choisi parmi une liaison simple ou 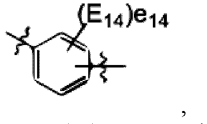
Q₁ à Q₅ sont choisis chacun indépendamment parmi N ou C(F₈), et au moins un de Q₁ à Q₅ est choisi parmi N ; lorsque deux ou plus de Q₁ à Q₅ sont choisis parmi C(F₅), deux quelconques de F₅ sont identiques ou différents ;
Q₆ à Q₁₃ sont choisis chacun indépendamment parmi N ou C(F₆), et au moins un de Q₆ à Q₁₃ est choisi parmi N ; lorsque deux ou plus de Q₆ à Q₁₃ sont choisis parmi C(F₆), deux quelconques de F₆ sont identiques ou différents ;
Q₁₄ à Q₂₃ sont choisis chacun indépendamment parmi N ou C(F₇) et au moins un de Q₁₄ à Q₂₃ est choisi parmi N ; lorsque deux ou plus de Q₁₄ à Q₂₃ sont choisis parmi C(F₇), deux quelconques de F₇ sont identiques ou différents ;
Q₂₄ à Q₃₃ sont choisis chacun indépendamment parmi N ou C(F₈), et au moins un de Q₂₄ à Q₃₃ est choisi parmi N ; lorsque deux ou plus de Q₂₄ à Q₃₃ sont choisis parmi C(F₈), deux quelconques de F₃ sont identiques ou différents ;
E₁ à E₁₄, F₅ à F₈ sont choisis chacun indépendamment parmi le deutérium, un halogène, un cyano, un hétéroaryle présentant 3 à 18 atomes de carbone, un aryle présentant 6 à 18 atomes de carbone et en option substitué par le deutérium, le fluor, le chlore ou un cyano, un trialkylsilyle présentant 3 à 12 atomes de carbone, un arylsilyle présentant 8 à 12 atomes de carbone, un alkyle présentant 1 à 10 atomes de carbone, un haloalkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, un alcoxy présentant 1 à 10 atomes de carbone, un alkylthio présentant 1 à 10 atomes de carbone, un aryloxy présentant 6 à 18 atomes de carbone, un arylthio présentant 6 à 18 atomes de carbone, un phosphoryloxy présentant 6 à 18 atomes de carbone ;
un quelconque de F₅ à F₈ peut également être choisi indépendamment parmi l'hydrogène ;
eᵣ est le nombre de substituants Eᵣ, r est un nombre entier quelconque de 1 à 14 ; lorsque r est choisi parmi 1, 2, 3, 4, 5, 6, 9, 13 ou 14, eᵣ est choisi parmi 0, 1, 2, 3 ou 4 ; lorsque r est choisi parmi 7 ou 11, eᵣ est choisi parmi 0, 1, 2, 3, 4, 5 ou 6 ; lorsque r vaut 12, eᵣ est choisi parmi 0, 1, 2, 3, 4, 5, 6 ou 7 ; lorsque r est choisi parmi 8 ou 10, eᵣ est choisi parmi 0, 1, 2, 3, 4, 5, 6, 7 ou 8 ; lorsque eᵣ est supérieur à 1, deux quelconques de Eᵣ sont identiques ou différents ;
K₃ est choisi parmi O, S, Se, N(E₁₅), C(E₁₆F₁₇), Si(E₁₆E₁₇) ; dans lequel E₁₅, E₁₆, E₁₇ sont choisis chacun indépendamment parmi un aryle présentant 6 à 18 atomes de carbone, un hétéroaryle présentant 3 à 18 atomes de carbone, un alkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un groupe hétérocycloalcényle présentant 4 à 10 atomes de carbone, ou E₁₆ ci-dessus et E₁₇ sont reliés l'un à l'autre pour former un cycle avec l'atome auquel ils sont reliés conjointement ;
K₄ est choisi parmi une liaison simple, O, S, Se, N(E₁₈), C(E₁₉E₂₀), Si(E₁₉E₂₀) ; dans lequel E₁₈, E₁₉, E₂₀ sont choisis chacun indépendamment choisis parmi un aryle présentant 6 à 18 atomes de carbone, un hétéroaryle présentant 3 à 18 atomes de carbone, un alkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, ou E₁₉ et E₂₀ ci-dessus sont reliés l'un à l'autre pour former un cycle avec l'atome auquel ils sont reliés conjointement.

6. Composé contenant de l'azote selon la revendication 1, dans lequel L est choisi parmi une liaison simple ou le groupe constitué des groupes suivants :

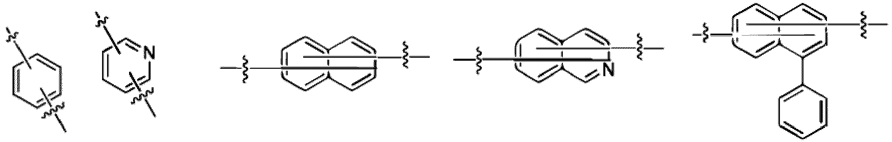

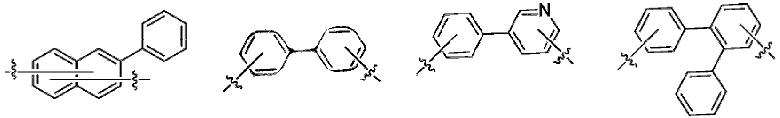

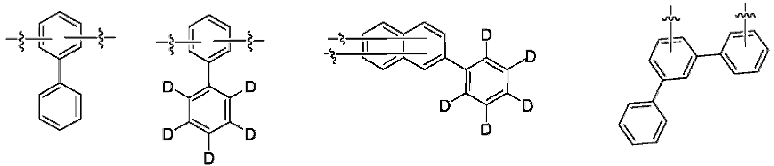

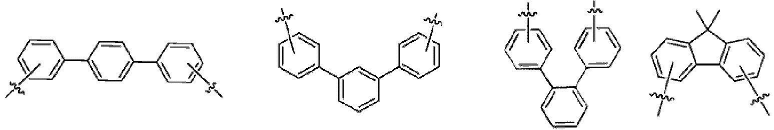

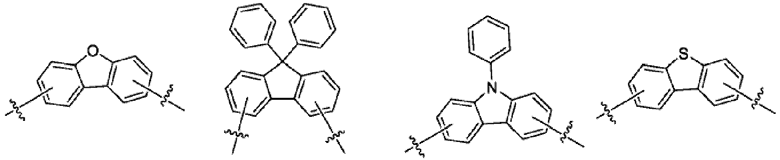

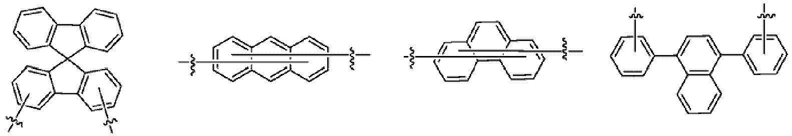

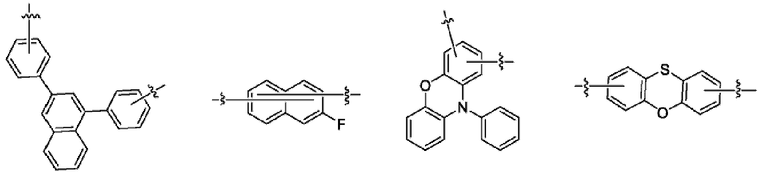

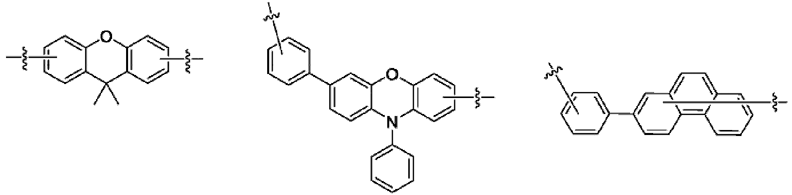

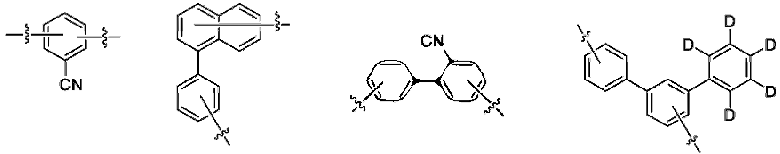

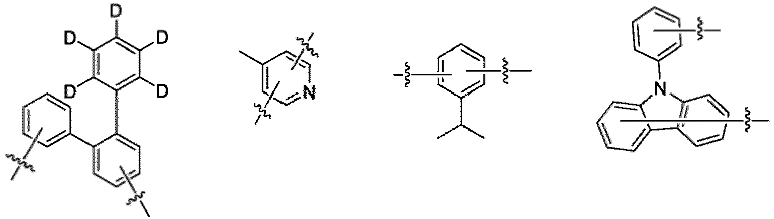

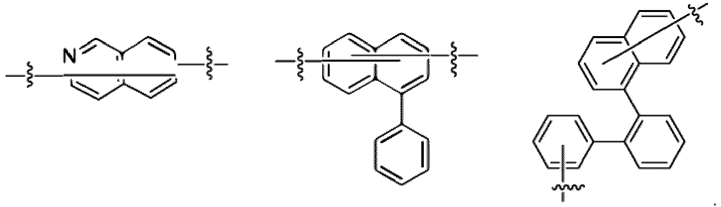

7. Composé contenant de l'azote selon la revendication 1, dans lequel Ar₁ et Ar₂ sont choisis chacun indépendamment parmi les groupes substitués ou non substitués suivants : un aryle présentant 6 à 25 atomes de carbone formant un cycle, ou un hétéroaryle présentant 5 à 20 atomes de carbone formant un cycle.

8. Composé contenant de l'azote selon la revendication 1, dans lequel Ar₁ et Ar₂ sont choisis chacun indépendamment parmi un aryle substitué ou non substitué présentant 6 à 25 atomes de carbone, ou un hétéroaryle substitué ou non substitué présentant 5 à 24 atomes de carbone ;
de préférence, les substituants dans Ar₁ et Ar₂ sont choisis chacun indépendamment parmi le deutérium, un halogène, un aryle présentant 6 à 12 atomes de carbone, un groupe hétéroaryle présentant 3 à 20 atomes de carbone, un alkyle présentant 1 à 5 atomes de carbone, un cycloalkyle présentant 3 à 10 atomes de carbone.

9. Composé contenant de l'azote selon la revendication 1, dans lequel Ar₁ et Ar₂ sont choisis chacun indépendamment parmi les substituants représentés par les formules chimiques i-1 à 1-15 suivantes : 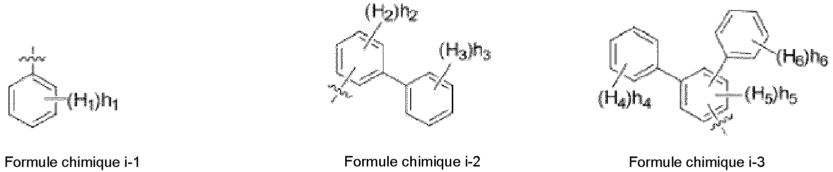 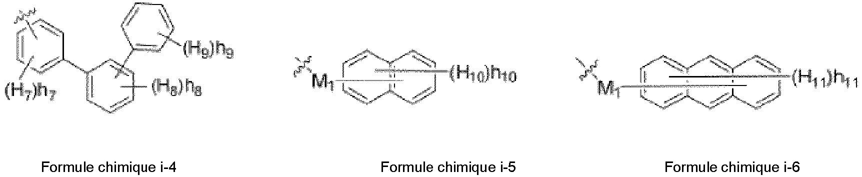 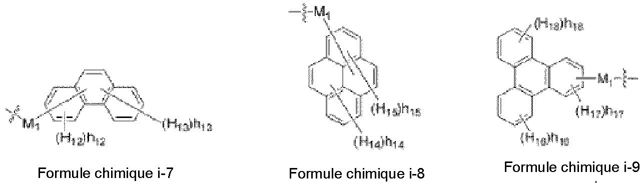 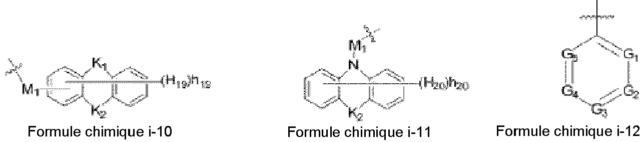 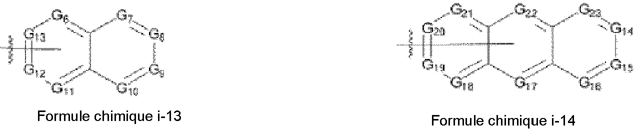 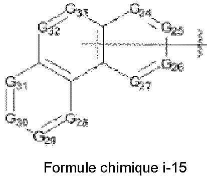
dans lequel M₁ est choisi parmi une liaison simple ou 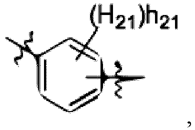
G₁ à G₅ sont choisis chacun indépendamment parmi N ou C(F₁), et au moins un de G₁ à G₅ est choisi parmi N ; lorsque deux ou plus de deux de G₁ à G₅ sont choisis parmi C(F₁), deux F₁ quelconques sont identiques ou différents ;
G₆ à G₁₃ sont choisis chacun indépendamment parmi N ou C(F₂), et au moins un de G₆ à G₁₃ est choisi parmi N ; lorsque deux ou plus de deux de G₆ à G₁₃ sont choisis parmi C(F₂), deux F₂ quelconques sont identiques ou différents ;
G₁₄ à G₂₃ sont choisis chacun indépendamment parmi N ou C(F₃), et au moins un de G₁₄ à G₂₃ est choisi parmi N ; lorsque deux ou plus de G₁₄ à G₂₃ sont choisis parmi C(F₃), deux F₂ quelconques sont identiques ou différents ;
G₂₄ à G₃₃ sont choisis chacun indépendamment parmi N ou C(F₄), et au moins un de G₂₄ à G₃₃ est choisi parmi N ; lorsque deux ou plus de G₂₄ à G₃₃ sont choisis parmi C(F₄), deux F₂ quelconques sont identiques ou différents ;
H₁ à H₂₁, F₁ à F₄ sont choisis chacun indépendamment parmi le deutérium, un halogène, un hétéroaryle présentant 3 à 18 atomes de carbone, un trialkylsilyle présentant 3 à 12 atomes de carbone, un arylsilyle présentant 8 à 12 atomes de carbone, un alkyle présentant 1 à 10 atomes de carbone, un haloalkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, un alcoxy présentant 1 à 10 atomes de carbone, un alkylthio présentant 1 à 10 atomes de carbone, un aryloxy présentant 6 à 18 atomes de carbone, un arylthio présentant 6 à 18 atomes de carbone, un phosphoryloxy présentant 6 à 18 atomes de carbone ;
un quelconque de H₄ à H₂₀ peut également être choisi indépendamment parmi un aryle présentant 6 à 18 atomes de carbone ;
un quelconque de F₁ à F₄ peut également être choisi indépendamment parmi un hydrogène et un aryle présentant 6 à 18 atomes de carbone ;
hₖ est le nombre de substituants H_{K}, k est un nombre entier quelconque de 1 à 21 ; dans lequel, lorsque k est choisi parmi 5 ou 17, hₖ est choisi parmi 0, 1, 2, 3, 4 ou 5 ; lorsque k est 3, hₖ est choisi parmi 0, 1, 2, 3, 4, 5 ou 6 ; lorsque k est 13, hk est choisi parmi 0, 1, 2, 3, 4, 5 ou 6 ; lorsque k est choisi parmi 10 ou 19, hk est choisi parmi 0, 1, 2, 3, 4, 5, 6 ou 7 ; lorsque k est choisi parmi 20, hk est choisi parmi 0, 1, 2, 3, 4, 5, 6, 7 ou 8 ; lorsque k est 11, hₖ est choisi parmi 0, 1, 2, 3, 4, 5, 6, 7, 8 ou 9 ;
lorsque hₖ est supérieur à 1, deux Hk quelconques sont identiques ou différents ;
K₁ est choisi parmi O, S, Se, N (H₂₂), C (H₂₃H₂₄), Si (H₂₃H₂₄) ; dans lequel H₂₂, H₂₃, et H₂₄ sont choisis chacun indépendamment parmi un aryle présentant 6 à 18 atomes de carbone, un hétéroaryle présentant 3 à 18 atomes de carbone, un alkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, ou H₂₃ et H₂₄ ci-dessus sont reliés l'un à l'autre pour former un cycle avec l'atome auquel ils sont reliés conjointement ;
K₂ est choisi parmi une liaison simple, O, S, Se, N(H₂₅), C(H₂₆H₂₇), Si(H₂₆H₂₇) ;
dans lequel H₂₅, H₂₆, H₂₇ sont choisis chacun indépendamment choisi parmi un aryle présentant 6 à 18 atomes de carbone, un hétéroaryle présentant 3 à 18 atomes de carbone, un alkyle présentant 1 à 10 atomes de carbone, un alcényle présentant 2 à 6 atomes de carbone, un alcynyle présentant 2 à 6 atomes de carbone, un cycloalkyle présentant 3 à 10 atomes de carbone, un hétérocycloalkyle présentant 2 à 10 atomes de carbone, un cycloalcényle présentant 5 à 10 atomes de carbone, un hétérocyclényle présentant 4 à 10 atomes de carbone, ou H₂₆ et H₂₇ ci-dessus sont reliés l'un à l'autre pour former un cycle avec l'atome auquel ils sont reliés conjointement.

10. Composé contenant de l'azote selon la revendication 1, dans lequel Ar₁ et Ar₂ sont chacun choisis indépendamment dans le groupe constitué par les groupes suivants :

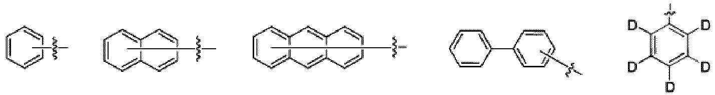

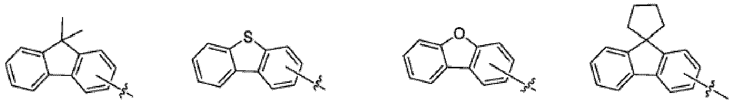

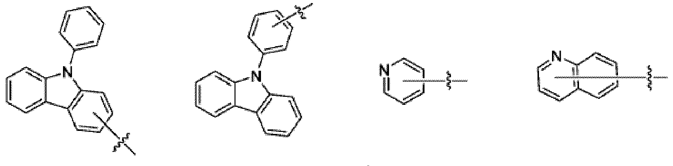

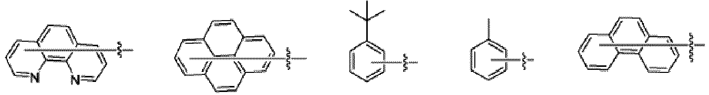

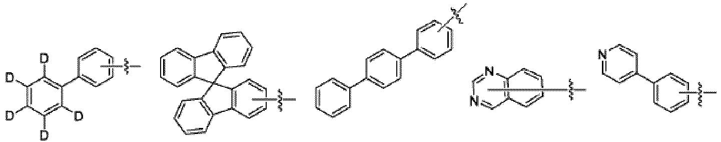

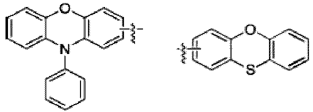

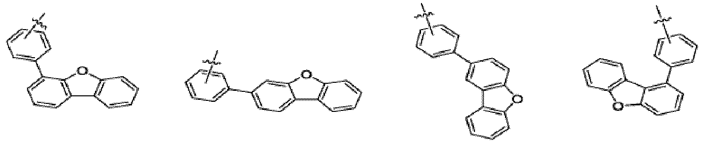

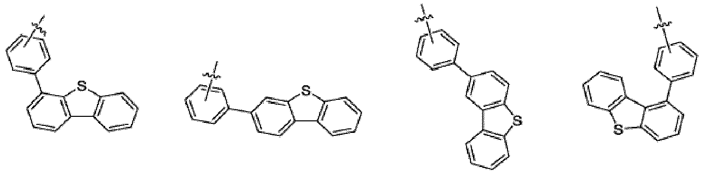

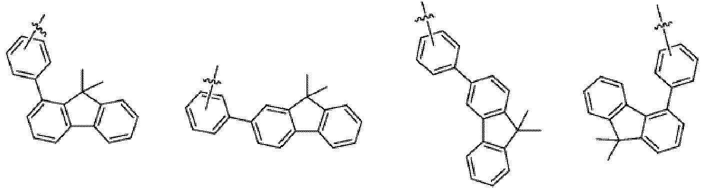

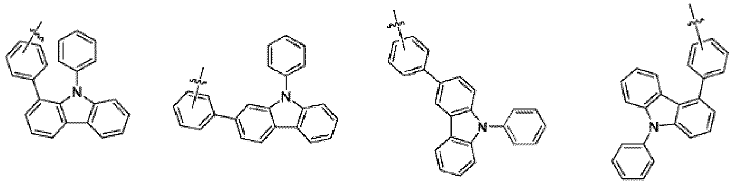

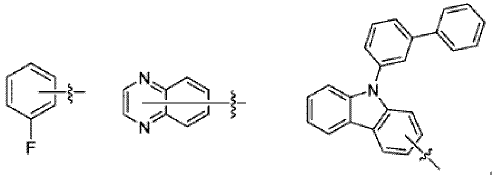

11. Composé contenant de l'azote selon la revendication 1, dans lequel Ar₁ et Ar₂ sont choisis chacun indépendamment dans le groupe constitué par les groupes suivants :

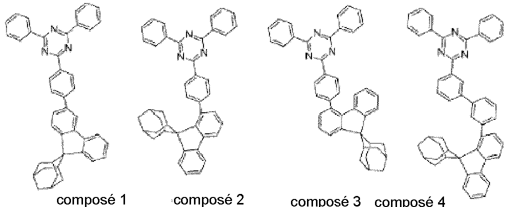

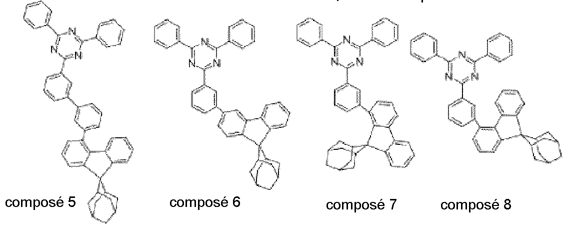

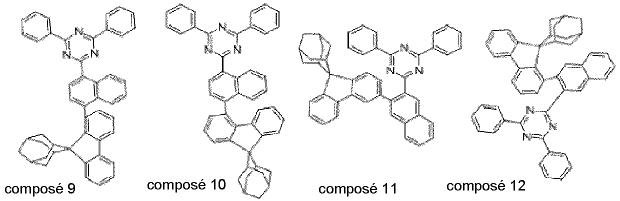

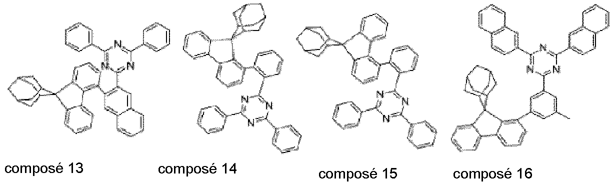

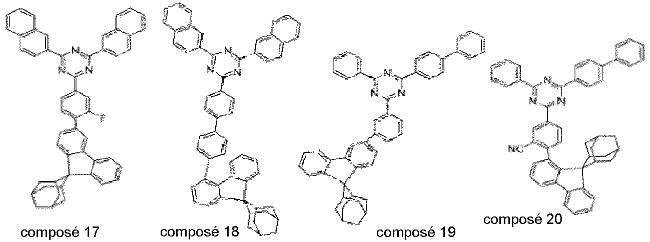

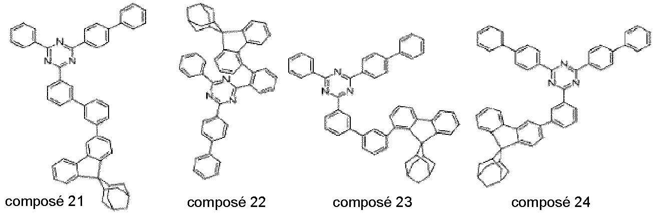

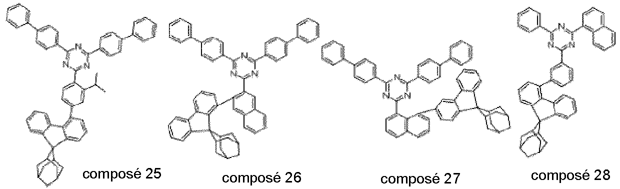

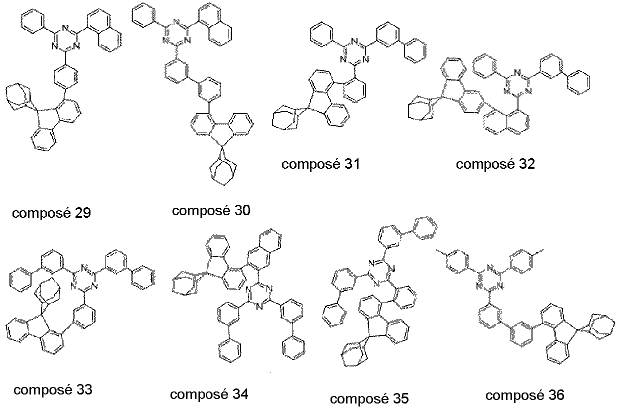

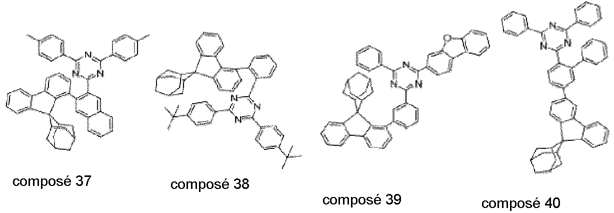

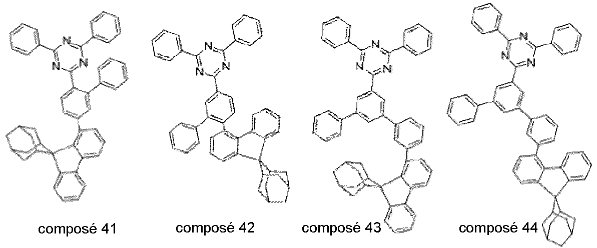

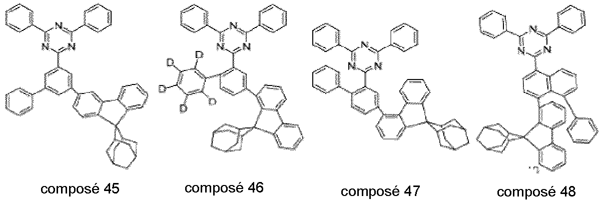

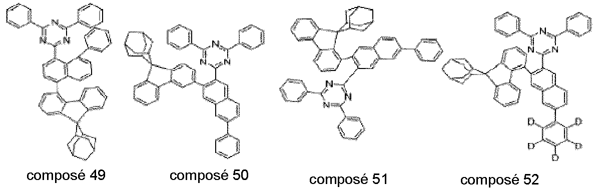

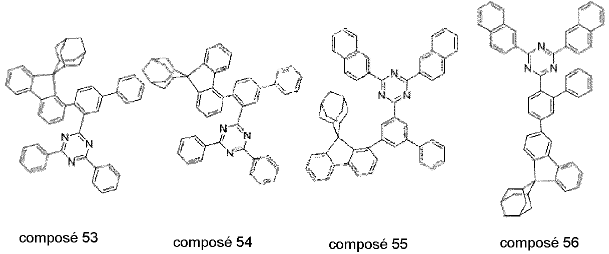

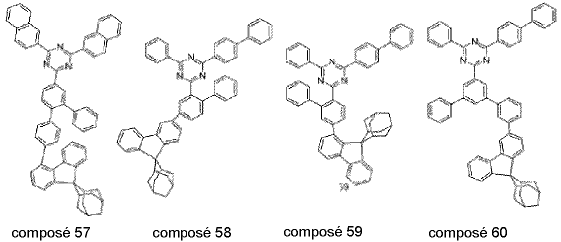

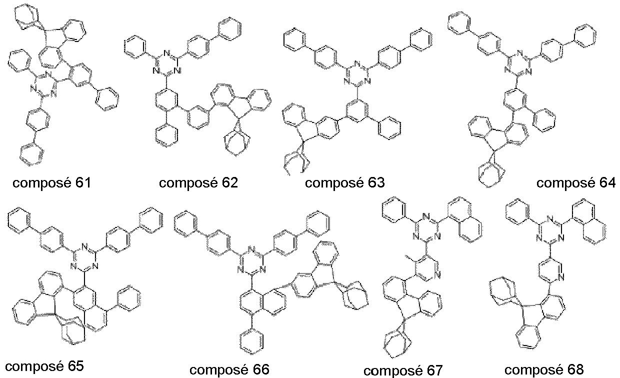

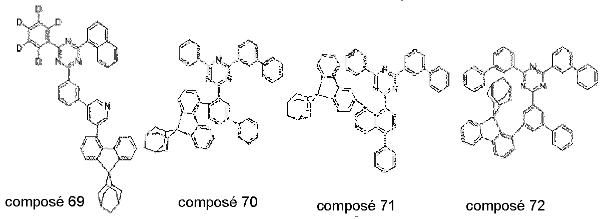

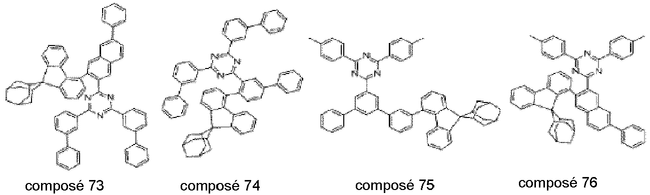

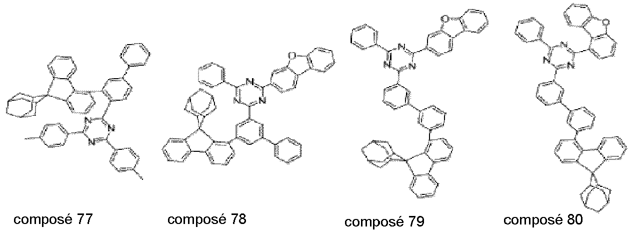

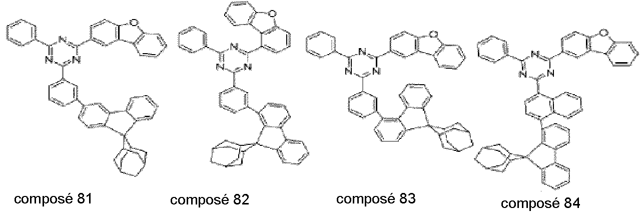

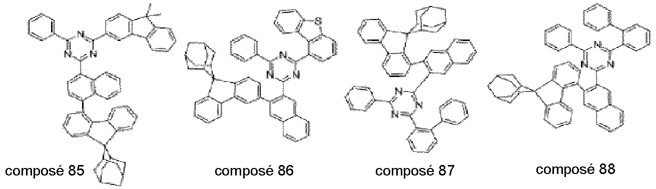

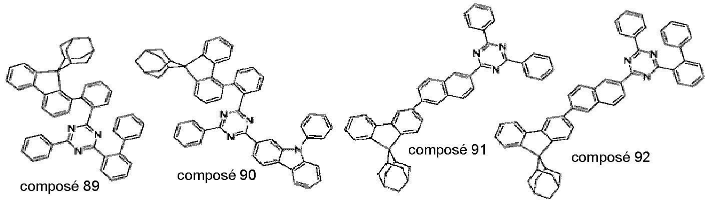

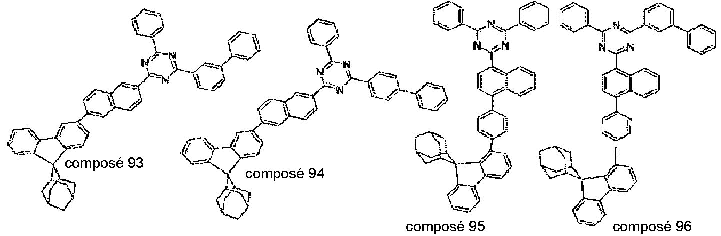

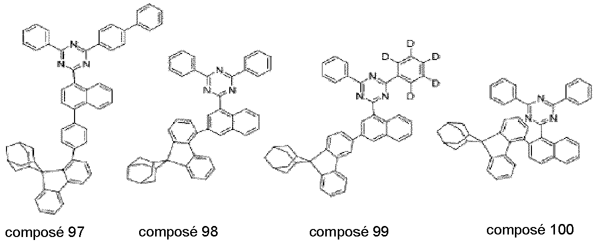

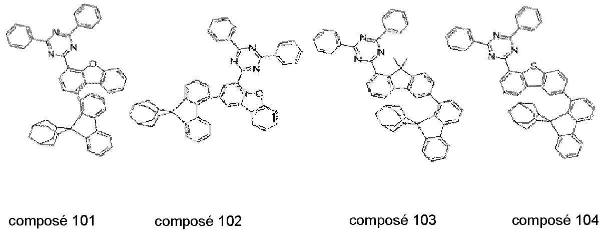

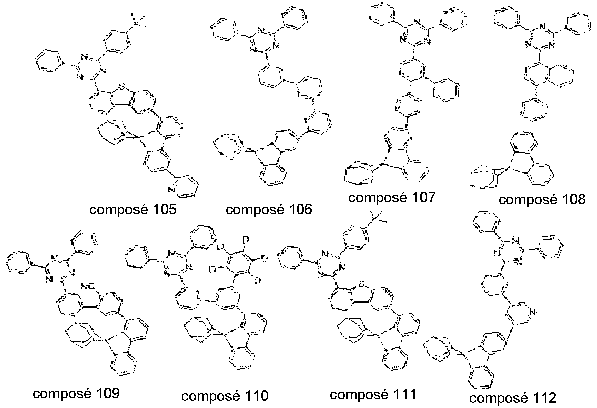

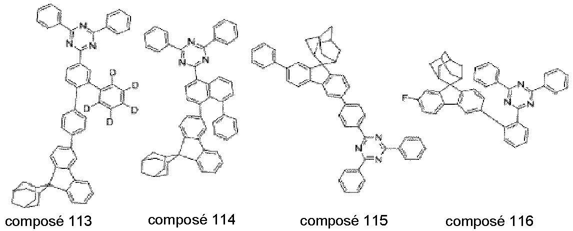

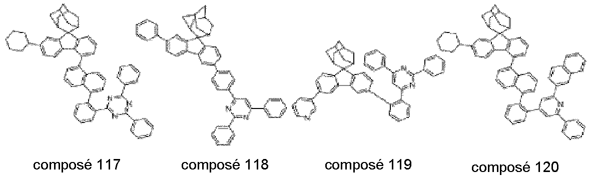

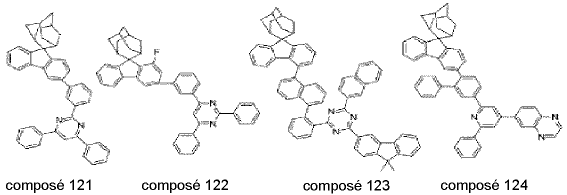

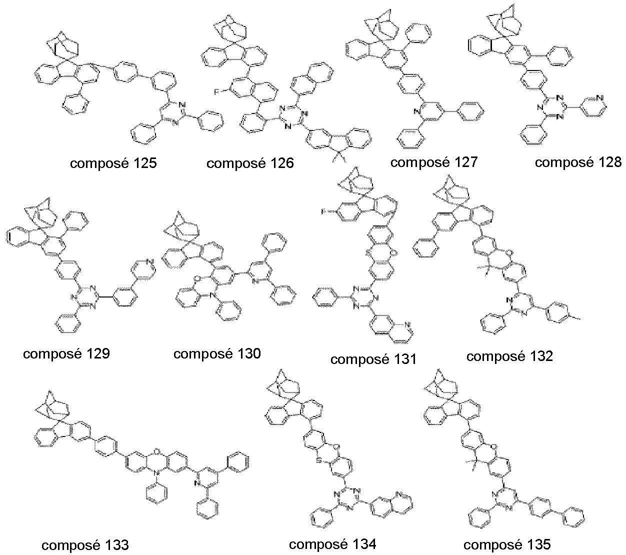

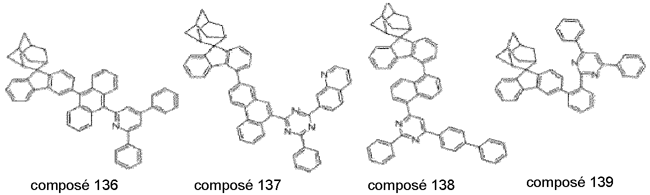

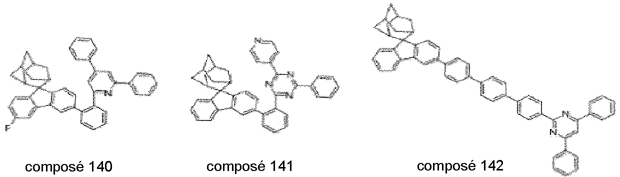

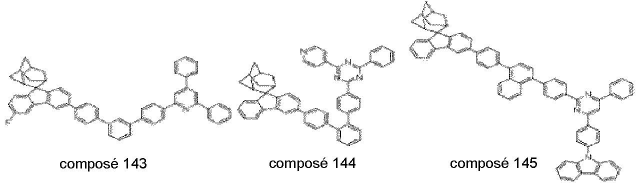

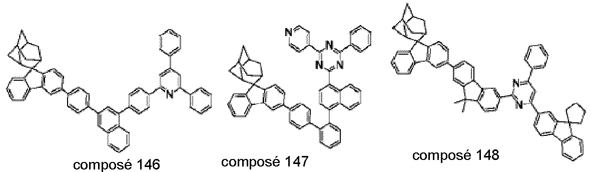

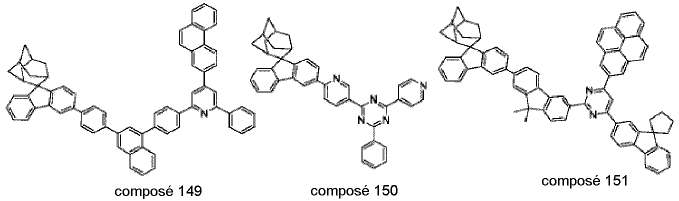

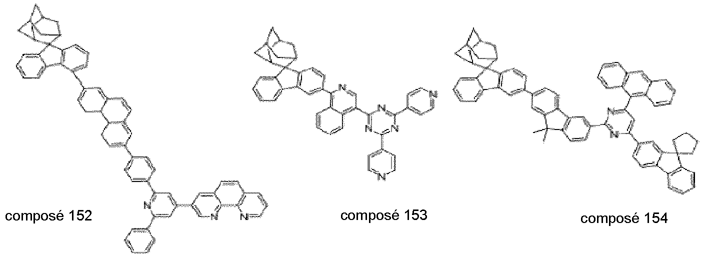

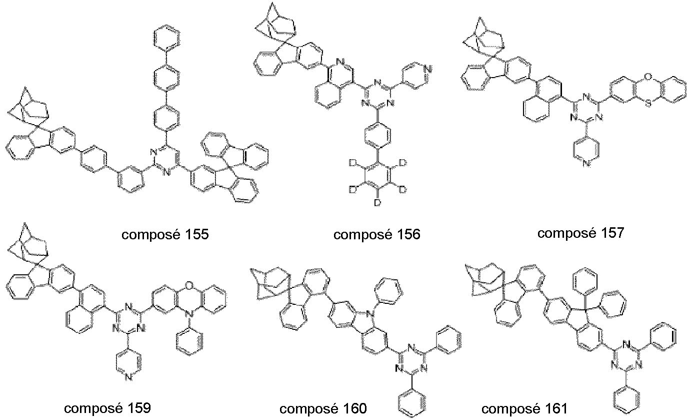

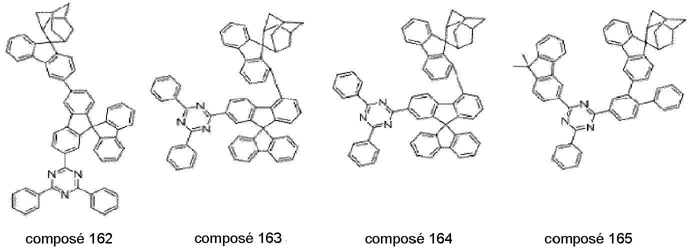

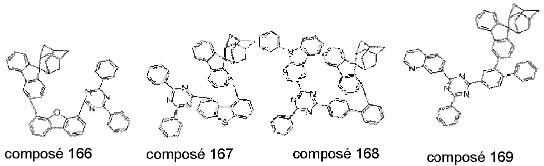

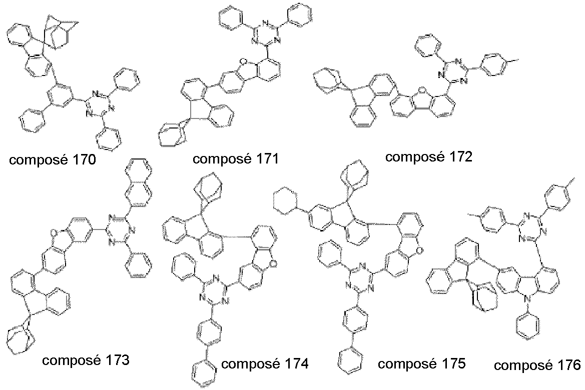

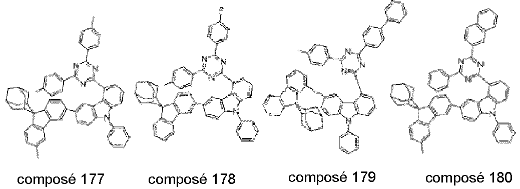

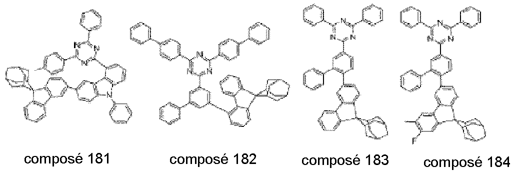

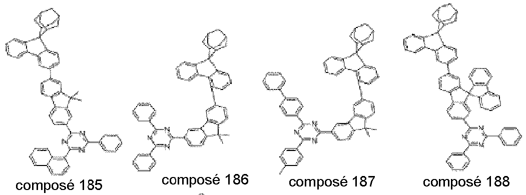

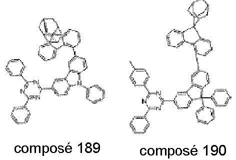

12. Elément électronique, dans lequel l'élément électronique comprend une anode et une cathode disposées de manière opposée, et une couche fonctionnelle disposée entre l'anode et la cathode ;
la couche fonctionnelle comprend le composé contenant de l'azote selon l'une quelconque des revendications 1 à 11 ;
de préférence, la couche fonctionnelle comprend une couche de transport d'électrons, et la couche de transport d'électrons comprend le composé contenant de l'azote ;
de préférence, la couche de transport d'électrons comprend en outre du LiQ ;
de préférence, l'élément électronique est un dispositif électroluminescent organique ou un dispositif de conversion photoélectrique ;
de préférence, le dispositif électroluminescent organique est un dispositif à lumière rouge.

13. Dispositif électronique comportant l'élément électronique selon la revendication 12.
